(19) Europäisches Patentamt
European Patent Office
Office européen des brevets

(11) EP 0 965 584 A2

# (12) EUROPEAN PATENT APPLICATION

(43) Date of publication:
**22.12.1999 Bulletin 1999/51**

(21) Application number: **99111504.9**

(22) Date of filing: **14.06.1999**

(51) Int. Cl.$^6$: **C07C 323/52**, C07D 233/54, C08F 120/60, A61K 48/00, C12N 15/87

(84) Designated Contracting States:
**AT BE CH CY DE DK ES FI FR GB GR IE IT LI LU MC NL PT SE**
Designated Extension States:
**AL LT LV MK RO SI**

(30) Priority: **15.06.1998 EP 98401471**

(71) Applicant: **TRANSGENE S.A.**
**67082 Strasbourg Cédex (FR)**

(72) Inventors:
- **Boussif, Otmane**
**67000 Strasbourg (FR)**
- **Vierling, Pierre**
**06950 Salicon (FR)**
- **Santaella, Catherine**
**04300 Saint Maime (FR)**

(74) Representative:
**VOSSIUS & PARTNER**
**Siebertstrasse 4**
**81675 München (DE)**

## (54) Polyamine compounds and compositions containing them useful for the transfer of active substances into a cell

(57) The application relates to polyamine telomer compounds comprising the group of the formula I

I

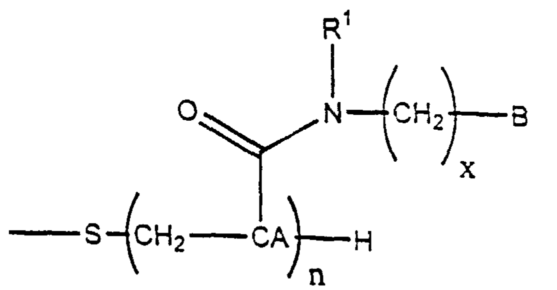

wherein:

A is H or an alkyl of 1 to 4 carbon atoms or an aryl of 5 to 7 carbon atoms,
the degree of polymerization n ranges from 1 to 100,
$R^1$ is selected, independently of one another in each $[CH_2-CA]_n$ repeat, from -H, $-CH_3$, -$C_2H_5$ and $-(CH_2)_u$-B,
x and u are integers from 2 to 4,
B being

EP 0 965 584 A2

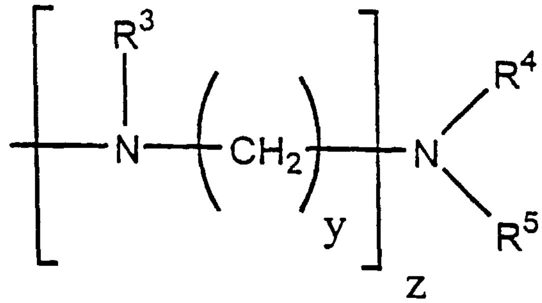

or

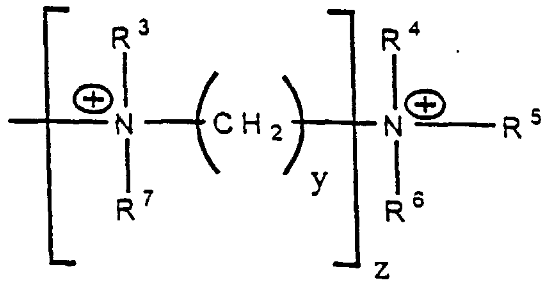

wherein:

y is an integer from 2 to 4,

z is an integer from 0 to 6,

$R^3$, $R^4$, $R^5$, $R^6$ and $R^7$ are selected, independently of one another, from H, alkyl or hydroxyalkyl of 1 to 4 carbon atoms.

Fig. 1

in vitro transfection with polyamine telomers

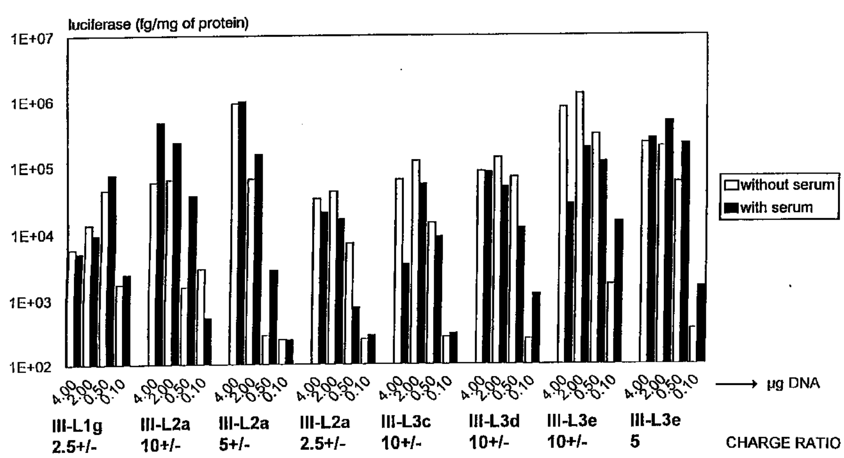

## Description

The present invention concerns new polyamine telomer compounds and new compositions containing them. More particularly, this invention relates to the use of these compounds or compositions to prepare a complex useful for the transfer of an active substance comprising negative charges, particularly a nucleic acid, into a cell, more particularly a mammalian cell.

Gene therapy has generally been conceived as principally applicable to heritable deficiency diseases (cystic fibrosis, dystrophies, haemophilias,...) where permanent cure may be effected by introducing a functional gene. However, a much larger group of diseases, notably acquired diseases (cancer, AIDS, multiple sclerosis,...) might be treatable by transiently engineering host cells to produce beneficial proteins.

Another application of gene therapy is vaccination. In this regard, the immunogenic product encoded by the nucleic acid introduced into cells of a vertebrate may be expressed and secreted or be presented by said cells in the context of the major histocompatibility antigens, thereby eliciting an immune response against the expressed immunogen. Functional nucleic acid can be introduced into cells by a variety of techniques resulting in either transient expression of the gene of interest, referred to as transient transfection, or permanent transformation of the host cells resulting from incorporation of the nucleic acid into the host genome.

Successful gene therapy depends on the efficient delivery to and expression of genetic information within the cells of a living organism. Most delivery mechanisms used to date involve viral vectors, especially adeno- and retroviral vectors. Viruses have developed diverse and highly sophisticated mechanisms to achieve this goal including crossing of the cellular membrane, escape from endosomes and lysosomal degradation, and finally delivery of their genome to the nucleus followed by expression of the viral genome. In consequence, viruses have been used in many gene delivery applications in vaccination or gene therapy applied to humans. The use of viruses suffers from a number of disadvantages: retroviral vectors can not accomodate large-sized DNA (e.g. the dystrophin gene, around 13 kb), the retroviral genome is integrated into host cell DNA and may thus cause genetic changes in the recipient cell and infectious viral particles could disseminate within the organism or into the environment; adenoviral vectors can induce a strong immune response in treated patients (Mc Coy et al, 1995, Human Gene Therapy, 6, 1553-1560; Yang et al., 1996, Immunity, 1, 433-442). Nevertheless, despite these drawbacks, viral vectors are currently the most useful delivery systems because of their efficiency.

In 1990, Wolff et al. (Science, 247, 1465-1468) have shown that injection of naked RNA or DNA, without any special delivery system, directly into mouse skeletal muscle results in expression of reporter genes within the muscle cells. Nevertheless, although these results indicate that nucleic acid by itself is capable of crossing the plasma membrane of certain cells *in vivo*, the efficiency of the transfection actually observed remains very limited due, in particular, to the polyanionic nature of nucleic acids which limits their passage through negatively-charged cell membranes.

In 1989, Feigner et al. (Nature, 337, 387-388) proposed the use of cationic lipids in order to facilitate the introduction of large anionic molecules such as nucleic acids into cells. These cationic lipids are capable of forming complexes with anionic molecules, thus tending to neutralize the negative charges of these molecules allowing to compact the complex, and favoring its introduction into the cell. In this way, related non-viral delivery systems have been developed which are based on receptor-mediated mechanisms (Perales et al., 1994, Eur. J. Biochem. 226, 255-266; Wagner et al., 1994, Advanced Drug Delivery Reviews, 14, 113-135), on polymer-mediated transfection such as polyamidoamine (Haensler et Szoka, 1993, Bioconjugate Chem., 4, 372-379), dendritic polymer (WO 95/24221), polyethylene imine or polypropylene imine (WO 96/02655), polylysine (US-A-5 595 897 or FR 2 719 316) or on lipid-mediated transfection (Feigner et al., 1989, Nature, 337, 387-388) such as DOTMA (Feigner et al., 1987, PNAS, 84, 7413-7417), DOGS or Transfectam™ (Behr et al.,1989, PNAS, 86, 6982-6986), DMRIE or DORIE (Felgner et al., 1993, Methods 5, 67-75), DC-CHOL (Gao et Huang, 1991, BBRC, 179, 280-285), DOTAP™ (McLachlan et al., 1995, Gene Therapy, 2,674-622) or Lipofectamine™. These systems present potential advantages with respect to large-scale production, safety, targeting of transfectable cells, low immunogenicity, and the capacity to deliver large fragments of DNA.

However, several studies (for example, Mahato et at., J. Pharm. Sci., 1995, 84, 1267-1271, Thierry et al., 1995, PNAS 92, 9742-9746) have shown that the transfer efficiency of the complexed anionic macromolecules into the cells, especially in the case of *in vivo* transfer, can vary in function of the interaction between the complexes and the cell membranes, the cell type involved, the lipid composition of the cationic components, the size of the complexes formed with the anionic molecules and, especially, the ratio of the positive to negative charges of the different components of the complex. Currently, very little is known concerning the mechanisms which enable the interaction of the complexes with the cell membranes and the transfer of the complexes into the cell, and the ongoing research remains highly empirical. Consequently, there is a need to develop new compounds, especially cationic compounds, with improved properties or properties different from those already described.

The applicant has now identified new polyamine telomer compounds which are capable of transfering negatively-charged active substances, in particular nucleic acids, into a cell and which can find an application in *in vivo* gene therapy.

**LEGEND OF FIGURES:**

Figures 1, 2, 3, and 4: Transfection of A549 cells with the complexes composed of polyamine telomer compounds of the invention and plasmid pTG11033 with a charge ratio +/- of 10, 5 or 2.5 (as indicated), in the presence or absence of serum. DNA amounts are indicated (μg DNA).The open columns list transfections without serum and the solid columns those containing 10% serum.

Figures 1 and 3: the analyzed complexes do not contain DOPE ; figures 2 and 4 : DOPE is present in the complexes.

According to the present invention, "polyamine telomer compound" designates products obtainable from polymerizing 1) at least one monomer (named taxogen M), for example an acrylamide or methylacrylamide derivative comprising a polyamine part, or one of its precursors, in the presence of 2) a transfer agent ZX (named telogen), for example alkanethiols or thiols containing perfluorinated groups, and of 3) an initiator, which could be a radical generator, for example iodine or α,α'-azo-isobutyronitrile (AIBN). The polyamine telomers according to the present invention are compounds of the formula Z-(M)n-X showing a low degree of polymerization ($1 \leq < 200$) and hence a relatively low molecular weight, whereas the compounds obtainable with the classic polymerization processes are of the formula (M)n wherein the degree of polymerization is high ($n >> 200$) and hence molecular weights are significantly higher. Moreover, the average degree of polymerization (n) of the polyamine telomer compounds being particularly linked to the taxogen/telogen molar ratio, the present invention makes it possible to control and modulate the number of amine functions of the telomers.

Thus, the present invention concerns a polyamine telomer compound comprising the group of the formula I:

I

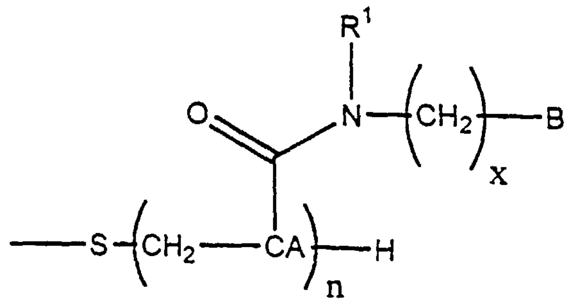

wherein:

A is H or an alkyl of 1 to 4 carbon atoms or an aryl of 5 to 7 carbon atoms , preferably A is a $C_1$-$C_4$ alkyl, more preferably A is $-CH_3$,
the degree of polymerization n ranges from 1 to 100, preferably from 2 to 90,
$R^1$ is selected, independently of one another in each $[CH_2\text{-}CA]_n$ repeat, from -H, $-CH_3$, $-C_2H_5$ and $-(CH_2)_u$-B,
x and u are integers from 2 to 4,
B being

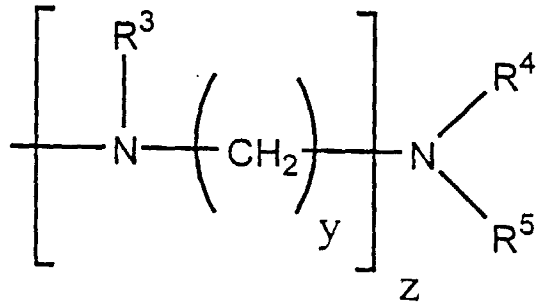

or

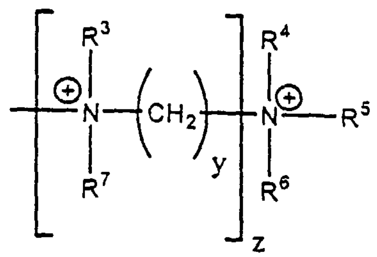

wherein:

y is an integer from 2 to 4,
z is an integer from 0 to 6, preferably from 0 to 3,
$R^3$, $R^4$, $R^5$, $R^6$ and $R^7$ are selected, independently of one another, from H, alkyl or hydroxyalkyl of 1 to 4 carbon atoms, especially from -H,$-CH_3$, $-C_2H_5$ or $HOCH_2CH_2$-, preferably $R^3$, $R^4$, $R^5$, $R^6$ and $R^7$ are, independently of one another, H- or $-CH_3$.

According to a preferred embodiment, said polyamine telomer compound is in cationic form, this means either a) it is in protonated form by binding a proton to at least one nitrogen atom present in the polyamine, or b) it is in ammonium form by binding $-CH_3$,$-C_2H_5$ or $HOCH_2CH_2$- to at least one nitrogen atom presents in the polyamine or c) it is in forms a) and b). In these cases, said cationic polyamine telomer compound would be associated with one or several biologically acceptable anions, such as, for example, trifluoroacetate, monoethylsulfate, acetate, iodide, chloride, bromide, etc.

In one specific embodiment, the invention provides compounds of formula II

**II**

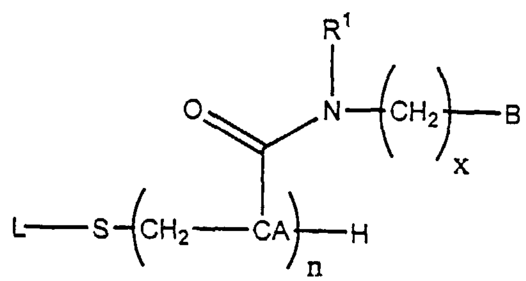

wherein:

A, $R^1$, B, n and x are defined as above,
L is selected from groups L0 , L1, L2, L3, L4 , L5 and L6
wherein L0 being selected from the group -H, alkyl radical of 1 to 4 carbon atoms optionally substituted with at least one substituent selected from OH, COOH and $NH_2$, especially $-CH_3$, $-C_2H_5$, $-C_3H_7$, $-CH_2CH_2$-OH, $-CH_2CH_2CH_2$-OH, $-CH_2CH_2$-COOH,

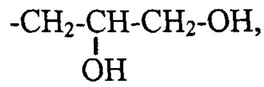

$-CH_2CH_2CH_2-COOH$, and L1 L2, L3, L4 , L5 and L6 having the formulas :

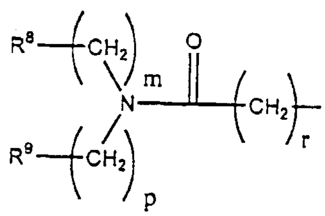

L1

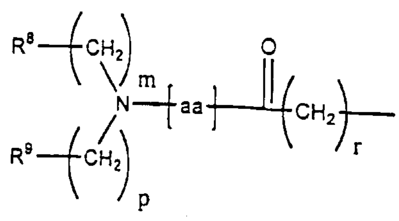

L2

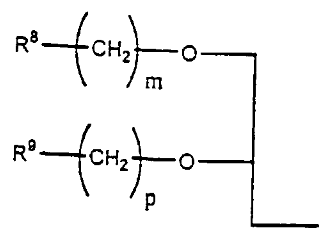

L3

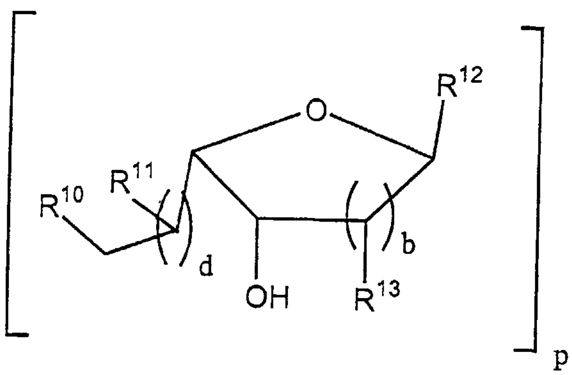

L4

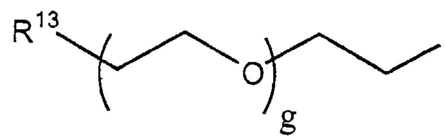

L5

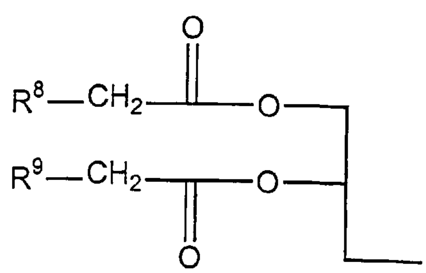

L6

wherein:

r = 1, 2 or 3,

b =1 or 2 and d= 0 or 1, b and d can be different of one another in each p repeat,

g ranges from 0 to 200,

m and p ranges, independently of one another from 1 to 20, preferably from 8 to 17,

$R^8$, $R^9$ are selected, independently of one another, from $CH_3$-, $CH_3-(CH_2)_w-CH=CH-$ (cis or trans) with w = 5 or 7, $C_sF_{2s+1}$ or $C_sF_{2s+1}-CH=CH-$ (cis or trans) with s = 2, 4, 6, 8 or 10,

at least one of $R^{10}$, $R^{11}$ or $R^{12}$ is the binding site where L4 is linked with S of the compound of formula II, the other

one(s) being selected, independently of one another, from the group -H, -OH, =O, $NH_2$, $-NH-COCH_3$, alkyl radical of 1 to 4 carbon atoms optionally substituted with at least one substituent selected from OH, COOH and $NH_2$, especially $-CH_3$, $-C_2H_5$, $-C_3H_7$, $-CH_2CH_2-OH$, $-CH_2CH_2CH_2-OH$, $-CH_2CH_2-COOH$, $-CH_2-CH(OH)-CH_2-OH$, $-CH_2CH_2CH_2-COOH$, and $R^{10}$, $R^{11}$ or $R^{12}$ can be different of one another in each p repeat,

$R^{13}$ is selected from the group -H, -OH, $-NH_2$, $-NHCOCH_3$, alkyl radical of 1 to 4 carbon atoms optionally substituted with at least one substituent selected from OH, COOH and $NH_2$, especially $-CH_3$, $-C_2H_5$, $-C_3H_7$, $-CH_2CH_2-OH$, $-CH_2CH_2CH_2-OH$, $-CH_2CH_2-COOH$, $-CH_2-CH(OH)-CH_2-OH$, $-CH_2CH_2CH_2-COOH$; $R^{13}$ can be different of one another in each b when b = 2.

[aa] is an $\alpha$-amino acid, bonded to the amine residue via its acid function and to the $CO(CH_2)_r$ moiety via its amine function, said $\alpha$-amino acid comprising an amine/ammonium function with a weak pKa, for example an imidazole/imidazolium or a carboxylate/carboxylic function. More specifically, [aa] is selected from the group consisting of histidine, 3-methylhistidine, aspartic and glutamic acid. According to a specific embodiment, said L group can be attached to the I part through a linkage part such as for example polyether (such as PEG), polythioether, polycyclic residues even saturated or not, polysaccharide or saccharide (such as dextran or hexose, respectively).

In the specific case of L4, when p is at least 2, the repeated motif can be linear or branched, and each of $R^{10}$, $R^{11}$, $R^{12}$ and $R^{13}$ can be substituted by $R^{14}$ or the hydrogen of OH may be substituted by $R^{14}$. $R^{14}$ may be independently selected from

(i) alkyl radical of 1 to 4 carbon atoms optionally substituted with at least one substitutent selected from -OH, -COOH, and $-NH_2$, especially $-CH_3$, $C_2H_5$, $-CH_2CH_2-OH$, $-CH_2CH_2CH_2OH$, $-CH_2-CH_2-COOH$, $-CH_2-CH(OH)-CH_2-OH$, $-CH_2CH_2CH_2-COOH$, and

(ii) labelling molecules, targeting or anchoring molecules, etc.

In a preferred embodiment, the substitution of the OH group leads to ether or ester compounds.

Usually, when the motif is linearly repeated, the repeats are engaged in a C1/C3 (or C4 depending on b values) linkage. More specifically, said L4 is a sacchride or a polysaccharide deriving, for example, from fructose, ribose, arabinose, glucose, glucosamine, galactose, galactosamine, mannose, lactose, maltose, cellobiose, etc.

According to the present invention, the new cationic polyamine telomer compounds show a modular structure. They consist first of a polar moiety including a variable number of primary and/or secondary amine functions and secondly in a hydrophobic moiety including hydrocarbon and/or perfluoroalkyl chains of variable-length. Because of their modular structure, the properties and biological behavior of these compounds can be adapted. Furthermore, due to the presence of primary or secondary amine functions, the compounds of the invention can be substituted. Such substitutions can consist in the addition of at least one labelling molecule (for example, see molecules disclosed in US-A-4711955) enabling, for example, visualization of the distribution of the compounds, or of complexes incorporating them, after *in vitro* or *in vivo* administration; a cell targeting molecule (ligand) or an anchoring molecule. In one specific embodiment, the invention also relates to a compound, such as the one described above, conjugated to one or more targeting element(s) bound to at least one of the secondary or primary nitrogen atoms of the polyamine chain. Such elements, which have been widely described in scientific publications, allow targeting of a specific cell type, facilitating penetration into the cell, lysis of endosomes or even intracellular transport towards the nucleus. These elements may be composed of all or part of sugars, glycol, peptides (e.g. GRP, Gastrin Releasing Peptide), oligonucleotides, lipids, hormones, vitamins, antigens, antibodies (or fragments thereof), specific membrane receptor ligands, ligands capable of reaction with an anti-ligand, fusogen peptides, nuclear localization peptides, or a combination of said compounds, e.g. galactosyl residues to target the asialoglycoprotein receptor on the surface of hepatocytes, the INF-7 fusogen peptide derivated from the HA-2 subunit of the influenza virus hemagglutinin (Plank et al. 1994, J. Biol. Chem. *269*, 12918-12924) for membrane fusion, or a nuclear signal sequence derived from the T-antigen of the SV40 virus (Lanford and Butel, 1984, Cell *37*, 801-813) or from the EBNA-1 protein of the Epstein Barr virus (Ambinder et al., 1991, J. Virol. *65*, 1466-1478). Compounds according to the present invention can also be substituted on carbon atoms, in particular carbon atoms selected from those in $R^1$ to $R^{13}$. It should be noted that the substitution of non-reactive groups, such as the carbon atoms in CH or $CH_2$, shall be performed during the synthesis of the claimed compounds using methods familiar to skilled person, whereas reactive groups, such as primary or secondary amines, can involve substitutions on the neosynthesized compounds of the invention.

Such substituted polyamine telomer compounds can be obtained easily using the techniques described in the literature, especially by chemical coupling, notably by using protector groups such as trifluoroacetyl, Fmoc (9-fluorenylmethoxycarbonyl) or BOC (tert-butyloxycarbonyl) on the polyamine moiety. Selective removal of a protector group then allows coupling of the targeting element, and then complete deprotection of the lipopolyamine.

In a special case of this invention, the polyamine telomer compounds designed with L1, L2 or L3, L4 , L5 or L6 include fluorinated or perfluoroalkyl termini on their lipidic part which allow to introduce some of the specific features

that make up the uniqueness of fluorocarbon material (i.e. high hydrophobicity and low lipophilicity) (Riess, 1994, Journal of Drug Targeting, 2, 455-468) which should render the resulting polyamine telomer compound/active substance complexes (called telopolyplexes) more stable.

In another special case of the invention, the thioeter group of the polyamine telomer compounds designed with L0, L1, L2, L3, L4, L5 and L6 has been oxidized into a sulfone ($-SO_2-$) or sulfoxide (-SO-).

In a preferred embodiment, the polyamine telomer compound is selected from the group consisting in the following formulas III-L1 to III-L3:

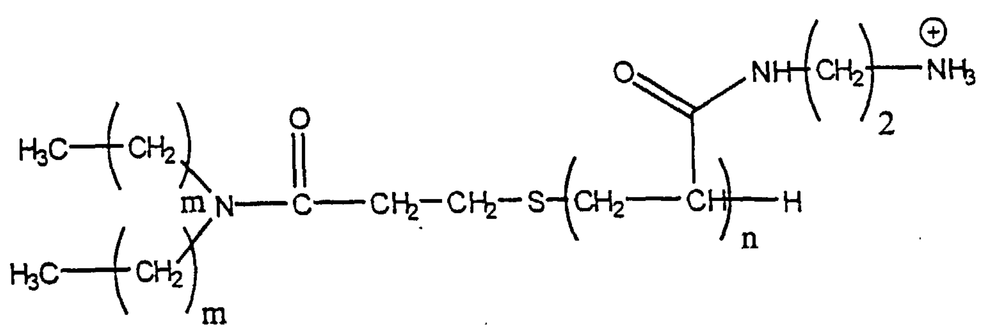

III-L1

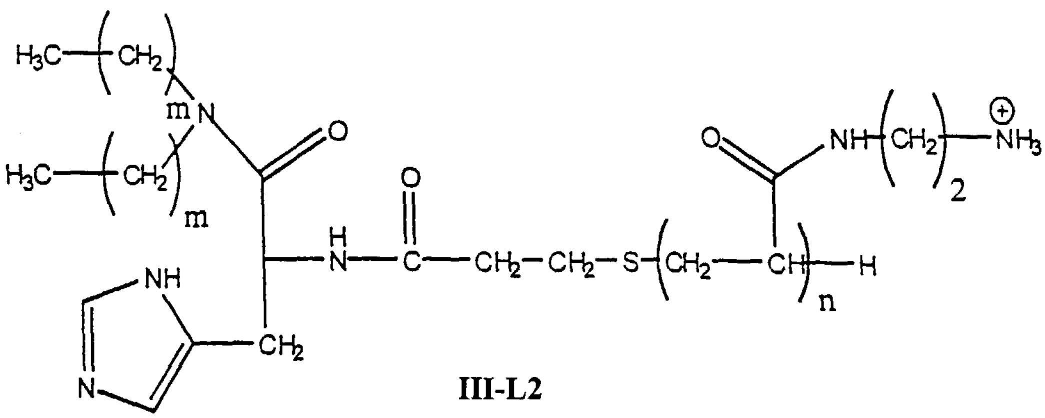

III-L2

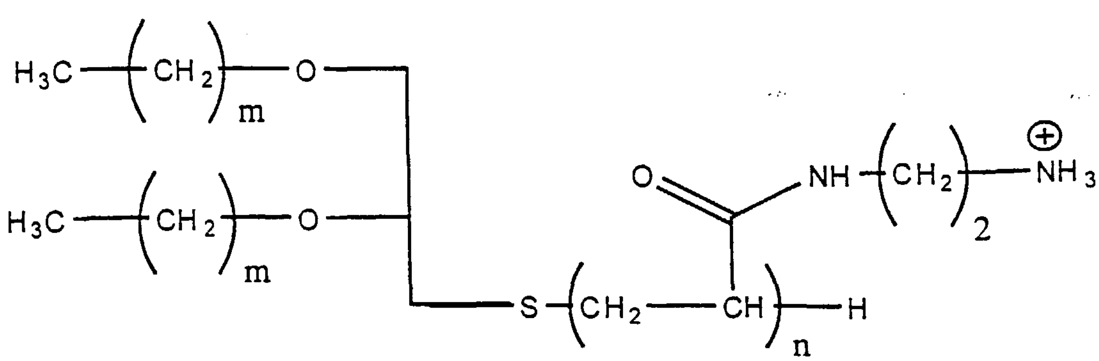

III-L3

wherein m=p .

Particularly preferred compounds of formula III-L1 are the following compounds:

compound III-L1a wherein m=17 and n=1
compound III-L1b wherein m=17 and n=2
compound III-L1c wherein m=17 and n=3
compound III-L1d wherein m=17 and n=4
compound III-L1e wherein m=17 and n=5
compound III-L1f wherein m=17 and n=10
compound III-L1g wherein m=17 and n=20
compound III-L1h wherein m=17 and n=40
compound III-L1i wherein m=17 and n=50
compound III-L1j wherein m=17 and n=60
compound III-L1k wherein m=13 and n=10
compound III-L1l wherein m=13 and n=20
compound III-L1m wherein m=13 and n=70

Particularly preferred compounds of formula III-L2 are the following compounds:

compound III-L2a wherein m=17 and n=2
compound III-L2b wherein m=17 and n=20
compound III-L2c wherein m=17 and n=30
compound III-L2d wherein m=17 and n=90

Particularly preferred compounds of formula III-L3 are the following compounds:

compound III-L3a wherein m=17 and n=1
compound III-L3b wherein m=17 and n=3
compound III-L3c wherein m=17 and n=6
compound III-L3d wherein m=17 and n=11
compound III-L3e wherein m=17 and n=16

According to an illustrative and not limitative method, polyamine telomer compounds of the present invention can be prepared by a polymerization reaction (named telomerization) between telogens for instance of the types L1-SH, L2-SH or L3-SH (a method for their synthesis is illustrated in Reaction Schemes 1 to 3, however, these telogens can also be obtained by other methods) and the taxogen $CH_2$=CA-CO-NHC$H_2$C$H_2$-NHG, $CH_2$=CA-CO-NH-$CH_2CH_2NMe_2$ or $CH_2$=CA-CO-N[$CH_2CH_2$-NHG]$_2$, with AIBN used as radical initiator. Said telomerization is then followed, if need be, by a deprotection reaction of the G groups (see Reaction Scheme 4, route d). This telomerization can be performed with variable telogen/taxogen molar ratios, which can easily be determined by those skilled in the art, thus allowing modulation of the average degree of polymerization (n) of the telomers.

Alternatively and as illustated in Reaction Scheme 4, route f, the telomerization can also be performed with mixtures of taxogens for the synthesis of polyamine co-telomers of the present invention, such as compounds VII-L wherein the -C(=O)-N(R1 or R2)-($CH_2$)x-B1 or B2 part is different in each or some of the [$CH_2$-CA]n repeats.

## Reaction Scheme 1

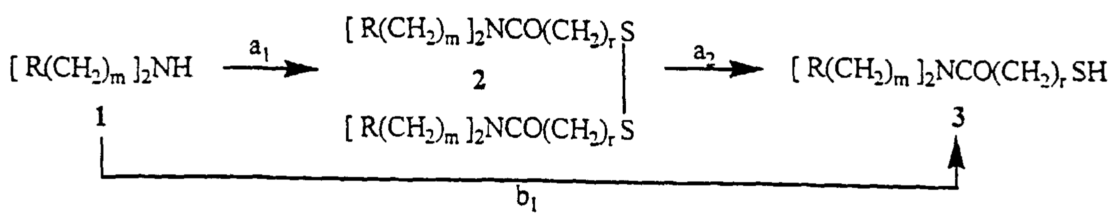


$a_1$: [ClCO($CH_2$)$_r$ S]$_2$ / $NEt_3$ / $CHCl_3$ $a_2$: Zn/AcOH or $NaBH_4$/THF

$b_1$: HOOC($CH_2$)$_r$ SH / DCC / HOBt

Reaction Scheme 2 A

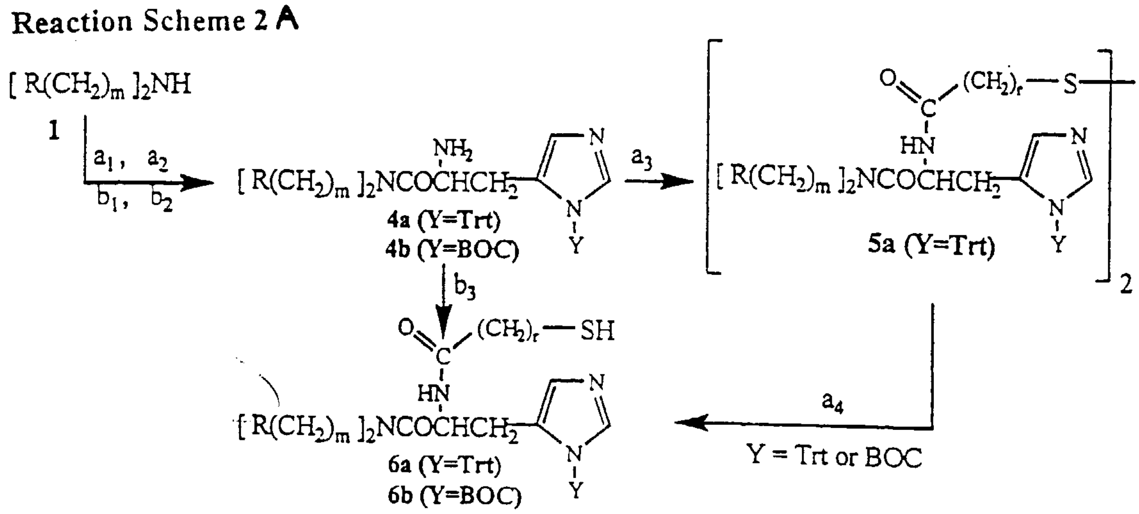


$a_1$: DCC / HNS / Fmoc-His(Trt)/$CHCl_3$ $a_2$: morpholine, DMF

$a_3$: $[ClCO(CH_2)_rS]_2$ / NEt3 / $CHCl_3$ $a_4$: $NaBH_4$ / THF

$b_1$: DCC / HNS / Fmoc-His(BOC)/$CHCl_3$ $b_2$: morpholine, DMF

$b_3$: $HOOC(CH_2)_rSH$ / EDC / HOBt / $CHCl_3$

Reaction Scheme 2B

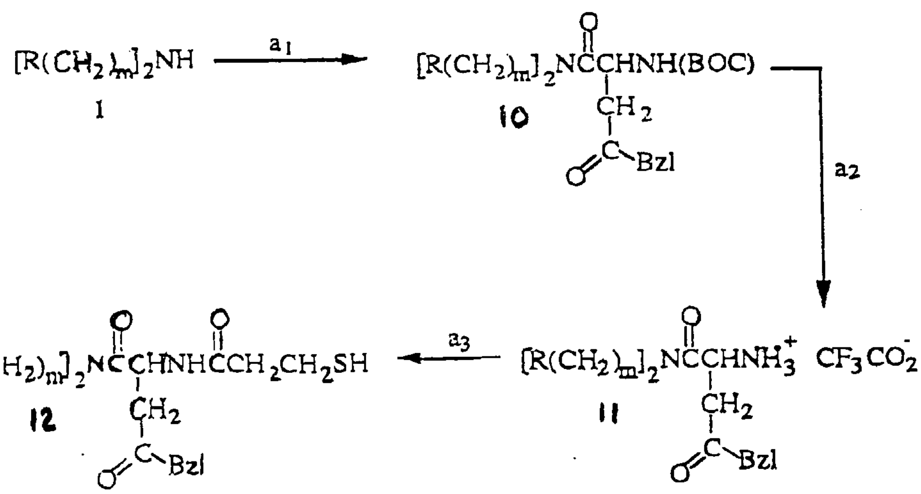


$a_1$ : PyBrop / $NEt_3$ / $CH_2Cl_2$

$a_2$ : TFA

$a_3$ : HOBt / EDC / $NEt_3$ / $CH_2Cl_2$

## Reaction Scheme 3

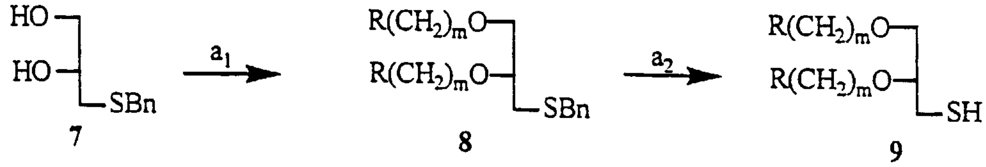


7 8 9

$a_1$: $R(CH_2)_mX$ (X = Br, I, OMs, OTs) / $Bu_4NHSO_4$, $Et_2O$, 9N KOH $a_2$: Na, tBuOH

Reaction Scheme 4

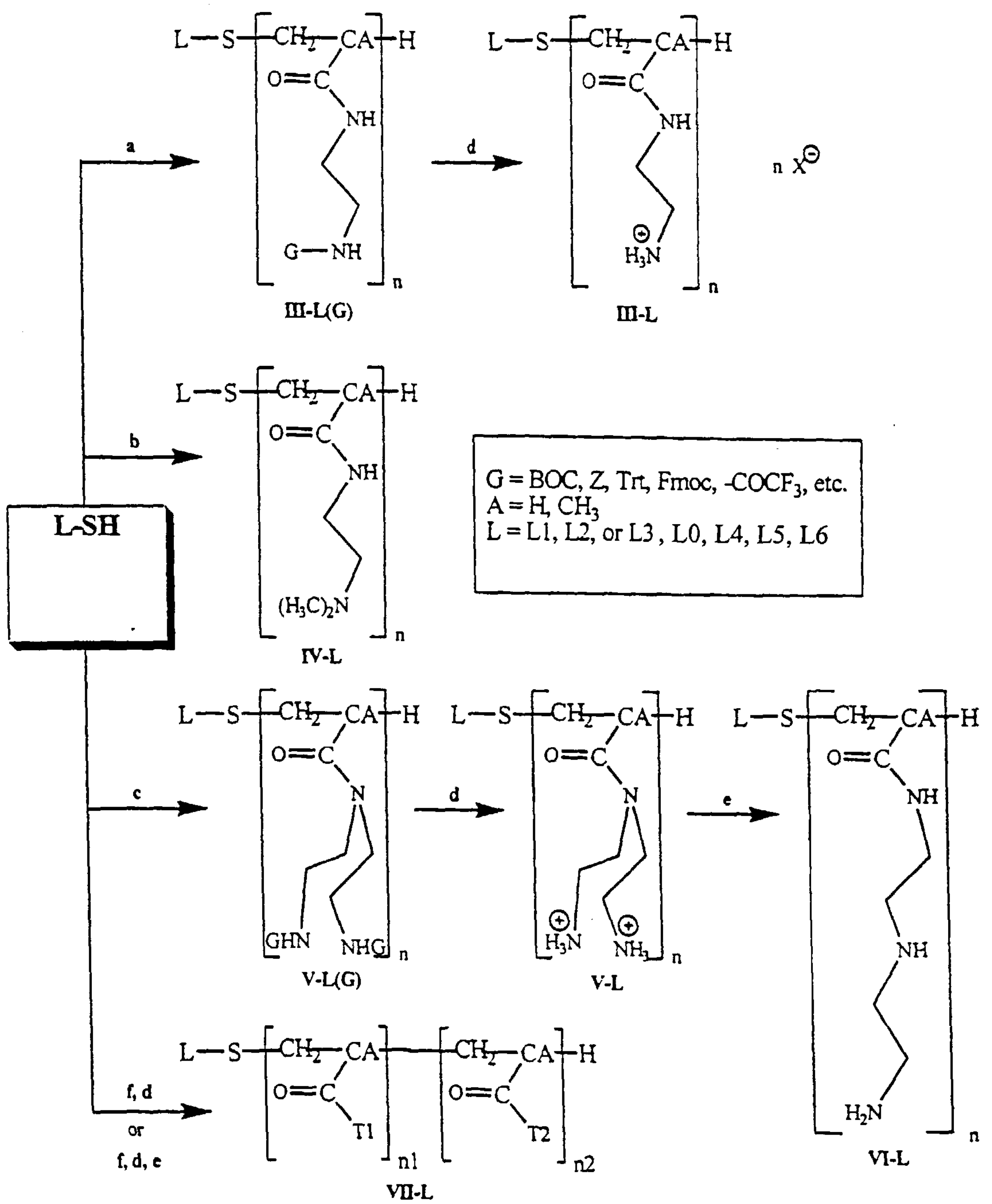


a: AIBN / $CH_3CN$ / $CH_2$=$CACONH(CH_2)_2NHG$ b: AIBN / $CH_3CN$ / $CH_2$=$CACONH(CH_2)_2NMe_2$
c: AIBN / $CH_3CN$ / $CH_2$=$CACON[(CH_2)_2NHG]_2$
d: deprotection: TFA if G = BOC, $X^-$ = $CF_3COO^-$; base if G = $COCF_3$ or Fmoc, $X^-$ = $OH^-$
e: isomerization in basic media f: AIBN / $CH_3CN$ / $CH_2$=CACOT1 and $CH_2$=CACOT2
T1, T2 = $-NH(CH_2)_2NHG$, $-NH(CH_2)_2NMe_2$, $-N[(CH_2)_2NHG]_2$

The way of synthesis of compounds 3, 6 and 9 as illustrated in Reaction Schemes 1 to 3 may for instance be used for the synthesis of telogens wherein R=$CH_3$, m=17 and r=2.

In a specific embodiment of the invention wherein A and $R^1$ to $R^6$ = H, x = 2, z = 0 and the degree of polymerization n = 1, 2, 3, 5, 6, 10, 11, 20, 30, 40, 50, 60, 70, one can refer to examples III-L1a to III-L1m, III-L2a to III-L2d, and III-L3a to III-L3e presented below.

The synthesis methods described are generally applicable to implementation and adaptation of the invention by a skilled person. Although, the polyamine telomer compounds of the present invention are not limited to those prepared by the method described above, the invention also encompasses polyamine telomer compounds obtainable by said method.

According to a further embodiment, the invention relates to a composition comprising at least one polyamine telomer compound as described above, and optionally at least one adjuvant likely to improve further formation of a complex between said polyamine telomer compound and at least one active substance, or to facilitate transport of said complex across the cell membrane and prevent endosomal degradation.

One particularly important aspect of the invention relates to a composition wherein said adjuvant is a negatively charged, neutral or zwitterionic lipid, for example a triglyceride, a diglyceride, cholesterol (see for example to US-A-5 438 044), and, in particular, a negatively charged, neutral or zwitterionic lipid which is or derivates from a phosphatidylethanolamine (PE), phosphatidylcholine, phosphocholine, sphingomyelin, ceramide or cerebroside, cationic lipid such as for example glycerolipids (see PCT/FR98/00250) or PEGylated lipids (see WO 98/08489). According to a preferred embodiment, said adjuvant is dioleylphosphatidylethanolamine (DOPE).

The ratio of polyamine telomer compound of the invention to said adjuvant (on a weight to weight basis) can range from 1:0.1 to 1:10, but this ratio may be adapted according to the type of compound. In a preferred embodiment said weight ratio is 1:1. A skilled person is capable of handling these minor adjustments. It is also possible to use a mixture of negatively charged, neutral and/or zwitterionic lipids.

From yet another perspective, the invention includes a complex comprising at least one compound or composition such as those described above and at least one active substance comprising at least one negative charge. According to the present invention, such complexes are referred to "telopolyplexes".

One particularly important aspect of the invention relates to such a complex wherein the active substance is selected from nucleic acid and protein, preferably, the active substance is a nucleic acid.

The nucleic acid may be a DNA and/or RNA fragment, single or double stranded, linear or circular, natural or synthetic, modified or not (see US-A-5525711, US-A-4711955 or EP-A-302175 for modification examples) defining a fragment or a portion of a nucleic acid, without size limitation. It may be, *inter alia*, a genomic DNA, a cDNA, a mRNA, an antisense RNA, a ribosomal RNA, a ribozyme, a tRNA or DNA encoding such RNAs. "Polynucleotides" and "nucleic acids" are synonyms with regard to the present invention. The nucleic acid may also be in the form of a plasmid or linear polynucleotide which contains at least one coding sequence of nucleic acid that can be transcribed and translated to generate polypeptide, ribozyme, antisense or other molecule of interest upon delivery to a cell. The nucleic acid can also be an oligonucleotide which is to be delivered to the cell, e.g., for antisense or ribozyme functions. According to the invention, said nucleic acid can also be formulated with viral proteins, or cationic lipids, or cationic polymers.

In a preferred embodiment, both DNA and mRNA can be delivered to cells to form therein a polypeptide of interest which may stay within the cell or which will be secreted from the cell. In a more preferred embodiment, plasmid DNA is preferred. If the nucleic acids contain the proper genetic information, they will direct the synthesis of relatively large amounts of the encoded polypeptide. When the nucleic acids delivered to the cells encode an immunizing polypeptide, the use according to the invention can be applied to achieve improved and effective immunity against infectious agents, including intracellular viruses, and also against tumor cells. The genetic information necessary for expression by a target cell comprise all the elements required for transcription of said DNA into mRNA and for translation of mRNA into polypeptide. Transcriptional promoters suitable for use in various vertebrate systems are well known. For example, suitable promoters include viral promoters RSV, MPSV, SV40, CMV or 7.5k, vaccinia promoter, inducible promoters, etc. Nucleic acids can also include intron sequences, targeting sequences, transport sequences, sequences involved in replication or integration. Said sequences have been reported in the literature and can be readily obtained by those skilled in the art. Nucleic acids can also be modified in order to be stabilized with specific components as spermine.

According to the invention, the nucleic acid can be homologous or heterologous to the target expressing cells. Advantageously said nucleic acid encodes all or part of a polypeptide, especially a therapeutic or prophylactic polypeptide. A polypeptide is understood to be any translational product of a polynucleotide regardless of size, and whether glycosylated or not, and includes peptides and proteins. Therapeutic polypeptides include as a primary example those polypeptides that can compensate for defective or deficient proteins in an animal, or those that act through toxic effects to limit or remove harmful cells from the body. They can also been immunity-conferring polypeptides which act as endogenous immunogens to provoke a humoral or cellular response, or both. Examples of encoded polypeptides according to the invention are enzyme, hormone, cytokine, membrane receptor, structural polypeptide, transport polypeptide, adhesine, ligand, transcription factor, transduction factor, replication factor, stabilisation factor, antibody, more especially CFTR, dystrophin, factors VIII or IX, E6 or E7 from HPV, MUC1, BRCA1, interferon $\beta$, interferon $\gamma$, interleukin (IL)2, IL-4, IL-6, IL-7, IL-12, GM-CSF (Granulocyte Macrophage Colony Stimulating Factor), tk gene from Herpes

Simplex type 1 virus (HSV-1), p53, VEGF. The polynucleotide can also code for an antibody. In this regard, antibody encompasses whole immunoglobulin of any class, chimeric antibodies and hybrid antibodies with dual or multiple antigen or epitope specificities, and fragments, such as $F(ab)_2$, Fab', Fab including hybrid fragments and anti-idiotypes (US-A-4,699,880).

In a preferred embodiment of the invention, the size of a cationic polyamine telomer compound/active substances complexes according to the invention is small-size (less than 500 nm, more advantageously less than 200 nm and preferably ranges between 20 and 100 nm). Complex size may be selected for optimal use in a particular application.

Measurements of the complex size can be achieved by a number of techniques including, but not limited to, dynamic laser light scattering (photon correlation spectrocopy, PCS), as well as other techniques known to those skilled in the art (see, Washington, Particle Size Analysis in Pharmaceutics and other Industries, Ellis Horwood, New York, 1992, 135-169).

The invention is also directed to methods for preparing complexes comprising cationic polyamine telomer compounds and active anionic substances. Said method is characterized in that one or more polyamine telomer compounds or compositions according to the invention is mixed with one or more active anionic substance, and that said complex is then recovered.

According to a first variant of the method, one or more cationic polyamine telomer compounds are dissolved in an appropriate quantity of solvent or in a mixture of water-miscible solvents, for example ethanol (EtOH), dimethylsulfoxide (DMSO) or preferably a 1:1 (v/v) EtOH/DMSO mixture, so as to form lipid aggregates according to the method described in patent application WO-A-9603977). In a second variant of the method, a suspension of said cationic polyamine telomer compounds is prepared using a suitable quantity of detergent solution such as an octylglucoside, e.g. n-octyl β-D-glucopyranoside or 6-0-(N-heptylcarbomoyl)-methyl-α-D-glucopyranoside. Said suspension can then be mixed with a solution of negatively-charged active substance and removal of detergent and concomitant formation of the telomer/active substance complex is achieved by dialysis or related techniques.

If the final complex is to include a neutral or zwitterionic lipid, prior to dissolving the cationic polyamine telomer compounds in the water-miscible solvent, a film is prepared, according to known methods, with a mixture incorporating a given cationic polyamine telomer and a given neutral or zwitterionic lipid such as DOPE.

Concentration of the negatively-charged active substance, preferably nucleic acid, which may be added to the cationic polyamine telomer compound or composition to form the complexes of the invention ranges from 10 μg/ml to 2000 μg/ml. In a preferred embodiment of the invention, the concentration of the negatively-charged active substance ranges from 100 μg/ml to 1000 μg/ml.

The telopolyplexes of the invention may also be characterized by their theoretical charge ratio (+/-), which is the ratio of the positive charges provided by the cationic polyamine telomer to the negative charges provided by the active substance present in the complex, assuming that all potentially cationic groups are in fact in the cationic state and all potentially anionic groups are in fact in the anionic state. In general, an excess of positive charges on the complex facilitates binding of the complex to the negatively-charged cell surface. To obtain such a ratio, the calculation shall take into account all negative charges in the active substance and shall then adjust the quantity of cationic polyamine telomer compound necessary to obtain the desired theoretical charge ratio indicated above. The quantities and the concentrations of the other ingredients shall be adjusted in function of their respective molar masses and their number of positive charges. The ratio is not specifically limited: quantities are selected so that the ratio between the number of positive charges in the cationic polyamine telomer compound or composition and the number of negative charges in the active substance is between 0.05 and 20, notably between 0.8 and 15, between 2.5 and 15, and preferably around 5 to 10.0

In the case of the second variant, and optionally, the suspension can be subsequently dialyzed in order to reduce the detergent and recuperate the complexes . The principle of such a method is for example described by Hofland et al. (1996, PNAS 93, p 7305-7309) and in chapter II of the document by Philippot et al. (G. Gregoriadis, 81-89, CRC Press 1993).

It has been demonstrated that the first variant method provides excellent results in regard to the size and reproducibility of the complexes obtained.

Using a third variant, a suspension is prepared comprising one or more cationic polyamine telomer compounds or composition and a buffer. This suspension is then subjected to sonication until being visibly homogeneous. The polyamine telomer compound suspension is then extruded, under appropriate pressure, through two micropore membranes of defined pore size. Said suspension, which can consist of liposomes, is then mixed with a negatively-charged active substance solution. This sonication-extrusion method is well known in the art.

The characteristics of the complexes thus formed can be evaluated using several methods to determine, for example:

- the state of complexation with the active substance: using gel electrophoresis to test for the free nucleic acids in

the case where the active substances are nucleic acids,

- the particle diameter: by quasi-elastic light scattering.
- the absence of precipitation over the long term.

This invention also relates to the complexes obtained via the methods listed above.

In accordance with another aspect of the invention, there is provided the use of a compound, composition or a complex according to the present invention for *in vitro, ex vivo* or *in vivo* transfer of at least one active substance, notably a nucleic acid, into cells. More especially, it relates to a method for therapeutical treatment of humans or animals.

According to the invention, "cells" includes prokaryote cells and eukaryote cells, yeast cells, plant cells, human or animal cells, in particular mammalian cells. In particular, cancer cells should be mentioned. The invention can be applied *in vivo* to the interstitial or luminal space of tissues in the lungs, the trachea, the skin, the muscles, the brain, the liver, the heart, the spleen, the bone marrow, the thymus, the bladder, the lymphatic system, the blood, the pancreas, the stomach, the kidneys, the ovaries, the testicles, the rectum, the peripheral or central nervous system, the eyes, the lymphoid organs, the cartilage, the endothelium. In preferred embodiments, the cell will be a muscle cell, a hematopoietic system stem cell or an airways cell, more especially a tracheal or pulmonary cell, and preferably a cell of the respiratory epithelium.

In accordance with this aspect of the invention, there is provided a polyamine telomer compound or composition/ active substance complex for use as medicinal preparation for curative, preventive or vaccinal purposes. Such complex can be used in a therapeutic treatment consisting of transferring at least one active substance, notably a nucleic acid, into cells.

The present invention also encompasses a process for transferring a nucleic acid into cells wherein said process comprises contacting said cells with at least one complex according to the invention. This process may be applied by direct administration of said complex to cells of the animal *in vivo*, or by *in vitro* treatment of cells which were recovered from the animal and then re-introduced into the animal body (*ex vivo* process). In *in vitro* application, cells cultivated on an appropriate medium are placed in contact with a suspension consisting of complexes of the invention. After an incubation time, the cells are washed and recovered. Introduction of the active substance can be verified (eventually after lysis of the cells) by any appropriate method.

The introduction or transfer process is by itself well known. "Introduction or transfer" means that the negatively-charged active substance is transferred into the cell and is located, at the end of the process, inside said cell or within or on its membrane. If the active substance is a nucleic acid, this is called "transfection". Transfection can be verified by any appropriate method, for example by measuring the expression of said gene or by measuring the concentration of the expressed protein.

The invention more particularly concerns the use of a compound, a composition or a complex of the invention for the made of a pharmaceutical preparation for curative, preventive or vaccinal treatment of humans or animals, and more specifically for gene therapy use.

In this particular case, the pharmaceutical preparation according to the invention may also comprise a pharmaceutically acceptable injectable carrier. The carrier is preferably isotonic, hypotonic or weakly hypertonic and has a relatively low ionic strength, such as provided by a sucrose solution. It includes any relevant solvent, aqueous or partly aqueous liquid carrier comprising sterile, pyrogen-free water, dispersion media, coatings, and/or equivalents. The pH of the pharmaceutical preparation is suitably adjusted and buffered.

For example, it is possible to prepare such a pharmaceutical preparation by solubilizing at least one compound, composition or complex according to the invention in the form of inverted micelles or emulsions in an oily injectable phase, for example a fatty acid compound or a fluorocarbon such as perfluorooctyl bromide (Perflubron®), or even solubilization in an oily phase or in a dispersed fluorocarbon in the form of an aqueous emulsion, using the methods well known by skilled persons.

The pharmaceutical preparation in accordance with the present invention can be administered into a vertebrate tissue. This administration may be made by intradermal, subdermal, intravenous, intramuscular, intranasal, intracerebral, intratracheal, intraarterial, intraperitoneal, intravesical, intrapleural, intracoronary or intratumoral injection, by means of a syringe or other devices. Transdermal administration is also contemplated, as are inhalation or aerosol routes.

According to the present invention, the pharmaceutical preparation can be administered into target tissues of the vertebrate body including those of muscle, skin, brain, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor, etc.

Applied to *in vivo* gene therapy, this invention allows repeated administration to the patient without any risk of the administered preparation to induce a significant immune reaction. Administration may be by single or repeated dose, once or several times after a certain period of time. Repeated administration allows a reduction in the dose of active substance, in particular DNA, administered at a single time. The route of administration and the appropriate dose

vary in function of several parameters, for example the individual patient, the illness being treated, or the nucleic acid being transferred.

The invention more particularly pertains to a pharmaceutical preparation comprising at least one of the complexes described above and also incorporating at least one adjuvant capable of improving the transfection capacity of said complex. Adjuvants may be selected in the group consisting in a chloroquine, protic polar compounds such as propylene glycol, polyethylene glycol, glycerol, EtOH, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives.

In the case of *in vivo* treatment according to the invention, in order to improve the transfection rate, the patient may undergo a macrophage depletion treatment prior to administration of the pharmaceutical preparations described above. Such a technique is described in the literature (refer particularly to Van Rooijen et al., 1997, TibTech, 15, 178-184).

Lastly, the invention relates to a cell transfected with a complex such as that described above, in particular a prokaryote cell, a yeast or eukaryote cell, notably a mammalian cell, and more especially a cancerous cell. The invention is particularly effective for airways cells, especially tracheal or pulmonary cells, and even more so for cells of the respiratory epithelium.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced otherwise than as specifically described.

## EXAMPLES

### Example 1: Synthesis of Polyamine Telomers of Formulas III-L1a to III-L1m, III-L2a to III-L2d, and III-L3a to III-L3e

#### 1.1 General experimental and analytical conditions

The purifications by column chromatography were carried out using silica gel 60 (Merck, 70-230 mesh) and chloroform ($CHCl_3$), dichloromethane ($CH_2Cl_2$), methanol (MeOH), diethylether ($Et_2O$), or mixtures thereof as indicated. Advancing of the polymerization reaction was followed by thinlayer chromatography (TLC) on silica plates $F_{254}$ (Merck). The following developing systems were used: UV light; $KMnO_4$; $H_2SO_4$/MeOH; Dragendorff reagents; DTNB (5,5'-dithiobis(2-nitrobenzoic acid)); ninhydrin reagent (Sigma).

IR spectra were recorded using a Bruker FT-ITS 45 spectrometer. Solid compounds were analyzed using the KBr disk method; liquids were analyzed as thin film between two KBr blocks. $^1$H, $^{13}$C and $^{19}$F NMR spectra were recorded at 200, 50.3 and 188.3 MHz, respectively, on a Bruker AC-200. Chemical shifts were measured relative to $CHCl_3$ ($\delta$ 7.27) or $CH_3OD$ ($\delta$ 3.35) for $^1$H, relative to $CDCl_3$ ($\delta$ 76.9) for $^{13}$C and expressed indirectly in relation to TMS, and relative to $CCl_3F$ as internal reference for $^{19}$F. The following abbreviations are used to describe the signal multiplicities: s (singulet), d (doublet), t (triplet), q (quadruplet), m (multiplet). Chemical shifts are expressed in ppm and listed as follows: shift in ppm (multiplicity, integration, coupling, attribution). Mass spectra (MS) were recorded on a Finningan MAT TSQ 7000 equipped with an atmospheric pressure ionisation (API) source. Electrospray ionisation (ESI) mass spectrometry was used depending on the polar moiety of the molecules to be studied. This method used in positive mode can give either a $M+H^+$ or a $M+Na^+$ signal. Molecules were also analyzed on a VG-ZAB-HF mass spectrometer in FAB mode or on a Fisons BioQ quadrupol, VG BioTech in positive ionisation electrospray mode. Elemental analyses were carried out by the Service Central de Microanalyses du CNRS.

#### 1.2 Synthesis of L-SH Telogens

##### 1.2.1 Synthesis of Telogen L1-SH, (compound 3a in Reaction scheme 1)

The synthesis of telogen 3a is carried out in two steps as described above in Reaction scheme 1.

##### 1.2.1.1 Synthesis of 3,3'-dithiobis(N,N-dioctadecyl propanamide (compound 2a in Reaction scheme 1)

To a solution of 4.0 g (7.2 mmoles) dioctadecylammonium hydrochloride (compound 1a; Fluka) and 8 ml (58 mmoles) triethylamine in 50 ml anhydrous $CHCl_3$, 0.88 g (3.6 mmoles) 3,3'-dithiodipropanoyl chloride (obtained by reaction of $SOCl_2$ with 3,3'-dithiodipropionic acid (Fluka)) in 3 ml anhydrous $CHCl_3$ were added dropwise and at room

temperature. After two days at room temperature, the reaction mixture is washed with an aqueous solution of 0.1 N HCl. The organic phase is concentrated and the concentrate is chromatographed on a silica column (eluent: $CH_2Cl_2$). 2.1 g (1.75 mmoles, 49%) 2a are obtained in form of a white solid. [TLC (hexane/$Et_2O$ 1/1, $KMnO_4$) R*f* = 0.4. IR (ν $cm^{-1}$, KBr): 1639 (CO). $^1H$ NMR ($CDCl_3$): δ 0.83 (t, 12H, $^3J$ = 6.0 Hz, $CH_3$); 0.90-1.40 (m, 120 H, $(CH_2)_{15}$); 1.40-1.65 (m, 8H, $CH_2CH_2NCO$); 2.60-2.80 (m, 4H, $CH_2CON$); 2.80-3.05 (m, 4H, $CH_2S$); 3.10-3.40 (m, 8H, $CH_2N$). $^{13}C$ NMR ($CDCl_3$): δ 14.2 ($CH_3$); 22.8 ($CH_2CH_3$); 27.1, 27.2, 27.7, 29.5, 29.6, 29.7, and 29.9 ($(CH_2)_{14}$); 32.0 ($CH_2CH_2CH_3$); 33.5 ($CH_2S$); 34.9 ($CH_2CO$); 46.5 and 48.5 ($CH_2N$); 170.4 (s, CO). MS-ESI (M+$Na^+$) m/z = 1240 (theoretical value for $C_{78}H_{156}N_2O_2S_2$ + $Na^+$ = 1239)].

### 1.2.1.2 Synthesis of compound 3a (Reaction scheme 1)

To a solution of 2.04 g (1.67 mmoles) compound 2a in 10 ml acetic acid at 60°C, 700 mg (10.7 mmoles) zinc powder are added in 4 portions during a period of 3h. The reaction mixture is stirred at 60°C for 3 h, then filtered hot. $CHCl_3$ is added and washing with $H_2O$ is carried out until neutrality is reached. The organic phase is dried over $Na_2SO_4$, then filtered and evaporated. The obtained concentrate is chromatographed on a silica column (eluent $CHCl_3$). 1.67 g (2.73 mmoles, 82%) telogen 3a are obtained in form of a white solid. [TLC (hexane/$Et_2O$ 2/3, DTNB) R*f* = 0.6; $^1H$ NMR ($CDCl_3$): δ 0.80 (t, 6H, $^3J$ = 6.0 Hz, $CH_3$); 1.00-1.35 (m, 60H, $(CH_2)_{15}$); 1.35-1.60 (m, 4H, $CH_2CH_2N$); 1.63 (t, 1H, $^3J$ = 8.0 Hz, SH); 2.55 (t, 2H, $^3J$ = 6.5 Hz $CH_2CON$); 2.65-2.85 (m, 2H, $CH_2S$); 3.05-3.35 (m, 4H, $CH_2N$). $^{13}C$ NMR ($CDCl_3$): δ 14.2 ($CH_3$); 20.8 ($CH_2S$); 22.8 ($CH_2CH_3$); 27.0, 27.2, 27.9, 29.2, 29.4, 29.5, and 29.8 ($(CH_2)_{14}$); 32.0 ($CH_2CH_2CH_3$); 37.2 ($CH_2CO$); 46.2 and 48.0 ($CH_2N$); 170.2 (CO). MS-ESI m/z = 610 (calc. for $C_{39}H_{79}NOS$ + $H^+$ = 610)].

### 1.2.2 Synthesis of Telogen L1-SH, (compound 3b in Reaction scheme 1)

The synthesis of telogen 3b is carried out in one step as described above (Reaction scheme 1). To a solution of 21 μl (0.24 mmoles) 3-mercaptopropionic acid (Aldrich) in 2 ml anhydrous $CHCl_3$, kept at 0°C, 51 mg (0.24 mmoles) dicyclohexylcarbodiimide (DCC; Aldrich), then 33 mg (0.24 mmoles) 1-hydroxy-1H-benzotriazole (HOBt, Aldrich), and 100 mg (0.24 mmoles) ditetradecylamine 1b in 8 ml anhydrous $CHCl_3$ are added. The reaction mixture is kept under stirring at room temperature for 12 h, then diluted with $CHCl_3$ and washed with $H_2O$. The organic phase is dried over $Na_2SO_4$, filtered and evaporated. The concentrate is chromatographed on a silica column (eluent $CHCl_3$). 37 mg (0.07 mmoles, 31%) telogen 3b are obtained in form of a white solid. TLC ($CH_2Cl_2$, DTNB) R*f* = 0.5. $^1H$ NMR ($CDCl_3$): δ 0.81 (t, 6H, $^3J$ = 6.0 Hz, $CH_3$); 1.00-1.35 (m, 44 H, $(CH_2)_{11}$); 1.35-1.60 (m, 4H, $CH_2CH_2N$); 1.65 (t, 1H, $^3J$ = 8.2 Hz, SH); 2.55 (t, 2H, $^3J$ = 6.5 Hz $CH_2CO$); 2.65-2.85 (m, 2H, $CH_2S$); 3.05-3.35 (m, 4H, $CH_2N$). $^{13}C$ NMR ($CDCl_3$): δ 14.2 ($CH_3$); 20.5 ($CH_2S$); 22.8 ($CH_2CH_3$); 27.0, 27.2, 27.9, 29.2, 29.4, 29.5, and 29.7 ($(CH_2)_{10}$); 32.0 ($CH_2CH_2CH_3$); 37.2 ($CH_2CO$); 46.2 and 48.0 ($CH_2NCO$); 170.2 (CO).

### 1.2.3 Synthesis of Telogen L2-SH, compound 6a (Y=Trt) (Reaction scheme 2)

The synthesis of telogen 6a (Y=Trt [= trityl]) is carried out in four steps starting from $N^\alpha$-Fmoc-im-trityl-L-histidine (Reaction scheme 2).

### 1.2.3.1 Synthesis of compound 4a (Y=Trt)(Fmoc) (Reaction scheme 2)

3.7 g (5.9 mmoles) $N^\alpha$-Fmoc-im-trityl-L-histidine (Sigma) are solubilized in 25 ml anhydrous $CHCl_3$. 0.68 g (5.9 mmoles) N-hydroxysuccinimide (NHS, Aldrich) and 1.2 g (5.9 mmoles) DCC are added together. The reaction mixture is kept, under stirring, at room temperature for 24 h. A solution of 3.3 g (5.9 mmoles) dioctadecylammonium chloride (compound 1a) and 0.59 g (5.9 mmoles) triethylamine in 50 ml anhydrous $CHCl_3$ is added dropwise at room temperature over 30 mm. The mixture is heated under $CHCl_3$ reflux for 24 h, then the solvent is evaporated. The dried reaction mixture is taken up in $CHCl_3$ and washed with $H_2O$. The organic phase is concentrated and chromatographed on a silica column (eluent: $CHCl_3$/MeOH, 98/2) to yield 3.2 g (2.8 mmoles, 48%) compound 4a (Y=Trt)(Fmoc) in the form of a colorless oil. [TLC ($CHCl_3$/MeOH, 9/1, $KMnO_4$) R*f* = 0.4. IR (ν $cm^{-1}$. KBr): 1719 (OCON); 1638 (CON).$^1H$ NMR ($CDCl_3$): δ 0.80 (t, 6H, $^3J$ = 6.0 Hz, $CH_3$); 0.97-1.35 (m, 60 H, $(CH_2)_{15}$); 1.35-1.55 (m, 4H, $CH_2CH_2NCO$); 2.70-3.20 (m, 6H, $CH_2NCO$ and $CH_2im$); 4.05-4.35 (m, 3H, $CHCH_2OCO$); 4.85 (td, 1H, $^3J$ = 8.0 Hz, $^3J$= 8.7 Hz, $CHCH_2im$); 5.80 (d, 1H, $^3J$ = 8.7 Hz, NH); 6.65 (s, 1H, CH=C (im)); 6.95-7.05 (m, 6H, Trt); 7.10-7.40 (m, 9H Trt, 4 H Fmoc, 1H CH=N); 7.50 (d, 2H, $^3J$ = 8.0 Hz, Fmoc); 7.68 (d, 2H, $^3J$ = 8.0 Hz, Fmoc). $^{13}C$ NMR ($CDCl_3$): δ 14.2 ($CH_3$); 22.7 ($CH_2CH_3$) ); 27.0, 27.2, 27.7, 29.4, 29.6, 29.7 and 29.8 ($(CH_2)_{14}$); 32.0 ($CH_2CH_2CH_3$); 33.0 ($CH_2im$); 46.5 and 48.0 ($CH_2NCO$); 47.3 ($CH_2CH$ (Fmoc)); 51.1 (CHNH); 67.1 ($CH_2OCO$); 75.2 (*C*-Ph); 119.4 (*C*H=C), 136.5 (CH=*C*), 138.7 (CH=N); 120.0, 125.4, 127.1, 127.7. (CH (Fmoc)); 128.1, 129.9 (CH (Trt)); 141.3 and 144.1 (C (Fmoc)); 142.6 (C (Trt)); 155.8 (CO (Fmoc));

171.2 (s, NCO)].

### 1.2.3.2 Synthesis of compound 4a (Y=Trt) (Reaction scheme 2)

2.9 g (2.6 mmoles) compound 4a (Y=Trt)(Fmoc) and 36 g (0.41 mole) morpholine are solubilized in 50 ml DMF. The reaction mixture is kept, under stirring, at room temperature for 2 h, is then concentrated, and chromatographed on a silica column (eluent: $CHCl_3$/MeOH, 96/4) to yield 2.0 g (2.3 mmoles, 86%) compound 4a (Y=Trt) in the form of a colorless oil. [TLC ($CHCl_3$/MeOH, 98/2, UV, $KMnO_4$) R*f* = 0.8. IR (v $cm^{-1}$. KBr): 1642 (CO). $^1H$ NMR ($CDCl_3$): δ 0.79 (t, 6H, $^3J$ = 6.0 Hz, $CH_3$); 0.95-1.30 (m, 60 H, $(CH_2)_{15}$); 1.30-1.55 (m, 4H, $CH_2CH_2NCO$); 2.55 and 2.80 (AB part of an ABX system, 2H, $^2J_{AB}$ = 14.0 Hz, $^3J_{AX}$ = 7.6 Hz and $^3J_{BX}$ = 5.6 Hz, $CH_2$im); 2.88 (m, 4H, $CH_2NCO$); 3.90 (dd, $^3J$ = 7.5 Hz, $^3J$ = 5.6 Hz, NCHCO); 6.54 (s, 1H, CH=C (im)); 7.0-7.15 and 7.20-7.26 (m, m, 6H, 9H, Trt); 7.29 (s, 1H, CH=N (im)). $^{13}C$ NMR ($CDCl_3$): δ 14.2 ($CH_3$); 22.7 ($CH_2CR_3$) ); 27.0, 27.2, 27.7, 29.4, 29.6, 29.7, 29.8 and 29.9 ($(CH_2)_{14}$); 32.0 ($CH_2CH_2CH_3$); 35.8 ($CH_2$im); 46.5 and 47.7 ($CH_2N$); 51.4 (CHN); 75.2 (C-Ph); 119.5 (*C*H=C), 137.6 (CH=*C*), 138.8 (CH=N); 128.1 and 129.8 (CH (Trt)); 142.5 (C (Trt)); 174.5 (CO)].

### 1.2.3.3 Synthesis of compound 5a (Y=Trt)(Reaction scheme 2)

The protocol described above for compound 2a, was applied to 0.27 g (1.13 mmoles) 3,3'-dithiodipropionylchloride, 2.04 g (2.26 mmoles) compound 4a (Y=Trt), 0.91 g (9.06 mmoles) triethylamine and 15 ml $CHCl_3$. 1.52 g (0.77 mmoles, 34 %) compound 5a (Y=Trt) were obtained in the form of a white solid. [TLC ($CHCl_3$/MeOH, 98/2, UV, $KMnO_4$) R*f* = 0.3. IR (v cm-1. KBr): 1631 (CO). $^1H$ NMR ($CDCl_3$): δ 0.80 (t, 6H, $^3J$ = 6.0 Hz, $CH_3$); 0.95-1.30 (m, 60H, $(CH_2)_{15}$); 1.30-1.70 (m, 4H, $CH_2CH_2NCO$); 2.1-3.4 (m, 10H, $CH_2S$, $CH_2CO$, $CH_2N$ and $CHCH_2$); 4.99 (m, 1H, CHCO); 6.49-6.60 (m, 1H, CH=C (im [= imidazole])); 7.00-7.15 (m, 6H, Trt); 7.15-7.35 (m, 10H, Trt and CH=N). $^{13}C$ NMR ($CDCl_3$): δ 14.2 ($CH_3$); 22.7 ($CH_2CH_3$); 27.0, 27.2, 27.7, 29.4, 29.7 and 29.8 ($(CH_2)_{14}$); 32.0 ($CH_2CH_2CH_3$); 33.6 ($CH_2S$); 34.7 ($CH_2CO$); 35.8 and 35.9 ($CH_2$im); 46.3 and 48.0 ($CH_2NCO$); 49.7 (CHN); 75.2 (NC (Trt)); 119.4 and 119.6 (*C*H=C); 136.6 and 136.8 (CH=*C*); 138.4 (CH=N); 128.0, 129.8 (CH (Ph)); 142.5 and 142.6 (C (Ph)); 169.9, 170.9 and 171.7 (CO) (the splitting up of certain signals results from the fact that compound 5a (Y=Trt) is a mixture of diastereoisomers and cis/trans isomers at the level of CONH)].

### 1.2.3.4 Synthesis of compound 6a(Y=Trt)(Reaction scheme 2)

To 1.01 g (0.51 mmoles) comopund 5a (Y=Trt) solubilized in 7 ml anhydrous THF, 0.24 g (6.1 mmoles) $NaBH_4$ are added. The reaction mixture is maintained under stirring and THF reflux for 48 h. After cooling to room temperature several drops of $H_2O$ are added. The reaction mixture is concentrated to dryness, the residue is taken up in $CHCl_3$ and washed with aqueous 0.05N HCl. The organic phase is dried and concentrated. After purification by silica column chromatography (eluent: $CHCl_3$/MeOH 98/2), 0.88 g (0.44 mmoles, 87%) compound 6a (Y=Trt) are obtained in the form of a colorless oil. [TLC ($CHCl_3$/MeOH, 96/4, UV, DTNB) R*f* = 0.8. $^1H$ NMR ($CDCl_3$): δ 0.79 (t, 6H, $^3J$ = 6.0 Hz, $CH_3$); 0.90-1.80 (m, 64H, $(CH_2)_{16}$); 1.80-3.80 (m, 10H, $CH_2$-im, $CH_2S$, $CH_2CO$ and $CH_2N$); 4.90-5.20 (m, 1H, CHCO); 6.40-6.70 (m, CH=C); 6.90-7.10 (m, 6H, Trt); 7.10-7.30 (m, 10 H, Trt and CH=N).$^{13}C$ NMR ($CDCl_3$): δ 14.2 ($CH_3$); 20.5 ($CH_2SH$); 22.8 ($CH_2CH_3$); 26.9, 27.7, 29.7, 29.8 and 29.9 ($(CH_2)_{14}$); 32.0 ($CH_2CH_2CH_3$); 33.5 ($CH_2CO$); 46.3 and 46.5 ($CH_2N$); 48.1 and 48.4 (CHN); 76.9 (NC (Trt)); 121.0 and 122.3 (*C*H=C); 134.0, 134.2 and 134.4 (*C*=CH); 138.0 and 138.3 (C=N); 128.1, 128.5, 128.7, 129.7 and 129.8 (CH (Ph)); 140.9 (C (Ph)); 169.8, 171.1, 172.3 and 172.6 (CO). MS-ESI: m/z = 989; [theoretical mass for M = [$C_{64}H_{100}N_4O_2S$] + $H^+$ = 989)].

### 1.2.4 Synthesis of Telogen L2-SH, compound 6b (Y=BOC) in Reaction scheme 2

6b (Y=BOC) is obtained in three steps from $N^{\alpha}$-Fmoc-im-BOC-L-histidine.

### 1.2.4.1 Synthesis of compound 4b (Y=BOC)(Fmoc) (Reaction scheme 2)

To a solution of 500 mg (1.04 mmoles) $N^{\alpha}$-Fmoc-im-BOC-L-histidine (Bachem) in 1 ml anhydrous $CHCl_3$, are added 0.15 ml (1.04 mmoles) triethylamine and 488 mg (1.04 mmoles) PyBroPn® (Novabiochem). Then 1.09 g (2.09 mmoles) dioctadecylammonium hydrochloride (compound 1) solubilized in 15 ml anhydrous $CHCl_3$ are added. The reaction mixture is kept, under stirring, at room temperature for 4 h and is then concentrated. The dried reaction mixture is taken up in $CHCl_3$ and washed with $H_2O$. The organic phase is evaporated, and the residue is extracted with $Et_2O$. The filtrate is concentrated and chromatographed on a silica column (eluent: $CHCl_3$). The yield is 550 mg (0.56 mmoles, 53%) compound 4b (Y=BOC)(Fmoc) in the form of a pale yellow oil. [TLC ($CHCl_3$/MeOH, 99/1, $KMnO_4$): R*f* = 0.7. $^1H$ NMR ($CDCl_3$): δ 0.84 (t, 6H, $^3J$ = 6.0 Hz, $CH_3$); 1.05-1.50 (m, 64 H, $(CH_2)_{16}$); 1.50-1.80 (m, 9H, $CH_3$ (BOC)); 2.75-3.70

(m, 6H, $CH_2NCO$ and $CH_2im$); 4.10-4.45 (m, 3H, $CHCH_2$ OCO); 4.94 (td, 1H, $^3J$ =8.0Hz, $^3J$ = 8.7 Hz, $CHCH_2im$); 5.90 (d, 1H, $^3J$ = 8.7 Hz, NH); 7.15 (s, 1H, CH=C (im)); 7.05-7.45 (m, 4H Fmoc); 7.55 (d, 2H, $^3J$ = 8.0 Hz, Fmoc); 7.70 (d, 2H, $^3J$ = 8.0 Hz, Fmoc); 7.96 (s, 1H, CH=N (im)). $^{13}C$ NMR ($CDCl_3$): δ 14.2 ($CH_3$); 22.7 ($CH_2CH_3$) ); 27.9 ($CH_3$ (BOC)); 27.0, 27.1, 27.7, 29.5 and 29.8 ($(CH_2)_{14}$); 32.0 ($CH_2CH_2CH_3$); 33.0 ($CH_2im$); 46.4 and 47.3 ($CH_2NCO$); 48.0 ($CH_2CH$ (Fmoc)); 51.1 (CHNH); 67.2 ($CH_2OCO$); 85.4 (*C* (BOC)); 114.8 (*C*H=C), 136.8 (CH=*C*), 139.0 (CH=N); 120.0, 125.3, 127.1, 127.7. (CH (Fmoc)); 141.3 and 144.1 (C (Fmoc)); 147.0 (CO (BOC)); 155.8 (CO (Fmoc)); 171.2 (NCO)].

**1.2.4.2 Synthesis of compund 4b (Y=BOC) (Reaction scheme 2)**

390 mg (0.39 mmoles) compound 4b (Y=BOC)(Fmoc) and 3.6 ml (60 mmoles) morpholine are solubilized in 7 ml DMF. The reaction mixture is kept, under stirring, at room temperature for 3 h, then concentrated. The residue is taken up in $Et_2O$ and filtered. The $Et_2O$ phase is washed with $H_2O$, evaporated and chromatographed on a silica column (eluent: $CHCl_3$ then $CHCl_3$/MeOH: 99/1). 187 mg (0.24 mmoles, 62%) compound 4b (Y=BOC) are obtained in the form of a pale yellow oil. [TLC ($CHCl_3$/MeOH, 9/1, UV, ninhydrine) R*f* = 0.5.$^1H$ NMR($CDCl_3$): δ 0.80 (t, 6H, $^3J$ = 6.0 Hz, $CH_3$); 1.05-1.30 (m, 60 H, $(CH_2)_{15}$); 1.30-1.45 (m, 4H, $CH_2CH_2NCO$); 1.45-1.60 (m 9H, $CH_3$ (BOC)); 2.50 and 2.85 (AB part of an ABX system, 2H, $^2J_{AB}$ = 4.0 Hz, $^3J_{AX}$ = 8.0 Hz and $^3J_{BX}$ = 5.3 Hz, $CH_2im$); 2.90-3.55 (m, 4H, $CH_2NCO$); 3.90 (dd, $^3J$ = 8.0 Hz, $^3J$ = 5.3 Hz, NCHCO); 7.10 (s, 1H, CH=C (im)); 7.98 (s, 1H, CH=C (im)].

**1.2.4.3 Synthesis of compound 6b (Y=BOC) (Reaction scheme 2)**

To a solution of compound 4b (Y=BOC) in 1.5 ml (0.15 mmoles) anhydrous $CH_2Cl_2$, kept at 0°C, 14 µl (0.15 mmoles) 3-mercaptopropionic acid, 23 mg (0.17 mmoles) HOBt and 33 mg (0.17 mmoles) 1-(3-dimethylaminopropyl)-3-ethylcarbodiimide hydrochloride (EDC, Aldrich) are added. The reaction mixture is kept under stirring at room temperature for 12 h, is then diluted with $CHCl_3$ and washed successively with an aqueous solution of 8% $NaHCO_3$, then with $H_2O$. The organic phase is dried over $Na_2SO_4$, filtered, and evaporated. The obtained residue is chromatographed on a silica column (eluent $CH_2Cl_2$ to $CH_2Cl_2/Et_2O$: 9/1). 31 mg (0.03 mmoles, 23%) compound 6b (Y=BOC) are obtained in the form of a pale yellow oil. [TLC ($CHCl_3$/MeOH, 98/2, UV, DTNB, $KMnO_4$) R*f* = 0.4. $^1H$ NMR ($CDCl_3$): δ 0.80 (t, 6H, $^3J$ = 6.0 Hz, $CH_3$); 1.05-1.45 (m, 64H, $(CH_2)_{16}$); 1.45-1.75 (m, 9H, $CH_3$ (BOC)); 2.35-2.55 (m, 2H, $CH_2CO$); 2.55-3.50 (m, 8H, $CH_2$-im, $CH_2SH$ and $CH_2N$); 5.00-5.30 (m, 1H, CHCO); 6.70-6.85 (m, 1H, NH); 7.10 (s, CH=C); 7.95 (s, CH=N). $^{13}C$ NMR ($CDCl_3$): δ 14.2 ($CH_3$); 20.4 ($CH_2SH$); 22.8 ($CH_2CH_3$); 27.9 ($CH_3$ (BOC)); 26.9, 27.1, 27.6. 29.4, and 29.8 ($(CH_2)_{14}$); 32.0 ($CH_2CH_2CH_3$); 32.1 ($CH_2im$); 40.4 ($CH_2CO$); 46.4 and 48.0 ($CH_2N$); 48.8 (CHN); 85.5 (C (BOC)); 114.7 (*C*H=C); 136.8 (*C*=CH); 138.8 (C=N);; 147.5 (CO (BOC)); 170.0, 170.8 (CO)].

**1.2.5 Synthesis of telogen L2-SH (compound 12 in Reaction scheme 2B)**

**1.2.5.1 Synthesis of compound 10 (Reaction scheme 2B)**

To 100 mg (0.31 mmoles) Boc-Asp(OBzl)-OH (Bachem) in 2 ml anhydrous $CH_2Cl_2$, 126 mg (0.31 mmoles) 1b, 0.12 ml (0.92 mmoles) anhydrous $NEt_3$ and 144 mg (0.31 mmoles) PyBrop® (Novabiochem) are added. The reaction is kept under stirring and reflux for 24 h and then concentrated. The residue is taken up in $CHCl_3$ and washed successively with 5% $KHSO_4$, 8% $NaHCO_3$ then with $H_2O$. The organic phase is dried over $Na_2SO_4$, filtered and evaporated. The residue is chromatographed on a silica column (eluent: $CHCl_3$ to $CHCl_3/CH_3OH$: 99/1). 156 mg (0.22 mmoles, 71%) compound 10 are obtained in the form of a colorless oil.
TLC ($CHCl_3/CH_3OH$, 85/15, ninhydrine) R*f* = 0.8. $^1H$ NMR ($CDCl_3$) : δ 0.80 (t, 6H, $^3J$=6.0 Hz, $CH_3$); 1.05-1.35 (m, 44H, $(CH_2)_{11}$); 1.35-1.60 (m, 13H, $CH_3$(BOC) and $CH_2CH_2N$); 2.55 and 2.76 (AB part of an ABX system, 2H, $^2J$ = 15.6 Hz, $^3J$ = 7.0 Hz, $^3J$ = 6.0 Hz, $CH_2CH$); 3.00-3.40 (m, 4H, $CH_2N$); 4.80-4.95 (m, 1H, CH); 5.02 (s, 2H, $CH_2Ph$); 5.32 (d, 1H, $^3J$ = 9.4 Hz, NH); 7.25 (s, 5H, Ph). $^{13}C$ NMR ($CDCl_3$) : δ 14.2 ($CH_3$); 22.7 ($CH_2CH_3$); 28.3 ($CH_3$(BOC)); 26.9, 27.0, 27.5, 29.3, 29.4, 29.6 and 29.7 ($(CH_2)_{11}$); 32.0 ($CH_2CH_2CH_3$); 38.4 ($CH_2CO$); 46.4, 47.4 and 48.0 ($CH_2N$ and $CH_2Ph$); 66.6 (CH); 79.9 (C(BOC)); 128.2, 128.3 and 128.5 (CH=CH); 135.8 (*C*=CH); 154.9 (CO(BOC)); 170.3 ($CO_2$); 170.5 (CON).

**1.2.5.2 Synthesis of compound 11 (Reaction scheme 2B)**

156 mg (0.22 mmoles) compound 10 are stirred with 4.2 ml (54.5 mmoles) TFA for 1 h at room temperature. The excess of TFA is removed by evaporation in the presence of cyclohexane. 126 mg (0.22 mmoles, 100%) compound 11 are obtained in the form of a colorless oil.
TLC ($CHCl_3/CH_3OH$, 95/5, ninhydrine) Rf = 0.6. $^1H$ NMR ($CDCl_3$) : δ 0.83 (t, 6H, $^3J$ = 6.0 Hz, $CH_3$); 1.05-1.75 (m, 48H, $(CH_2)_{12}$); 2.70-3.60 (m, 6H, $CH_2N$ and $CH_2CH$); 4.55-4.75 (m, 1H, CH); 5.08 (m, 2H, $CH_2Ph$); 7.28 (s, 5H, Ph). $^{13}C$ NMR ($CDCl_3$): δ 14.2 ($CH_3$); 22.8 ($CH_2CH_3$); 26.9, 27.1, 27.3, 28.8, 29.3, 29.5, 29.6 and 29.8 ($(CH_2)_{11}$); 32.0

($CH_2CH_2CH_3$); 35.4 ($\mathit{C}H_2CO$); 46.7, 47.3 and 48.0 ($CH_2N$ and $CH_2Ph$); 67.7 (CH); 128.7 (CH=CH); 135.1 (*C*=CH); 161.9 (q, $^1J_{CF}$=35 Hz, $CF_3CO_2^-$); 167.1 ($CO_2$); 169.7 (CON).

### 1.2.5.3 Synthesis of compound 12 (Reaction scheme 2B)

To 154 mg (0.21 mmoles) compound 11 are added 0.1 ml (0.72 mmoles) anhydrous $NEt_3$ and 1.5 ml (0.31 mmoles) of a solution of 0.18 ml 3-mercaptopropionic acid (Aldrich) in 10 ml anhydrous $CH_2Cl_2$. The solution is kept at 0°C under nitrogen atmosphere and then 45 mg (0.33 mmoles) HOBt are added. After 5 min, 64 mg (0.33 mmoles) EDC and 1 ml anhydrous $CH_2Cl_2$ are added. The reaction mixture is left under stirring, at room temperature, for 17 h and then concentrated. The residue is taken up in $CHCl_3$ and washed successively with an aqueous solution of 8% $NaHCO_3$, 5% $KHSO_4$ and then with $H_2O$. The organic phase is dried over $Na_2SO_4$, filtered and evaporated. The residue is chromatographed on a silica column (eluent : $CH_2Cl_2$/hexane : 8/2 to $CH_2Cl_2$) giving 55 mg of the starting compound 8 (36%) and 65 mg (0.09 mmoles; 43%) compound 12 as a colorless oil.
TLC ($CHCl_3$, ninhydrine, DTNB) R*f* = 0.2. $^1H$ NMR ($CDCl_3$): $\delta$ 0.80 (t, 6H, $^3J$ = 6.0 Hz, $CH_3$); 1.05-1.75 (m, 48H, $(CH_2)_{12}$); 1.49 (t, 1H, $^3J$ = 8.2 Hz, SH); 2.39 (t, 2H, $^3J$ = 6.5 Hz, $CH_2CONH$); 2.50-2.90 (m, 4H, $CH_2SH$ and $\mathit{C}H_2CH$); 3.00-3.45 (m, 4H, $CH_2N$); 5.02 (s, 2H, $CH_2Ph$); 5.22 (dd, 1H, $^3J$=15.0 Hz, $^3J$=6.5 Hz, CH); 6.90-7.05 (m, 1H, NH); 7.26 (s, 5H, Ph). $^{13}C$ NMR ($CDCl_3$) : $\delta$ 14.2 ($CH_3$); 20.4 ($CH_2SH$); 22.8 ($\mathit{C}H_2CH_3$); 26.9, 27.1, 29.3, 29.5 and 29.8 ($(CH_2)_{11}$); 32.0 ($\mathit{C}H_2CH_2CH_3$); 38.2 ($\mathit{C}H_2CO_2$); 40.2 ($CH_2CONH$); 46.0, 46.6 and 48.2 ($CH_2N$ and $CH_2Ph$); 66.8 (CH); 128.4, 128.5 and 128.6 (CH=CH); 135.7 (*C*=CH); 170.1 and 170.3 (CO).

### 1.2.6. Synthesis of telogen L3-SH, compound 9 in Reaction scheme 3

Telogen 9 was obtained in three steps from *rac*-3-mercaptopropane-1,2-diol.

### 1.2.6.1 Synthesis of rac-3-benzylthiopropane-1,2-diol (compound 7 in Reaction scheme 3)

3.2 g (29 mmoles) *rac*-3-mercaptopropane-1,2-diol (Aldrich) are dissolved in a mixture of 28 ml 2N NaOH and 35 ml EtOH. To this solution, kept at 0°C, 5.6 g (44 mmoles) benzylchloride are added dropwise. After complete reaction of the thiol (TLC), the pH is ajusted to 5 with HCl, and the solvent is evaporated. The residue is purified by destillation (bp.: 150°C, 0.4 mm Hg) to yield 3.9 g (20 mmoles, 68 %) 7 in the form of a colorless oil. [TLC ($CHCl_3$/MeOH 98/2, DTNB, UV) R*f* = 0.3. $^1H$ NMR ($CDCl_3$): $\delta$ 2.59 (d, 2H, $^3J$ = 6.0 Hz, $\mathit{C}H_2SBn$ [Bn = benzyl]); 3.45-3.78 (m, 2H, $CH_2O$); 3.78-3.91 (m, 3H, CH and $S\mathit{C}H_2Ph$); 7.26-7.50 (m, 5H, $C_6H_5$). $^{13}C$ NMR ($CDCl_3$): $\delta$ 34.5 ($\mathit{C}H_2SBn$); 36.5 ($S\mathit{C}H_2Ph$); 65.3 ($CH_2O$); 70.7 (CH); 127.2 and 128.9 (C ortho and para); 128.6 (C meta); 138.0 (quaternary C)].

### 1.2.6.2 Synthesis of rac-1.2-dioctadecyloxy-3-benzylthio-propane (compound 8 in Reaction scheme 3)

3.9 g (20 mmoles) compound 7 and 15 g (43 mmoles) stearylmesylate are solubilized in 30 ml $Et_2O$. [stearylmesylate is obtained by reaction of mesylchloride with 1-octadecanol in pyridine: TLC ($CH_2Cl_2$, $H_2SO_4$, UV) R*f* = 0.6. $^1H$ NMR ($CDCl_3$): $\delta$ 0.85 (t, 3H, $^3J$ = 5.8 Hz, $CH_3$); 1.15-1.55 (m, 30H, $(CH_2)_{15}$); 1.65-1.85 (m, 2H, $\mathit{C}H_2CH_2OMs$); 2.97 (s, 3H, $CH_3S$); 4.19 (t, 2H, $^3J$ = 6.6 Hz, $CH_2OMs$). $^{13}C$ NMR($CDCl_3$): $\delta$ 14.1 ($CH_3$); 22.7 ($\mathit{C}H_2CH_3$); 25.4 ($\mathit{C}H_2CH_2CH_2O$); 29.0 ,29.2, 29.3, 29.4, 29.5, 29.6 and 29.7 ($(CH_2)_{13}$); 31.9 ($\mathit{C}H_2CH_2CH_3$); 37.4 ($CH_3S$); 70.2 ($CH_2OMs$)].To this solution 0.94 g (2.8 mmoles) tetrabutylammonium hydrogenosulfate (Aldrich) and 25 ml of an aqueous solution of 9N KOH are added. The reaction mixture is kept under $Et_2O$ reflux for 96 h. The organic phase is washed with $H_2O$, then evaporated, and the residue is purified by chromatography on a silica column (eluent: petrole ether/$Et_2O$, 95/5). 10.5 g (15 mmoles, 76 %) compound 8 are obtained in the form of a white solid. [TLC (petrole ether/$Et_2O$, 95/5, $H_2SO_4$) R*f* = 0.8. $^1H$ NMR ($CDCl_3$): $\delta$ 0.82 (t, 6H, $^3J$ = 6.0 Hz, $CH_3$); 1.10-1.36 (m, 60H, $(CH_2)_{15}$); 1.39-1.62 (m, 4H, $\mathit{C}H_2CH_2O$); 2.40-2.68 (m, 4H, $\mathit{C}H_2SBn$); 3.30-3.52 (m, 7H, $CH_2O$ and CHO); 3.68 (s, 2H, $S\mathit{C}H_2Ph$); 7.11-7.35 (m, 5H $C_6H_5$). $^{13}C$ NMR($CDCl_3$): $\delta$ 14.1 ($CH_3$); 22.7 ($\mathit{C}H_2CH_3$); 26.2 ($CH_2$ $\gamma$ O); 29.4; 29.5; 29.7; 29.8 and 30.1 ($(CH_2)_{13}$); 32.0 ($\mathit{C}H_2CH_2CH_3$); 32.9 ($\mathit{C}H_2SBz$); 37.1 ($S\mathit{C}H_2Ph$);70.4; 71.6 and 71.8 ($CH_2O$); 78.6 (CHO); 126.9 and 129.0 (C ortho and meta); 128.4 (C para); 138.6 (quarternary C)].

### 1.2.6.3 Synthesis of compound 9 in Reaction scheme 3

2.3 g (3.3 mmoles) compound 8 are solubilized in 35 ml anhydrous tBuOH (tertiary butanol). 0.86 g (38 mmoles) Na are added to this solution.The reaction mixture is heated (70°C) under reflux for 12 h. Then several drops of $H_2O$ are added. After evaporation of the solvent, the residue is taken up in hexane, then washed with $H_2O$. The organic phase is concentrated and the residue is purified by chromatography on a silica column (eluent: $CHCl_3$) to yield 1.6 g (2.6 mmoles, 78 %) compound 9 in the form of a white solid. [TLC ($CHCl_3$, DTNB) R*f* = 0.7. $^1H$ NMR ($CDCl_3$): $\delta$

0.80 (t, 6H, $^3J$ = 6.0 Hz, $CH_3$); 1.00-1.37 (m, 60H, $(CH_2)_{15}$); 1.37-1.70 (m, 4H, $CH_2CH_2O$); 2.48-2.76 (m, 2H, $CH_2S$); 3.27-3.65 (m, 7H, $CH_2O$ and CH). $^{13}C$ NMR($CDCl_3$): δ 14.0 ($CH_3$); 22.6 ($CH_2CH_3$); 26.1 ($CH_2S$); 29.3, 29.4, 29.6, 29.7, 30.0 and 31.9 ($(CH_2)_{15}$); 70.3, 71.0 and 71.6 ($CH_2O$); 79.3 (CH)].

### 1.2.7 Synthesis of telogen L4-SH (6-deoxy-6-thio-1,2:3,4-di-O-isopropylidene-D-galactopyranose)

#### 1.2.7.1 Synthesis of 6-deoxy-6-iodo-1,2:3,4-di-O-isopropylidene-D-galactopyranose

This synthesis has been performed as described in P.J. Garegg and B. Samuelsson, JCS Perkin I, 1980, 2866-2869.

To a solution of 1.2 g (4.7 mmoles) 1,2:3,4-di-O-isopropylidene-D-galactopyranose (Aldrich) in 120 ml toluene are added 3.7 g (14 mmoles) triphenylphosphine (Aldrich), 1.0 g (14 mmoles) imidazol (Aldrich) and 2.4 g (9.4 mmoles) iodine (Aldrich). The reaction mixture is heated under reflux for 3.5 h until 1,2:3,4-di-O-isopropylidene-D-galactopyranose has disappeared (followed by TLC/$H_2SO_4$). The mixture is stirred with saturated aqueous $NaHCO_3$ for 5 mm. Iodine is then added until coloration of the organic phase. The mixture is stirred for 15 mm and then aqueous $Na_2S_2O_3$ is added until decoloration. The reaction mixture is washed whit $H_2O$ and the organic phase is dried over $Na_2SO_4$, filtered and evaporated. The residue is extracted with $Et_2O$. The filtrate is concentrated and chromatographed on a silica column (eluent : petroleum ether to petroleum ether/$Et_2O$, 7/3). 1.6 g (4.2 mmoles, 89%) of 6-deoxy-6-iodo-1,2:3,4-di-O-isopropylidene-D-galactopyranose are obtained in the form of a pale yellow solid.
TLC (petroleum ether /$Et_2O$ 1/1, $H_2SO_4$) R*f* = 0.9. $^1H$ NMR ($CDCl_3$) : δ 1.25, 1.27, 1.36, 1.46 (4s, 12H, $CH_3$); 3.12 (A part of an ABX system, 1H, $^2J$ = 9.9 Hz, $^3J$ = 6.9 Hz, $GalH_6$); 3.24 (B part of an ABX system, 1H, $^3J$ = 6.9 Hz, $GalH_6$); 3.85 (ddd, 1H, $^3J$ = 6.9 Hz, $^3J$ = 6.9 Hz, $^3J$= 1.8 Hz, $GalH_5$); 4.22 (dd, 1H, $^3J$ = 5.0 Hz, $^3J$ = 2.5 Hz, $GalH_2$); 4.32 (dd, 1H, $^3J$ = 7.8 Hz, $^3J$ = 1.8 Hz, $GalH_4$); 4.53 (dd, 1H, $^3J$ = 7.8 Hz, $^3J$ = 2.5 Hz, $GalH_3$); 5.46 (d, 1H, $^3J$ = 5.0 Hz, $GalH_1$). $^{13}C$ NMR ($CDCl_3$): δ 2.3 ($GalC_6$); 24.4, 24.8, 25.9 and 26.0 ($CH_3$); 88.9 ($GalC_4$); 70.5 ($GalC_3$); 71.1 ($GalC_2$); 71.5 ($GalC_5$); 96.6 ($GalC_1$); 108.8 and 109.5 ($C(CH_3)_2$).

#### 1.2.7.2 Synthesis of L4-SH, 6-deoxy-6-thio-1,2:3,4-di-O-isopropylidene-D-galactopyranose

A solution of 200 mg (0.54 mmoles) 6-deoxy-6-iodo-1,2:3,4-di-O-isopropylidene-D-galactopyranose and 123 mg (1.62 mmoles) thiourea (Aldrich), in 1 ml $CH_3CN$ and 1 ml DMF, is heated (110°C) for 36 h, until the iodo derivative has been consumed (followed by TLC/$HSO_4$). A solution of 50 mg (1.25 mmoles) NaOH in 0.5 ml $H_2O$ is then added and the resultin reaction mixture is heated (100°C) for 3 h. After evaporation of the solvent, the residue is taken up in $CHCl_3$, washed with an aqueous solution of $KHSO_4$ (pH 5) and with $H_2O$. The organic phase is dried over $Na_2SO_4$, filtered and evaporated. The concentrate is chromatographed on a silica column (eluent: hexane/$Et_2O$, 95/5, to $Et_2O$) yielding 128 mg (0.23 mmoles, 83%) of the disulfide corresponding to 6-deoxy-6-thio-1,2,3,4-di-O-isopropylidene-D-galactopyranose as a white solid. This disulfide with 107 mg (0.69 mmoles) DTT (Aldrich), and 0.1 ml (0.69 mmoles) anhydrous $NEt_3$, in 1 ml anhydrous $CHCl_3$, is left under dinitrogen during 2.5 h. The reaction mixture is concentrated, taken up in $CHCl_3$, washed with an aqueous solution of $KHSO_4$ (pH=5) and with $H_2O$. The organic phase is dried over $Na_2SO_4$, filtered and evaporated. The residue is chromatographed on a silica column (eluent: petroleum ether to petroleum ether/$Et_2O$ : 95/5). 107 mg (0.38 mmoles, 72%) 6-deoxy-6-thio-1,2 :3,4-di-O-isopropylidene-D-galactopyranose are obtained as a colorless oil.
TLC (petroleum ether/$Et_2O$, 7/3, DTNB, $H_2SO_4$) R*f* = 0.6. $^1H$ NMR ($CDCl_3$) : δ 1.22, 1.24, 1.33, 1.43 (s, 12H, $CH_3$); 1.48-1.56 (m, 1H, SH); 2.48-2.73 (m, 2H, $GalH_6$); 3.67 (t, 1H, $^3J$ = 6.9 Hz, $GalH_5$); 4.19-4.26 (m, 2H, $GalH_2$ and $GalH_4$); 4.52 (dd, 1H, $^3J$ = 7.8 Hz, $^3J$ = 2.5 Hz, $GalH_3$); 5.42 (d, 1H, $^3J$ = 5.0 Hz, $GaH_1$). $^{13}C$ NMR ($CDCl_3$) : δ 24.2 and 24.3 ($CH_3$); 24.8 ($GalC_6$); 25.8 and 25.9 ($CH_3$); 69.7 ($GalC_4$); 70.4 ($GalC_3$); 70.7 ($GalC_2$); 71.1 ($GalC_5$); 96.4 ($GalC_1$); 108.5 and 109.1 ($C(CH_3)_2$).

### 1.3.1 Synthesis of Taxogen A (N-{2-[(BOC)aminoethyl]} acrylamide)

The synthesis of taxogen A is carried out as described below.

A solution of 1.7 g (18.7 mmoles) acryloyl chloride (Aldrich) in 5 ml anhydrous $CHCl_3$ is added dropwise, under a nitrogen atmosphere, at 0°C and during 30 mm, to 2.0 g (12.4 mmoles) N-(BOC)-ethylene diamine (Fluka) and 5.0 g (49,1 mmoles) triethylamine in 35 ml anhydrous $CHCl_3$. The reaction mixture is left, under stirring, for 90 mm at room temperature, then concentrated to dryness. After purification on a silica column (eluent: $CHCl_3$/MeOH 98/2), one obtains 2.0 g (9,2 mmoles, 75%) taxogen A as a white solid. TLC ($CHCl_3$/MeOH, 98/2. $KMnO_4$) R*f* = 0.2. $^1H$ NMR ($CDCl_3$): δ 1.36 (m, 9H, $CH_3$); 3.10-3.34 (m, 2H, $CH_2NH$); 3.34-3.50 (m, 2H, $NCH_2$); 5.55 (dd,1H, $^3J$ = 9,6 Hz, $^2J$ = 2.2 Hz, CH=$CH_2$); 6.05 (dd, 1H, $^3J$ = 9,6 Hz, $^3J$ = 17.0 Hz, CH=$CH_2$); 6.20 (dd, 1H, $^3J$ = 17.0 Hz, $^2J$ = 2.2 Hz, CH=$CH_2$). $^{13}C$ NMR ($CDCl_3$): δ 28.3 ($CH_3$); 40.1 and 40.7 ($CH_2N$); 79.6 (C (BOC)); 126.1 ($CH_2$=CH); 130.9 (CH=$CH_2$); 157.0

(CO(BOC)); 166.3 (*C*OCH). Elemental analysis: theoretical values for $C_{10}H_{18}N_2O_3$ (M=214.2): C 56.05; H 8.46; N 13.07; Exp.: C 55.79; H 8.47; N 13.16.

### 1.3.2 Synthesis of Taxogen B (N-{2-{dimethylaminoethyl}}acrylamide hydrochloride)

A solution of 2 ml (18.3 mmoles) anhydrous 2-dimethylamino-ethylamin (Fluka) in 25 ml anhydrous $CH_2Cl_2$, is added dropwise, under a nitrogen atmosphere, at 0°C and during 2.5 h, to 2.2 ml (27.4 mmoles) acryloyl chloride in 18 ml anhydrous $CH_2Cl_2$. The reaction mixture is left, under stirring at 0°C during 2 h and at room temperature for 2 h, then evaporated to dryness yielding a white solid consisting into the taxogen B which was used without further purification for the following telomerization reactions. According to $^1$H NMR, its purity is of 90 %.
TLC ($CHCl_3/CH_3OH$, 6/4, UV, $KMnO_4$) Rf = 0.4. $^1$H NMR ($CD_3OD$): δ 3.00 (s, 6H, NMe) ;3.40 and 3.72 (t, t, J = 6.0 Hz, $CH_2N$); 5.77 (dd, 1H, J = 3.8 and 8.1 Hz, *H*CH=); 6.28 (dd, 1H, J = 3.8 and 17.0 Hz, HC*H*=); 6.38 (dd, 1H, J = 8.1 and 17.0 Hz, $CH_2$=C*H*). $^{13}$C NMR ($CD_3OD$): δ 34.2 ($CH_2NHCO$); 42.3 (NMe); 56.7 ($CH_2N$); 126.0 ($CH_2$=) ;130.1 (CH=); 167.5 (CO).

### 1.4 Synthesis of Telomers (Reaction scheme 4)

### 1.4.1 Telomers III-L1a(BOC) to III-L1m(BOC) from Telogen L1-SH (compound 3a) and Taxogen A

A solution of 500 mg (0.82 mmoles) telogen 3a, 1.68 g (7.85 mmoles) taxogen A (i.e. a molar ration taxogen/telogen of 9.5) and 40 mg (0.24 mmoles) AIBN in 15 ml anhydrous acetonitrile is stirred under reflux during 24h until the telogen 3a has disappeared (followed by TLC/DTNB). The solution is concentrated and chromatographed on a silica column (eluent: $CHCl_3$ to $CHCl_3$/ MeOH 98/2) and fractionated on a Sephadex LH 20 column (Sigma) with $CHCl_3$ as eluent. In this way one can isolate, as white solids, 83 mg telomer III-L1a(BOC) with n = 1, 85 mg telomer III-L1b(BOC) with n = 2, 93 mg telomer III-L1c(BOC) of an average degree of polymerization (aDP) n = 3 and 1.64 g telomer III-L1f(BOC) of an aDP n =10. The aDP is determined by $^1$H NMR. The aDP is equal to the integration ratio of the signal corresponding to the taxogen ethyldiamido methylenes relatively to the signal corresponding to the telogen methyl groups.

This protocol applied to telogen 3a and taxogen A at a taxogen/telogen molar ratio of 40 leads, under the same conditions of concentration and temperature, after chromatography and fractionation to telomers of aDP n = 4 (telomer III-L1d(BOC)), aDP n = 5 (telomer III-L1e(BOC)), aDP n = 20 (telomer III-L1h(BOC)), aDP n = 40 (telomer III-L1i(BOC)), aDP n = 50 (telomer III-L1i(BOC)) and aDP n = 60 (III-L1j(BOC)). aDP is determined by $^1$H NMR, as described above.

Telomer III-L1a(BOC) (aDP n = 1). $^1$H NMR ($CDCl_3$): δ 0.80 (t, 6H, $^3$J = 6.0 Hz, $CH_3$); 1.00-1.30 (m, 60H, $(CH_2)_{15}$); 1.25-1.75 (m, 13H, C*H*$_2$$CH_2NCO$, and $CH_3$ (BOC)); 2.43 (t, 2H, $^3$J = 7.0 Hz, $CH_2CO$); 2.51 (t, 2H, $^3$J = 7.0 Hz, $CH_2CO$); 2.65-2.85 (m, 4H, $CH_2S$); 3.05-3.35 (m, 8H, $CH_2NCO$ and C*H*$_2$NHCO); 5.20-5.30 (m, 1H, NH (BOC)); 6.55-6.75 (m, 1H, NH). $^{13}$C NMR ($CDCl_3$): δ 14.1 ($CH_3$); 22.7 (*C*H$_2$$CH_3$) ); 28.5 ($CH_3$ (BOC)); 27.0, 27.2, 27.3, 27.9, 28.3, 29.2, 29.4, 29.5 and 29.8 ($(CH_2)_{14}$); 32.0 (*C*H$_2$$CH_2CH_3$); 33.4 ($CH_2S$); 36.7 (*C*H$_2$CO); 40.5 (*C*H$_2$NHCO); 46.3 and 48.1 (*C*H$_2$NCO); 79.5 (C(BOC)); 156.8 (NCO (BOC)); 170.7 and 172.0 (CON).

Telomer III-L1b(BOC) (aDP n = 2): $^1$H NMR ($CDCl_3$): δ 0.80 (t, 6H, $^3$J = 6.0 Hz, $CH_3$); 1.00-1.30 (m, 60H, $(CH_2)_{15}$); 1.30-1.75 (m, 22H, C*H*$_2$$CH_2NCO$, $CH_3$ (BOC)); 1.75-1.95 (m, 2H, CHC*H*$_2$$CH_2CONH$); 1.95-2.30 (m, 2H, $CHCH_2$C*H*$_2$CONH); 2.30-2.85 (m, 7H, $CH_2CON$, C*H*CONH, $CH_2S$); 3.00-3.60 (m, 12H, $CH_2NCO$ and C*H*$_2$NHCO); 5.35-5.60 (m, 2H, NH (BOC)); 6.75-6.95 (m, 1H, NH); 6.95-7.10 (m, 1H, NH). MS-ESI: m/z = 1061 (calculated for $C_{59}H_{115}N_5O_7S + Na^+$ = 1061).

Telomer III-L1c(BOC) (aDP n = 3). $^1$H NMR ($CDCl_3$): δ 0.80 (t, 6H, $^3$J = 6.0 Hz, $CH_3$); 1.00-1.30 (m, 60H, $(CH_2)_{15}$); 1.30-1.75 (m, (4+9n)H, C*H*$_2$$CH_2NCO$, $CH_3$ (BOC)); 1.75-2.90 (m, (3n+5)H, CHCO, $CH_2CON$, $CH_2S$, C*H*$_2$$CH_2CO$ and C*H*$_2$CHCO); 3.00-3.60 (m, (4+4n)H, $CH_2NCO$ and C*H*$_2$NHCO); 5.15-5.65 (m, nH, NH (BOC)); 6.45-7.20 (m, nH, NH). $^{13}$C NMR ($CDCl_3$): δ 14.1 ($CH_3$); 22.7 (*C*H$_2$$CH_3$); 28.5 ($CH_3$ (BOC)); 27.0, 27.2, 27.4, 27.9, 28.1, 28.4, 29.2, 29.4 and 29.8 ($(CH_2)_{14}$ and CH*C*H$_2$CH); 32.0 (*C*H$_2$$CH_2CH_3$); 33.4 and 34.2 ($CH_2S$); 35.0 (*C*H$_2$CO); 39.9, 40.2, 40.5 and 40.9 (*C*H$_2$NHCO); 46.0 (*C*HCONH); 46.3 and 48.1 (*C*H$_2$NCO); 79.5 (C(BOC)); 156.8 (CO (BOC)); 171.0, 173.3 and 174.6 (CO).

Telomer III-L1d(BOC) (aDP n = 4). The $^1$H NMR spectrum is identical to that of telomer III-L1c(BOC) but with aDP n = 4. $^{13}$C NMR ($CDCl_3$): δ 14.1 ($CH_3$); 22.7 (*C*H$_2$$CH_3$) ); 28.6 ($CH_3$ (BOC)); 27.0, 27.2, 27.9, 29.2, 29.4 and 29.8 ($(CH_2)_{14}$ and CH*C*H$_2$CH); 32.0 (*C*H$_2$$CH_2CH_3$); 46.3 and 48.1 (*C*H$_2$NCO); 79.6 (C (BOC)); 156.8 (CO (BOC)). The resonances corresponding to the carbons *C*H$_2$NHCO, *C*H$_2$CO, *C*HCO and to the carbons the NCO appear, respectively, as large signals between 38 and 43 ppm and between 170 and 177 ppm. The *C*H$_2$S carbons are not detectable.

Telomer III-L1e(BOC) (aDP n = 5). The $^1$H NMR spectrum is identical to that of telomer III-L1c(BOC) but with aDP n = 5. The $^{13}$C NMR spectrum is identical to that of telomer III-L1d(BOC).

Telomer III-L1f(BOC) (aDP n = 10). The $^1$H NMR spectrum is identical to that of telomer III-L1c(BOC) but with

aDP n = 10. The $^{13}C$ NMR spectrum is identical to that of telomer III-L1d(BOC). MS-ESI shows the presence of compounds with n = 3 to 15: m/z = (610 + 214.2n + 23) with n = 3 to 15 in agreement with the masses calculated for $[C_{39}H_{79}NOS + n(C_{10}H_{18}N_2O_3) + Na^+]$ with n = 3 to 15..

Telomer III-L1g(BOC) (aDP n = 20). The $^1H$ NMR spectrum is identical to that of telomer III-L1c(BOC) but with aDP n = 20. The $^{13}C$ NMR spectrum is identical to that of telomer III-L1d(BOC). MS-ESI shows the presence of compounds with n = 4 to 33: m/z = (610 + 214.2n + 23) with n = 4 to 15, in agreement with the masses calculated for $[C_{39}H_{79}NOS + n(C_{10}H_{18}N_2O_3)+Na^+]$ with n = 4 to 15; m/2z = (305 + 107.1n + 23) with n = 9 to 33 in agreement with the masses calculated for $[C_{39}H_{79}NOS + n(C_{10}H_{18}N_2O_3) + 2Na^+]/2$ with n = 9 to 33.

Telomer III-L1h(BOC) (aDP n = 40). The $^1H$ NMR spectrum is identical to that of telomer III-L1c(BOC) but with aDP n = 40. The $^{13}C$ NMR spectrum is identical to that of telomer III-L1d(BOC).

Telomer III-L1i(BOC) (aDP n = 50). The $^1H$ NMR spectrum is identical to that of telomer III-L1c(BOC) but with aDP n = 50. The $^{13}C$ NMR spectrum is identical to that of telomer III-L1d(BOC).

Telomer III-L1j(BOC) (aDP n = 60). The $^1H$ NMR spectrum is identical to that of telomer III-L1c(BOC) but with aDP n = 60. The $^{13}C$ NMR spectrum is identical to that of telomer III-L1d(BOC).

### 1.4.2 Telomers III-L1k(BOC) to III-L1m(BOC) from Telogen L1-SH (compound 3b) and Taxogen A

This protocol applied to telogen 3b and taxogen A at a taxogen/telogen molar ratio of 40 leads, under the same conditions of concentration and temperature, after chromatography and fractionation to telomers III-L1k(BOC) of aDP n = 10, III-L1l(BOC) of aDP n = 20, and III-L1m(BOC) of aDP n = 70. aDP is determined by $^1H$ NMR, as described above.

Telomer III-L1k(BOC) (aDP n = 10). $^1H$ NMR ($CDCl_3$): δ 0.80 (t, 6H, $^3J$ = 6.0 Hz, $CH_3$); 1.00-1.30 (m, 44H, $(CH_2)_{11}$); 1.30-1.75 (m, (9n+4)H, $CH_2CH_2NCO$, and $CH_3$ (BOC)); 1.75-3.00 (m, (3n+5)H, CHCO, $CH_2CON$, $CH_2S$, $CH_2CH_2CO$ and $CH_2CHCO$); 3.00-3.60 (m, (4+4n)H, $CH_2NCO$ and $CH_2NHCO$); 5.15-5.65 (m, nH, NH (BOC)). MS-ESI shows The presence of components of n = 2 to 10: m/z = (498 + 214.2n + 23) with n = 2 to 10, in agreement with the masses calculated for $[C_{31}H_{63}NOS + n(C_{10}H_{18}N_2O_3) + Na^+]$ with n = 2 to 10.

Telomer III-L1l(BOC) (aDP n = 20). The $^1H$ NMR spectrum is identical to that of telomer III-L1k(BOC) but with aDP n = 20. MS-ESI shows the presence of components of n = 8 to 20: m/2z = (249 + 107.1n + 23) in agreement with the masses calculated for $[C_{31}H_{63}NOS + n(C_{10}H_{18}N_2O_3) + 2Na^+]/2$ with n = 8 to 20.

Telomer III-L1m(BOC) (aDP n = 70). The $^1H$ NMR spectrum is identical to that of telomer III-L1k(BOC) but wit aDP n = 70.

### 1.4.3 Telomers III-L1a to III-L1m: Deprotection of Telomers

The deprotection of telomers III-L1a(BOC) to III-L1m(BOC) is quantitatively achieved by dissolving and keeping these telomers in a large excess of trifluoroacetic acid (TFA; Aldrich) at room temperature for 1 h. The excess of TFA is removed by evaporation in the presence of cyclohexane. The telomers III-L1a to III-L1m are obtained in the form of white solids.

Telomer III-L1a (n = 1): $^1H$ NMR ($CDCl_3/CD_3OD$): δ 0.85 (t, 6H, $^3J$ = 6.0 Hz, $CH_3$); 1.15-1.45 (m, 60H, $(CH_2)_{15}$); 1.45-1.60 (m, 4H, $CH_2CH_2N$); 2.51 and 2.57(t, t, 2H, 2H, $^3J$ = 7.0 Hz, $CH_2CO$); 2.70-2.90 (m, 4H, $CH_2S$); 3.04 (t, 2H, $^3J$ = 5.5 Hz, $CH_2NH_3$); 3.15-3.40 (m, 4H, $CH_2N$); 3.45 (t, 2H, $^3J$ = 5.5 Hz, $CH_2NH$). $^{13}C$ NMR ($CDCl_3/CD_3OD$): δ 14.2 ($CH_3$); 22.8 ($CH_2CH_3$); 27.1, 27.2, 27.8, 29.5, 29.6, 29.8 ($(CH_2)_{14}$); 32.0 ($CH_2CH_2CH_3$); 33.4 ($CH_2S$); 34.8 ($CH_2CO$); 37.5 ($CH_2NH_3$); 39.9 ($CH_2NH$); 46.3 and 48.1 ($CH_2N$); 117.0 (q, $^1J$ = 300 Hz, $CF_3CO_2^-$); 162.8 (q, $^2J$ = 46 Hz, $CF_3CO_2^-$); 171.0 and 175.2 (CO).

Telomer III-L1b (n = 2): $^1H$ NMR ($CDCl_3$): δ 0.80 (t, 6H, $^3J$ = 6.0 Hz, $CH_3$); 1.00-1.30 (m, 60H, $(CH_2)_{15}$); 1.30-1.65 (m, 4H, $CH_2CH_2NCO$); 1.65-1.95 (m, 2H, $CHCH_2CH_2CONH$); 1.95-2.30 (m, 2H, $CHCH_2CH_2CONH$); 2.30-2.85 (m, 7H, $CH_2CON$, CHCON, $CH_2S$); 3.00-3.60 (m, 12H, $CH_2NH_3$, $CH_2NCO$ and $CH_2NHCO$). $^{13}C$ NMR($CDCl_3$): δ 14.2 ($CH_3$); 22.8 ($CH_2CH_3$) ); 27.1, 27.2, 27.8, 29.5, 29.6, 29.8 ($(CH_2)_{14}$); 32.0 ($CH_2CH_2CH_3$); 33.4 ($CH_2S$); 34.8 ($CH_2CO$); 37.5 ($CH_2NH_3^+$); 39.9 ($CH_2NH$); 46.0 (CHCO); 46.3 and 48.1 ($CH_2NCO$); 117.0 (q, $^1J_{CF}$ = 300 Hz, $CF_3CO_2^-$); 162.8 (q, $^2J_{CF}$ = 46 Hz, $CF_3CO_2^-$); 171.0, 175.2 and 177.0 (CON). MS-ESI: m/z = 839, in agreement with the calculated mass for $M[=C_{39}H_{79}NOS + 2(C_5H_{10}N_2O)] + H^+$.

Telomers III-L1c to III-L1j: $^1H$ NMR($CD_3OD$): δ 0.80 (t, 6H, $^3J$ = 6.0 Hz, $CH_3$); 1.00-1.35 (m, 60H, $(CH_2)_{15}$); 1.35-2.60 (m, (9+3n)H, $CH_2CH_2N$, $CH_2CON$, CHCO, $CH_2S$, $CH_2CH_2CO$ and $CH_2CHCO$); 2.70-3.60 (m, (4n+4)H, $CH_2NH_3^+$, $CH_2NCO$ and $CH_2NHCO$) with, respectively, an aDP n = 3 (telomer III-L1e), 4 (telomer III-L1d), 5 (telomer III-L1e), 10 (telomer III-L1f), 20 (telomer III-L1h), 40 (telomer III-L1i), 50 (telomer III-L1i), and 60 (telomer III-L1j). $^{13}C$ NMR ($CD_3OD$): δ 14.3 ($CH_3$); 23.3 ($CH_2CH_3$); 29.0, 29.6, 29.9 and 30.3 ($CHCH_2CH$ and $(CH_2)_{14}$); 32.5 ($CH_2CH_2CH_3$); 37.7 ($CH_2NCO$); 40.0 ($CH_2NH_3^+$); 117.0 (q, $^1J$ = 300 Hz, $CF_3CO_2^-$); 162.8 (q, $^2J_{CF}$ = 46 Hz, $CF_3CO_2^-$); 176.2 (NCO). The resonances corresponding to carbons *C*HCO and NH*C*O appear as broad signals between 41 and 45 ppm and

between 176.5 and 179 ppm, respectively. The resonances corresponding to carbons $CH_2S$ and $CH_2CO$ are not detectable.

MS-ESI of telomer III-L1f shows the presence of compounds with n = 3 to 15: m/z = (610 + 114.1n + 1) with n = 3 to 10, in agreement with the calculated masses for [$C_{39}H_{79}NOS$ + n($C_5H_{10}N_2O$) + $H^+$] with n = 3 to 10; m/2z = (305 + 57n + 1) with n = 3 to 15, in agreement with the calculated masses for [$C_{39}H_{79}NOS$ + n($C_5H_{10}N_2O$) + $2H^+$]/2 with n = 3 to 15; m/3z = (203 + 38n + 1) with n = 5 to 13 in agreement with the calculated masses for [$C_{39}H_{79}NOS$ + n($C_5H_{10}N_2O$)+ $3H^+$]/3, with n = 3 to 13.

Telomers III-L1k to III-L1m:$^1H$ NMR($CD_3OD$): δ 0.80 (t, 6H, $^3J$ = 6.0 Hz, $CH_3$); 1.00-1.35 (m, 44H, $(CH_2)_{11}$); 1.35-2.60 (m, (9+3n)H, $CH_2CH_2N$, $CH_2CON$, CHCO, $CH_2S$, $CH_2CH_2CO$ and $CH_2CHCO$); 2.70-3.60 (m, (4n+4)H, $CH_2NH_3^+$, $CH_2NCO$ and $CH_2NHCO$) with respectively an aDP of 10 (telomer III-L1k), 20 (telomer III-L1l) and 70 (telomer III-L1m). The $^{13}C$ NMR spectra are identical to those of telomers III-L1c to III-L1j.

### 1.4.4 Telomer III-L2a (Y=Trt)(BOC) from Telogen L2-SH (compound 6a) and Taxogen A

The above described procedure for the synthesis of telomers III-L1a(BOC) to III-L1m(BOC) (cf. 1.4.1) was applied to 846 mg (0.85 mmoles) telogen 6a (Y=Trt), 0.77 g (3.6 mmoles) taxogen A (taxogen/telogen ratio of 4.5) and 0.029 g (0.17 mmoles) AIBN in 20 ml anhydrous acetonitrile and gave, after purification and fractionation, 345 mg (0.24 mmoles) telomer III-L2a (Y=Trt)(BOC) (aDP n = 2) in the form of a white solid.

Telomer III-L2a (Y=Trt)(BOC) (n = 2): $^1H$ NMR ($CDCl_3$): δ 0.87 (t, 6H, $^3J$ = 6.0 Hz, $CH_3$); 1.10-1.40 (m, 60H, $(CH_2)_{15}$); 1.40-2.00 (m, 22H, $CH_2CH_2N$ and $CH_3$ (BOC)); 2.00-3.50 (m, 25H,$CH_2CO$, $CH_2S$, $CH_2$-im, $CH_2N$ and $CH_2CHCH_2CH_2CO$); 4.90-5.20 (m, 1H, CHN); 6.5-7.6 (m, 17H, Trt and im). $^{13}C$ NMR ($CDCl_3$): δ 14.1 ($CH_3$); 22.7 ($CH_2CH_3$); 26.9, 27.1, 27.7, 29.4 and 29.7 ($(CH_2)_{14}$); 28.5 ($CH_3$ (BOC)); 31.9 ($CH_2CH_2CH_3$); 46.6 and 48.1 ($CH_2N$); 49.5 (CHN); 75.2 (N-C (Trt)); 79.1 (OC (BOC)); 119.4 (CH=C); 136.3 (CH=C); 138.4 (CH=N); 128.0 and 129.7 (CH (Ph)); 142.3 (C (Ph)); 156.6 (CO (BOC)); 170.7, 173.4 and 175.9 (NCO). $CH_2$-im and $(CH_2CH)_nCH_2CH_2CO$ appear as a broad signal between 33 and 44 ppm.

### 1.4.5 Telomer III-L2b-d(Y=BOC)(BOC) from Telogen L2-SH (compound 6h) and Taxogen A

The protocol described above for the synthesis of telomers III-L1a(BOC) to III-L1m(BOC), was applied to 20 mg (0.02 mmoles) telogen 6b(Y=BOC), 202 mg (0.9 mmoles) taxogen A (taxogen/telogen ratio 45) and 2 mg (0.01 mmoles) AIBN in 2 ml anhydrous acetonitrile. It yielded, after purification and fractionation, 26 mg of telomer III-L2b(Y=BOC)(BOC) of aDP n = 20, 40 mg of telomer III-L2c(Y=BOC)(BOC) of aDP n = 30, and 156 mg of telomer III-L2d(Y=BOC)(BOC) of aDP n = 90, in the form of white solids.

Telomers III-L2b(Y=BOC)(BOC) to III-L2d(Y=BOC)(BOC): The $^1H$ NMR ($CDCl_3$) spectra of III-L2b(Y=BOC)(BOC) to III-L2d(Y=BOC)(BOC) are identical: δ 0.81 (t, 6H, $^3J$ = 6.0 Hz, $CH_3$); 1.10-1.75 (m, 60H, $(CH_2)_{15}$); 1.40-1.80 (m, (13+9n)H, $CH_2CH_2N$ and $CH_3$ (BOC)); 1.80-3.50 (m, (11+7n)H, $CH_2CO$, $CH_2S$, $CH_2$-im, $CH_2N$ and $CH_2CHCH_2CH_2CO$); 4.90-6.50 (m, (n+1)H, CHN, NH(BOC)); 6.5-8.0 (m, NH, im), with aDP n = 20, 30, and 90, respectively.

### 1.4.6 Telomers III-L2a (Y=Trt)(BOC) and III-L2b (Y=BOC)(BOC) to III-L2d(Y=BOC)(BOC): Deprotection of Telomers

The deprotection of telomer III-L2a(Y=Tr)(BOC) is achieved by dissolving and keeping the compound in a large excess of TFA at room temperature for 24 h. The reaction mixture is evaporated, the dry residue is taken up in 3 ml THF, 5 ml 6N HCl (30 mmoles) are added, and the reaction mixture is kept, under stirring, at room temperature for 3 h. After evaporation of the solvents and washing of the residue with $CH_3CN$, telomer III-L2a is obtained.

The deprotection of telomers III-L2b(Y=BOC)(BOC) to III-L2d(Y=BOC)(BOC) is quantitatively achieved by dissolving and keeping the compound in a large excess of TFA at room temperature for 1 h. Excess TFA is removed by evaporation in the presence of cyclohexane. Telomers III-L2b to III-L2d are obtained in the form of white solids.

Telomer III-L2a (aDP n = 2): $^1H$ NMR($CDCl_3$): δ 0.87 (t, 6H, $^3J$ = 6.0 Hz, $CH_3$); 1.10-1.40 (m, 60H, $(CH_2)_{15}$); 1.40-1.80 (m, 4H, $CH_2CH_2N$); 1.80-3.50 (m, (11+7n)H, $CH_2CO$, $CH_2S$, $CH_2$-im, $CH_2NCO$, $CH_2NH_3$ and $CH_2CHCH_2CH_2CO$); 4.90-5.20 (m, 1H, CHN). $^{13}C$ NMR ($CDCl_3$): δ 14.1 ($CH_3$); 22.7 ($CH_2CH_3$); 26.9, 27.1, 27.7, 29.4 and 29.7 ($(CH_2)_{14}$ and $CH_2CHCH_2$); 32.0 ($CH_2CH_2CH_3$); 47.0 and 48.5 ($CH_2N$ and CHN); 118.0 (CH=C); 134.0 (CH=C and CH=N); 170.3 and 171.1 (NCO). The resonances corresponding to carbons $CH_2NHCO$, $CH_2NH_3^+$, $CH_2CO$, $CH_2S$, CHCO, and $CH_2$-im appear as broad signals at 34.6 and 35.9 ppm.

Telomer III-L2b (aDP n = 20): $^1H$ NMR ($CD_3OD$): δ 0.94 (t, 6H, $^3J$ = 6.0 Hz, $CH_3$); 1.20-1.50 (m, 60H, $(CH_2)_{15}$); 1.50-2.10 (m, (2+2n)H, $CH_2CH_2NCO$ and $CHCH_2CH$); 2.10-3.00 (m, (9+n)H, $CH_2CO$, $CH_2S$, $CH_2$-im and CHCO); 3.00-3.80 (m, (4+4n)H, $CH_2NCO$, $CH_2NHCO$ and $CH_2NH_3$); 7.38 (s, 1H, CH=C); 8.86 (s, 1H, CH=N). The signal corresponding to CHN is masked by that of $H_2O$ at 5 ppm $^{13}C$ NMR ($CD_3OD$): δ 29.7 ($CH_2CHCH_2$ and $(CH_2)_{14}$); 37.7 ($CH_2NCO$); 40.0 ($CH_2NH_3^+$)); 117.0 (q, $^1J$ = 300 Hz, $CF_3CO_2^-$); 162.8 (q, $^2J$ = 46 Hz, $CF_3CO_2^-$); 177.5 (NHCO). The resonances corresponding to carbons $CH_3$, $CH_2CH_3$, $CH_2CH_2CH_3$, $CH_2N$, CHN, $CH_2CO$, $CH_2S$, $CH_2$-im, $(CH_2CH)_nCH_2CH_2CO$, NCO, CH=C and CH=N could not be localised.

Telomers III-L2c (aDP n = 30) and III-L2d (aDP n = 90): their $^1H$ NMR($CD_3OD$) spectra are identical to that of telomer III-L2b but with aDP n = 30 and 90, respectively. Their $^{13}C$ NMR ($CD_3OD$) spectra are identical to that of telomer III-L2b.

### 1.4.7 Telomers III-L2e(Bzl)(BOC) and III-L2f(Bzl)(BOC) from Telogen L2-SH (compound 9) and Taxogen A

The above described procedure for the synthesis of III-L1a(BOC) to III-L1m(BOC) was applied to 32 mg (0.045 mmoles) telogen 9, 195 mg (0.91 mmoles) taxogen A (i.e. a taxogen /telogen molar ratio of 20) and 3 mg (0.018 mmoles) AIBN in 1.8 ml anhydrous acetonitrile it yielded after purification and fractionation 64 mg telomer III-L2a(Bzl)(Boc) of aDP n = 20 and 138 mg III-L2b(Bzl)(Boc) of aDP n = 70, in the form of white solids.

Telomer III-L2e(Bzl)(BOC) (aDP n = 20). $^1H$ NMR ($CDCl_3$) : δ 0.80 (t, 6H, $^3J$ = 6.0 Hz, $CH_3$); 1.05-1.20 (m, 44H, $(CH_2)_{11}$); 1.20-1.60 (m, (4+9n)H, $CH_2CH_2NCO$ and $CH_3$(Boc)); 1.60-2.90 (m, (3n+7)H, CHCO, $CH_2CO$, $CH_2S$, $CH_2CH_2CO$ and $CH_2CHCO$); 2.90-4.00 (m, (4n+4)H, $CH_2NCO$ and $CH_2NHCO$); 5.05 (s, 2H, $CH_2Ph$); 5.10-5.40 (m, 1H, CH); 5.40-6.50 (m, nH, NH); 7.30 (s, 5H, Ph). $^{13}C$ NMR ($CDCl_3$): δ 14.2 ($CH_3$); 22.7 ($CH_2CH_3$); 28.5 ($CH_3$(BOC)); 26.9, 27.0, 27.5, 29.3 and 29.6 ($(CH_2)_{11}$); 31.9 ($CH_2CH_2CH_3$); 46.1, 46.7 and 48.1 ($CH_2N$ and $CH_2Ph$); 66.7 (CH); 128.3 and 128.5 (CH=CH); 135.6 (C=CH); 156.6 (CO(BOC)); 170.2 (CO). The resonances corresponding to the carbons $CHCH_2CH$, CH2NHCO, CHCO and NCO appear as large signals between 32 and 38 ppm, 38 and 45 ppm, and between 175 and 178 ppm, respectively. The $CH_2S$, $CH_2CO$ and $CH_2CO_2$ carbons are not detectable.

Telomer III-L2f(Bzl)(BOC) (aDP n = 70). $^1H$ NMR ($CDCl_3$) : identical to that of Telomer III-L2e(Bzl)(BOC) but with n = 70.

### 1.4.8 Telomers III-L2e and III-L2f:Deprotection of Telomers III-L2e(Bzl)(BOC) and III-L2f(Bzl)(BOC)

The deprotection of telomers III-L2e(Bzl)(BOC) and III-L2f(Bzl)(BOC) is achieved by dissolving and keeping these telomers in a large excess of 30% HBr/AcOH (Aldrich) at 0¡C for 2 h. After evaporation, the residue is dissolved in MeOH and aceton is added until a precipate appears. Filtration, and washing with $CHCl_3$ and cyclohexane, yielded telomers III-L2e and III-L2f (as their $Br^-$ salts) in the form of white solids.

Telomer III-L2e (n = 20): $^1H$ NMR ($CD_3OD$): 0.90 (t, 6H, J = 6.0 Hz), $CH_3$) ; 1.27 (bs, 44H, $(CH_2)_{11}$) ; 1.40-2.15 (m, (2n+2)H, $CHCH_2CH$, $CH_2CH_2N$); 2.15-3.00 (m, (n+9)H, $CH_2S$, $CH_2CO$ and CHCO); 3.00-3.40 (m, (2n+4)H, $CH_2NHCO$, $CH_2N$) ; 3.40-3.90 (m, 2n, $CH_2NH_3^+$) ; 5.15 (m, 1H, COCHN). $^{13}C$ NMR ($CD_3OD$): 14.4 (Me) ; 23.6 ($CH_2Me$) ;30.4 and 30.7 (($CH_2$)11) ; 33.0 ($CH_2CH_2Me$) ; 38.4 (bs, $CH_2NHCO$) ;40.8 (bs, $CH_2NH_3$) ;178.3 (bs, CO). The resonances corresponding to the carbons $CHCH_2CH$, and $CHCH_2CH$ appear as large signals between 32 and 38 ppm, 42 and 48 ppm, respectively. The $COCH_2CH_2SCH_2$ and $COCHCH_2CO$ carbons are not detectable.

Telomer III-L2f (aDP n = 70): $^1H$ NMR ($D_2O$) : identical to that of telomer III-L2e but with an aDP n = 70. $^{13}C$ NMR ($D_2O$) : δ 37.0 (bs, $CH_2NHCO$) ; 38.9 (bs, $CH_2NH_3$) ; 42.62 (CHCO); 177.2 (bs, CO). The resonances corresponding to the carbons $CHCH_2CH$ appear as a large signal between 32 and 40 ppm. The $COCH_2CH_2SCH2$, $CH_3(CH_2)_{13}N$ and $COCHCH_2CO$ carbons are not detectable.

### 1.4.9 Telomers III-L3a(BOC) to III-L3e(BOC) from Telogen L3-SH, 9, and Taxogen A (Reaction scheme 3)

The protocol described above for the synthesis of telomers III-L1a(BOC) to III-L1m(BOC) was applied to telogen L3-SH 9 and to taxogen A, at a molar ratio of taxogen to telogen of 3 and 5. It leads to two series of telomers in the form of white solids after purification by chromatography on a silica column (eluent: $CHCl_3$) and with a yield of 60 %. The fractionation on Sephadex LH-20 of the first series leads to telomers III-L3a(BOC) (n = 1), III-L3b(BOC) (aDP n = 3) and III-L3d(BOC) (aDP n = 11); the fractionation of the second series allows to isolate, in addition to the first three telomers, telomers III-L3c(BOC) (aDP n = 6) and III-L3e(BOC) (aDP n = 16). The aDPs are determined by $^1H$ NMR, as detailed above (see 1.41).

Telomer III-L3a(BOC) (n = 1): $^1H$ NMR ($CDCl_3$): δ 0.80 (t, 6H, $^3J$ = 6.0 Hz, $CH_3$); 1.02-1.32 (m, 60H, $(CH_2)_{15}$); 1.37 (s, 9H, $CH_3$ (BOC)); 1.44-1.55 (m, 4H, $CH_2CH_2O$); 2.39 (t, 2H, $^3J$ = 6.9 Hz, $CH_2CO$); 2.64 (d, 2H, $^3J$ = 4.8 Hz, $CH_2S$); 2.78 (t, 2H, $^3J$ = 6.2 Hz, $SCH_2CH_2CO$); 3.12-3.60 (m, 11H, $CH_2O$, CHO and $CH_2N$); 5.16 and 6.60 (m 2H, NH). $^{13}C$ NMR ($CDCl_3$): δ 14.0 ($CH_3$); 22.7 ($CH_2CH_3$); 26.1 ($CH_2$ γ O); 28.4 ($CH_3$ (BOC)); 29.3, 29.5, 29.6, 29.7 and 30.0 ($(CH_2)_{13}$); 31.9 ($CH_2CH_2CH_3$); 33.9 ($CH_2S$); 36.7 ($CH_2CO$); 40.3 and 40.5 ($CH_2N$); 70.5, 71.5 and 71.7 ($CH_2O$); 78.5 (CHO); 79.5 (O-C (BOC)); 156.7 (C=O (BOC)); 171.9 (C=O).

Telomer III-L3b(BOC) (aDP n = 3):$^1H$ NMR ($CDCl_3$): δ 0.80 (t, 6H, $^3J$ = 6.0 Hz, $CH_3$); 0.98-1.30 (m, 60H,

$(CH_2)_{15}$); 1.30-1.60 (m, (9n+4)H, $CH_3$ (BOC) and $CH_2CH_2O$); 1.60-3.00 (m, (3n+3)H, $CH_2CO$, CHCO, $CH_2CHCO$, $CH_2CH_2CO$ and $CH_2S$); 3.05-3.55 (m, (4n+7)H, $CH_2O$, CHO and $CH_2N$). $^{13}C$ NMR ($CDCl_3$): δ 14.1 ($CH_3$); 22.6 ($CH_2CH_3$); 26.1 ($CH_2$ γ O); 28.4 ($CH_3$ (BOC)); 29.3, 29.5, 29.6, 29.7 and 30.1 ($(CH_2)_{13}$ and $CH_2CHCO$); 31.9 ($CH_2CH_2CH_3$); 33.8 and 34.5 ($CH_2S$); 35.6 ($CH_2CO$); 35.8 and 36.2 (CHCO); 70.4, 71.6 and 71.7 ($CH_2O$); 78.5 (CHO); 79.6 (O-C (BOC)); 156.7 (C=O (BOC)); 172.0, 173.1 and 174.4 (CO). The $CH_2N$ carbons appear as a broad resonance between 39 and 41 ppm.

Telomer III-L3c(BOC) (aDP n = 6): the $^1H$ NMR ($CDCl_3$) spectrum is identical to that of III-L3b(BOC) but with an aDP n = 6. $^{13}C$ NMR ($CDCl_3$): δ 14.0 ($CH_3$); 22.6 ($CH_2CH_3$); 26.0 ($CH_2$ γ O); 28.4 ($CH_3$ (BOC)); 29.3, 29.4, 29.6 and 29.7 ($(CH_2)_{13}$ and $CH_2CHCO$); 31.8 ($CH_2CH_2CH_3$); 33.6 and 34.4 ($CH_2S$); 70.4, 71.7 and 71.9 ($CH_2O$); 78.5 (CHO); 79.6 (O-C (BOC)); 156.6 (C=O (BOC)); the $CH_2$(C=O), CHC=O, $CH_2N$, and NHC=O carbons appear as very broad resonance signals between 38 and 44 ppm and between 173 and 176 ppm.

Telomers III-L3d(BOC) (aDP n = 11) and III-L3e(BOC) (aDP n = 16): their $^1H$ and $^{13}C$ NMR spectra are identical to those of telomer III-L3c(BOC) but with an aDP of 11 and 16, respectively.

### 1.4.10 Telomers III-L3a(BOC) to III-L3e(BOC): Deprotection of Telomers

The removal of the BOC protection groups from telomers III-L3a(BOC) to III-L3e(BOC) is quantitatively achieved by dissolving and keeping these telomers in a large excess of TFA at room temperature for 1h. The excess TFA is removed by coevaporation in the presence of cyclohexane. Telomers III-L3a to III-L3e are obtained in the form of white solids.

Telomer III-L3a (n = 1): IR (υ $cm^{-1}$, KBr): 1875 (C=O). $^1H$ NMR ($CDCl_3$): δ 0.80 (t, 6H, $^3J$ = 6.0 Hz, $CH_3$); 1.00-1.36 (m, 60H, $(CH_2)_{15}$); 1.37-1.70 (m, 4H, $CH_2CH_2O$); 2.43 (t, 2H, $^3J$ = 6.1 Hz, $CH_2CO$); 2.62 (d, 2H, $^3J$ = 5.0 Hz, $CH_2S$); 2.74 (t, 2H, $^3J$ = 6.2 Hz, $SCH_2CH_2CO$); 3.00-3.25 (m, 2H, $CH_2NH_3$); 3.30-3.65 (m, 9H, $CH_2O$, CHO and $CH_2N$). $^{13}C$ NMR ($CDCl_3$): δ 14.1 ($CH_3$); 22.7 ($CH_2CH_3$); 26.1 ($CH_2$ γ O); 29.3, 29.5, 29.7 and 30.0 ($(CH_2)_{13}$); 31.9 ($CH_2CH_2CH_3$); 33.8 ($CH_2S$); 36.1 ($CH_2CO$); 37.5 ($CH_2N$); 39.4 ($CH_2NH_3$); 70.6, 71.6 and 71.8 ($CH_2O$); 78.3 (CHO); 79.5 (O-C (BOC)); 171.9 (C=O). $^{19}F$ NMR ($CDCl_3$): δ -76.0 ($CF_3CO_2^-$).

Telomer III-L3b (aDP n = 3): $^1H$ NMR ($CDCl_3$, $CD_3OD$): δ 0.84 (t, 6H, $^3J_{HH}$ = 5.8 Hz, $CH_3$); 1.00-1.45 (m, 60H, $(CH_2)_{15}$); 1.45-1.75 (m, 4H, $CH_2CH_2O$); 1.75-3.25 (m, (5n+3)H, $CH_2CH_2CO$, $CH_2CHCO$, $CH_2CO$, CHCO, $CH_2NH_3$ and $CH_2S$); 3.25-3.70 (m, (2n+7)H, $CH_2O$, CHO and $CH_2N$). $^{13}C$ NMR ($CDCl_3$. $CD_3OD$): δ 15.0 ($CH_3$); 23.7 ($CH_2CH_3$); 27.2 ($CH_2$ γ O); 29.0, 29.1, 30.4, 30.6, 30.7, 30.8 and 30.9 ($(CH_2)_{13}$ and $CH_2CHCO$); 33.0 ($CH_2CH_2CH_3$); 33.7 ($CH_2S$); 38.1 ($CH_2CO$); 40.7 ($CH_2N$); 41.0 ($CH_2NH_3$); 43.4 (CHCO); 70.5, 70.6 and 71.8 ($CH_2O$); 78.3 (CHO); 163.4 (q, $^2J_{CF}$ = 34 Hz, $CF_3CO_2^-$); 174.8, 175.1 and 177.1 (NC=O). The CH-O carbon is not detectable. $^{19}F$ NMR ($CDCl_3$. $CD_3OD$): δ -76.6 ($CF_3CO_2^-$).

Telomer III-L3c (aDP n = 6): The $^1H$ NMR and $^{19}F$ NMR ($CDCl_3$, $CD_3OD$) spectra, respectively, are identical to those of telomer III-L3b, but with aDP n = 6. $^{13}C$ NMR ($CDCl_3$, $CD_3OD$): δ 14.8 ($CH_3$); 23.7 ($CH_2CH_3$); 27.1 ($CH_2$ γ O); 30.4, 30.5, 30.7 and 31.1 ($(CH_2)_{13}$ and $CH_2CHCO$); 33.0 ($CH_2CH_2CH_3$); 34.0 ($CH_2S$); 35.0 ($CH_2CO$); 38.1 ($CH_2N$); 40.7 ($CH_2NH_3$); 71.5, 72.8 and 72.9 ($CH_2O$); 79.6 (CHO); 163.0 (q, $^2J_{CF}$ = 36 Hz, $CF_3CO_2^-$); 176.2 and 177.9 (NC=O). The CH(C=O) carbons appear as broad resonance signals between 41 and 46 ppm. MS-FAB and MS-ESI show the presence of n = 4 to 9 oligomers: m/z = 1085.5; 1199.5; 1313.6; 1427.6; 1541.6; 1657.2 in agreement with M(= $C_{39+5n}H_{80+11n}N_{2n}O_{2+n}S$) + 16 + $H^+$] for n = 4 to 9, respectively. The mass M + 16 corresponds to the oxidation of the sulfur under the conditions of the analysis.

Telomer III-L3d (aDP n = 11): The $^1H$ and $^{19}F$ NMR spectra ($CDCl_3$, $CD_3OD$) are, respectively, identical to those of telomer III-L3b, but with aDP n = 11. $^{13}C$ NMR ($CDCl_3$, $CD_3OD$): δ 14.8 ($CH_3$); 23.7 ($CH_2CH_3$); 27.1 ($CH_2$ γ O); 30.3, 30.6 and 30.7 ($(CH_2)_{13}$ and $CH_2CHCO$); 32.3 ($CH_2CH_2CH_3$); 33.2 ($CH_2S$); 163.0 (q, $^2J_{CF}$ = 36 Hz, $CF_3CO_2^-$); 165.0 (NC=O). The resonances corresponding to carbons $CH_2N$, $CH_2CO$ and CHCO appear as broad signals between 38 and 43 ppm; the resonances corresponding to carbons $CH_2O$ and CHO are not detectable. MS-FAB and MS-ESI show the presence of oligomers of n = 4 to 15: m/z= 1085.9; 1199.9; 1314.0; 1428.1; 1542.1; 1656.2; 1770.3; 1884.4; 1998.4; 2113.5; 2226.5 in agreement with M(= $C_{39+5n}H_{80+11n}N_{2n}O_{2+n}S$) + 16 for n = 4 to 14, respectively. MS-ESI: m/2z = 771.8; 829.0; 886.0; 943.0; 1000.0; 1057.3; 1114; 1171 in agreement with [M(= $C_{39+n}H_{80+11n}N_{2n}O_{2+n}S$) + 16 + 2H+]/2, for n = 8 to 15, respectively. The mass M + 16 corresponds to the oxidation of the sulfur under the conditions of the analysis.

Telomer III-L3e (aDP n = 16). The $^1H$ and $^{19}F$ NMR ($CDCl_3$, $CD_3OD$) spectra are, respectively, identical to those of telomer III-L3b, but with aDP n = 16. $^{13}C$ NMR($CDCl_3$, $CD_3OD$): δ 14.8 (CH); 23.7 ($CH_2CH_3$); 27.1 $CH_2$ γ O); 30.3, 30.6 and 30.7 ($(CH_2)_{13}$ and $CH_2CHCO$); 32.3 ($CH_2CH_2CH_3$); 33.2 ($CH_2S$); 163.0 (q, $^2J_{CF}$ = 36 Hz, $CF_3CO_2^-$); the resonances corresponding to carbons $CH_2N$, $CH_2CO$, CHCO, and NCO, respectively, appear as broad signals between 38 and 43 ppm and between 176 and 179 ppm, respectively; the resonances corresponding to carbons $CH_2O$ and CHO are not detectable. MS-FAB and MS-ESI show the presence of oligomers of n = 6 to 17: m/z = 1314; 1428.1; 1542.2; 1656.3; 1771.3; 1885.4; 1999.5; 2113.5; 2227.6; 2341.4; 2455; 2569; 2683; 2798 in agreement with M

(=$C_{39+5n}H_{80+11n}N_{2n}O_{2+n}S$) + 16 for n = 6 to 17, respectively.The mass M + 16 corresponds to the oxidation of sulfur under the conditions of the analysis.

**1.4.11 Telomers LII-L4a(BOC) and III-L4b(BOC) from Telogen L4-SH, 6-deoxy-6-thio-1,2:3,4-di-O-isopropylidene-D-galactopyranose, and Taxogen A.**

The above described procedure for the synthesis of telomers III-L1a(BOC) to III-L1m(BOC) was applied to 16 mg (0.058 mmoles) telogen 6-deoxy-6-thio-1,2:3,4-di-O-isopropylidene-D-galactopyranose, 248 mg (1.16 mmoles) taxogen A (taxogen/telogen ratio of 20) and 4 mg (0.023 mmoles) AIBN in 2.3 ml anhydrous acetonitrile. It yielded after chromatography and fractionation to 205 mg telomer III-L4a(BOC) of aDP n = 15 and 46 mg III-L4b(BOC) of aDP n = 30. The aDP is determined by $^1H$ NMR.

Telomer III-L4a(BOC) (aDP n = 15). $^1H$ NMR ($CDCl_3$) : δ 1.00-1.60 (m, (9n+12)H, $CH_3$(BOC) and $(CH_3)_2C$); 1.60-2.60 (m, (3n-1)H, $CH_2CO$, CHCO and CHC*H*$_2$CH); 2.60-4.00 (m, (4n+4)H, $CH_2S$ and $CH_2NHCO$); 4.05-4.15 (m, 1H, $GalH_5$) ; 4.15-4.35 (m, 2H, $GalH_2$ and $GalH_4$) ; 4.55-4.65 (m, 1H, $GalH_3$) ; 5.40-5.50 (m, 1H, $GalH_1$) ; 5.50-6.50 (m, nH, NH). $^{13}C$ NMR ($CDCl_3$) : δ 24.4, 25.1 and 26.0 ((*C*$H_3$)$_2$C); 28.5 (*C*$H_3$(BOC)); 70.5, 70.9, 71.8 and 71.9 ($GalC_{2-5}$); 96.4 ($GalC_1$); 79.5 (C(BOC)); 108.6 and 109.3 (*C*$(CH_3)_2$); 156.7 (CO(BOC)). The resonances corresponding to the carbons CH*C*$H_2$CH, *C*H2NHCO, *C*HCO and NCO appear as large signals between 32 and 38 ppm, 38 and 45 ppm, and between 175 and 178 ppm, respectively. The *C*$H_2$S and *C*$H_2$CO carbons are not detectable. MS-ESI shows the presence of compounds with n = 2 to 21 : m/z = (276.3 + 214.2n + 23) with n = 2 to 10 in agreement with the masses calculated for [$C_{12}H_{20}O_5S$ + n($C_{10}H_{18}N_2O_3$) + $Na^+$] and n = 2 to 10; m/2z = (138.1 + 107.1n + 23) with n = 7 to 21 in agreement with the masses calculated for [$C_{12}H_{20}O_5S$ + n($C_{10}H_{18}N_2O_3$) + 2$Na^+$]/2 and n = 7 to 21.

Telomer III-L4b(BOC) (aDP n =30). $^1H$ NMR ($CDCl_3$) :the $^1H$ NMR spectrum is identical to that of telomer III-L4a(BOC) but with aDP n = 30.

**1.4.12 Telomers III-L4a(O)(BOC) and III-L4b(O)(BOC) from Telomers III-L4a(BOC) and III-L4b(BOC), respectively. Oxidation of Telomers**

Telomers III-L4a(O)(BOC) and III-L4b(O)(BOC) are obtained by oxidation of telomers III-L4a(BOC) and III-L4b(BOC), respectively. This oxidation is performed quantitatively by suspending these telomers in commercial $Et_2O$ and in the presence of air.

Telomer III-L4a(O)(BOC) (aDP n = 15). $^1H$ and $^{13}C$ NMR ($CDCl_3$) : identical to those of telomer III-L4a(BOC) (aDP n = 15), respectively. MS-ESI shows the presence of compounds with n = 2 to 27 : m/z = (292.3 + 214.2n + 23) with n = 2 to 13 in agreement with the masses calculated for [$C_{12}H_{20}O_6S$ + n($C_{10}H_{18}N_2O_3$) +$Na^+$] and n = 2 to 13; m/2z = (146.1 + 107.1n + 23) with n = 12 to 27 in agreement with the masses calculated for [$C_{12}H_{20}O_6S$ + n($C_{10}H_{18}N_2O_3$) + 2$Na^+$]/2 and n = 12 to 27.

Telomer III-L4b(O)(BOC) (aDP n = 30). $^1H$ NMR ($CDCl_3$) : identical to that of telomer III-L4a(BOC) (aDP n = 30). $^{13}C$ NMR ($CDCl_3$) : identical to that of telomer III-L4a(BOC) (aDP n = 15), but the Gal resonances are not detectable.

**1.4.13 Telomers III-L4a, III-L4a(O), III-L4b and III-L4b(O) from Telomers III-L4a(BOC), III-L4a(O)(BOC), III-L4b(BOC) and III-L4b(O)(BOC), respectively. Deprotection of Telomers**

The deprotection of telomers III-L4a(BOC), III-L4a(O)(BOC), III-L4b(BOC), and III-L4b(O)(BOC) is quantitatively achieved in a large excess of TFA at room temperature for 1 h. The excess of TFA is removed by coevaporation in the presence of cyclohexane. The telomers III-L4a, III-L4a(O), III-L4b and III-L4b(O) are obtained in the form of white solids.

Telomer III-L4a (aDP n = 15). $^1H$ NMR ($CD_3OD$) : δ 1.25-2.05 (m,(2n-2)H, C*H*$_2$CHCO); 2.05-2.95 (m, (n+5)H, $CH_2S$, $CH_2CO$ and CHCO); 2.95-3.90 (m, 4nH $CH_2N$), the Gal-H resonances are not detectable. $^{13}C$ NMR ($CD_3OD$) : δ 38.3 (bs, *C*H2NHCO) ; 40.4 (bs, CH2NH3) ; 118.3 (q, J = 292 Hz, CF3) ; 163.0 (q, J = 35 Hz, CF3CO) ; 178.0 (bs, NCO). The resonances corresponding to the carbons CH*C*$H_2$CH, and *C*HCO appear as large signals between 35 and 38 ppm, 42 and 48 ppm, respectively. The Gal, *C*$H_2$S and *C*$H_2$CO carbons are not detectable.

Telomer III-L4a(O) (aDP n = 15).$^1H$ NMR ($CD_3OD$) : identical to that of III-L4a (aDP n = 15). $^{13}C$ NMR ($CD_3OD$) : identical to that of III-L4a.

Telomer III-L4b (aDP n = 30). $^1H$ NMR ($CD_3OD$) : identical to that of III-L4a but with aDP n = 30. $^{13}C$ NMR ($CD_3OD$) : identical to that of III-L4a.

Telomer III-L4b(O) (aDP n= 30).$^1H$ NMR ($CD_3OD$) : identical to that of III-L4a but with aDP n = 30. $^{13}C$ NMR ($CD_3OD$) : identical to that of III-L4a.

### 1.4.14 Telomer III-L0(BOC) from Telogen L0-SH (1-propanethiol) and Taxogen A.

The above described procedure for the synthesis of telomers III-L1a(BOC) to III-L1m(BOC) was applied to 0.25 ml (0.023 mmoles) of a 0.092 N solution of 1-propanethiol (Aldrich) in anhydrous acetonitrile, 200 mg (0.93 mmoles) taxogen A (taxogen/telogen ratio of 40) and 2 mg (0.012 mmoles) AIBN and 1.75 ml anhydrous acetonitrile. It yielded after chromatography and fractionation to 168 mg telomer III-L0(BOC).

Telomer III-L0(BOC): $^1H$ NMR ($CDCl_3$): δ 1.35 (s, 9nH, $CH_3$(BOC)) and 0.90-2.80 (m, (3n+1)H, $CH_2CO$ and $CH_2CHCO$) ; 2.80-3.90 (m, 4n, $CH_2N$). $^{13}C$ NMR ($CDCl_3$) : δ 28.7 ($CH_3$(BOC)), 79.5 (C, BOC); 156.7 (CO, BOC); the resonances corresponding to the carbons CH*C*H$_2$CH, *C*H$_2$N, *C*HCO and NCO appear as large signals between 32 and 38 ppm, 38 and 45 ppm, and between 175 and 178 ppm, respectively. The propyl $^1H$ and $^{13}C$ resonances are not detectable.

### 1.4.15 Telomer III-L0 from Telomer III-L0(BOC): Deprotection of Telomer III-L0(BOC)

The deprotection of 150 mg telomer III-L0(BOC) is achieved by dissolving and keeping this telomer in a large excess of TFA at room temperature for 1 h. The excess of TFA is removed by coevaporation in the presence of cyclohexane. The residue is washed with $CHCl_3$, and chromatographed on a Sephadex LH 20 column (eluent $CH_3OH$) giving 100 mg telomer III-L0 in the form of a white solid.

Telomer III-L0 : $^1H$ NMR ($CD_3OD$): δ 1.20-2.60 (m, (3n+1)H, $CH_2CO$ and $CH_2CHCO$) ; 2.80-3.30 (m, 2n, $CH_2NCO$) ; 3.30-3.80 (m, 2n, $CH_2N$). $^{13}C$ NMR ($CDCl_3$) : δ 38.1 (ls, *C*H$_2$NHCO), 40.3 (ls, $CH_2NH_3$), 117.7 (q, J = 292 Hz, $CF_3$) ;162.4 (q, J = 35 Hz, $CF_3CO$); 177.9 (ls, NCO) ; the resonances corresponding to the carbons CH*C*H$_2$CH and *C*HCO appear as large signals between 32 and 38 ppm, and between 42 and 45 ppm, respectively. The propyl $^1H$ and $^{13}C$ resonances are not detectable.

### 1.4.16 Telomers IV-L1a and IV-L1b from Telogen L1-SH (compound 3a) and Taxogen B

A solution of 112 mg (0.18 mmoles) telogen 3a, 164 mg (0.91 mmoles) taxogen B (i.e. a molar ratio taxogen /telogen of 5) and 12 mg (0.07 mmoles) AIBN in 2 ml anhydrous methanol is stirred under heating (60°C). After 24 h, 5 mg (0.03 mmoles) AIBN are added to the reaction mixture and left under stirring at 60 °C for 4 h, until the taxogen has disappeared (followed by TLC/DTNB, $KMnO_4$). After evaporation, the residue is taken up in $CHCl_3$ and filtered. The precipitate is fractionated on a Sephadex LH 20 column (eluent $CH_3OH$). 37 mg telomer IV-L1a of aDP n = 35 and 114 mg telomers IV-L1b of aDP n = 80 are obtained in the form of white solids. The aDP is determined by $^1H$ NMR.

Telomer IV-L1a (aDP n = 35). $^1H$ NMR ($CDCl_3$+$CD_3OD$): δ 0.80 (t, 6H, $^3J$ = 6.0 Hz, $CH_3$); 1.10-1.30 (m, 60H, $(CH_2)_{15}$); 1.30-1.90 (m, (2+2n)H, *C*H$_2$CH$_2$NCO and *C*H$_2$CHCO); 1.90-2.80 (m, (7+n)H, $CH_2S$, $CH_2CO$ and CHCO); 2.80-3.10 (m, 6nH, $(CH_3)_2N$); 3.10-4.00 (m, (4+4n)H, $CH_2N$ and $CH_2NH$). Telomer IV-L1a (aDP n = 35). $^{13}C$ NMR ($CDCl_3$+$CD_3OD$) : δ 14.6 ($CH_3$) ; 23.7 (*C*H$_2$CH$_3$) ; 27.1, 28.1, 28.8, 30.4, 30.7 ($(CH_2)_{15}$) ; 33.0 (*C*H$_2$CH$_2$CH$_3$) ; ; 36.0 (bs, CONH*C*H$_2$CH$_2$N(CH$_3$)$_2$) ; 44.2 (bs, $N(CH_3)_2$) ; 58.1 (bs, *C*H$_2$N(CH$_3$)$_2$) ; 177.8 (bs, NCO). The resonances corresponding to the carbons CH*C*H$_2$CH and *C*HCO, appear as large signals between 35 and 38 ppm and between 40 and 48, respectively. The $CH_2S$, $CH_2N$ and *C*H$_2$CO carbons are not detectable.

Telomer IV-L1b (aDP n = 80). $^1H$ NMR ($CD_3OD$) : The $^1H$ NMR spectrum is identical to that of telomer IV-L1a but with aDP n = 80. The $^{13}C$ NMR spectrum is identical to that of telomer IV-L1a.

### 1.4.17 Co-telomers VII-L1a(BOC) from Telogen L1-SH (compound 3a), and Taxogenes A and B

129 mg (0.21 mmoles) telogen 3a are added to a solution of 38 mg (0.21 mmoles) taxogen B and 455 mg (2.12 mmoles) taxogen A in 4.5 ml anhydrous $CH_3OH$ (telogen/taxogen A/B molar ratio of 1/10/1). The mixture is heated to 40°C and 14 mg (0.08 mmoles) AIBN are then added. The resulting reaction mixture is stirred under heating (62°C) during 24 h, until the telogen 3a has disappeared (followed by TLC/DTNB). The solution is concentrated and chromatographed on a Sephadex LH 20 column (eluent $CH_3OH$). The residue is washed with n-hexan giving 555 mg co-telomer VII-L1a(BOC) of aDP1 n = 10 (corresponding to the number of A units) and aDP2 n' = 1(corresponding to the number of B units), in the form of a white solid. The aDP values were determined by $^1H$ NMR.

Co-telomer VII-L1a(BOC) (aDP n = 10 and n' = 1). $^1H$ NMR ($CDCl_3$) : δ 0.88, (t, 6H, J= 6.0 Hz, $CH_3$) ; 1.05-1.30 (m, 60H, $(CH_2)_{15}$); 1.30-1.60 (m, (9n+4)H, *C*H$_2$CH$_2$N and $CH_3$(BOC)); 1.60-2.00 (m, (2n+2n'-2)H, *C*H$_2$CHCO) ; 2.00-2.85 (m, (n+n'+7)H, $CH_2S$, $CH_2CO$ and CHCO) ; 2.85-3.05 (m, 6n'H, $(CH_3)_2N$) ; 3.05-3.90 (m, (4n+4n'+4)H, $CH_2N$) ; 5.40-6.50 (m, nH, NH(BOC)). $^{13}C$ NMR ($CDCl_3$) : δ 14.1 ($CH_3$) ; 22.7 (*C*H$_2$CH$_3$) ; 28.5 ($CH_3$(BOC)) ; 27.0, 27.1, 27.8, 29.4, 29.7 ($(CH_2)_{15}$) ; 31.9 (*C*H$_2$CH$_2$CH$_3$) ; 79.4 (bs, C(BOC)); 156.6 (bs, CO(BOC)). The resonances corresponding to the carbons CHCH$_2$CH, $CH_2N$, $N(CH_3)_2$, *C*HCO and NCO appear as large signals between 32 and 38 ppm, 38 and 48 ppm, and between 175 and 178 respectively. The *C*H$_2$S, *C*H$_2$N(CH$_3$)$_2$, and *C*H$_2$CO carbons are not

detectable.

### 1.4.18 Co-telomers VII-L1b(BOC) from Telogen L1-SH (compound 3a), and Taxogenes A and B

To a solution of 40 mg (0.065 mmoles) telogen 3a in 2 ml anhydrous acetonitrile is added a solution of 140 mg (0.65 mmoles) taxogen A and 117 mg (0.65 mmoles) taxogen B in 1 ml anhydrous methanol (telogen/taxogen A/B molar ratio of 1/10/10). 5 mg (0.03 mmoles) AIBN are added to the heated (80°C) reaction mixture and stirred under heating (60°C) during 19 h, until the telogen 3a has disappeared (followed by TLC/DTNB). The solution is concentrated and chromatographed on a Sephadex LH 20 column (eluent $CHCl_3$). 282 mg telomer VII-L1b(BOC) of aDP1 n = 10 and aDP2 n' = 17 are obtained in the form of a pale yellow solid.

Co-telomer VII-L1b(BOC) (aDP n = 10 and n' = 17). $^1H$ NMR ($CD_3OD$) : the $^1H$ NMR spectrum is identical to that of telomer VII-L1a(BOC) but with aDP n = 10 and n'=17. $^{13}C$ NMR ($CDCl_3$) : δ 14.2 ($CH_3$) ; 22.8 ($\mathit{C}H_2CH_3$) ; 28.7 ($CH_3$(BOC)) ; 29.4, 29.8 ($(CH_2)_{15}$) ; 32.0 ($\mathit{C}H_2CH_2CH_3$) ; 43.7 (bs, $N(CH_3)_2$); 79.5 (bs, C(BOC)) ; 156.7 (bs, CO(BOC)). The resonances corresponding to the $CH\mathit{C}H_2CH$, $CH_2NH$, $\mathit{C}HCO$ and NCO carbons appear as large signals between 32 and 38 ppm, 38 and 48 ppm, and between 175 and 178, respectively. The $\mathit{C}H_2S$, $\mathit{C}H_2N(CH_3)_2$, and $\mathit{C}H_2CO$ carbons are not detectable.

### 1.4.19 Co-telomers VII-L1a and VII-L1b : Deprotection of Telomers VII-L1a(BOC) and VII-L1a(BOC)

The deprotection of co-telomers VII-L1a(BOC) and VII-L1b(O)(BOC) is quantitatively achieved in a large excess of TFA at room temperature for 1 h. The excess of TFA is removed by coevaporation in the presence of cyclohexane. The co-telomers VII-L1a, VII-L1b are obtained in the form of white solids.

Co-telomer VII-L1a (aDP n = 10 and n' = 1). $^1H$ NMR ($CD_3OD$): δ 0.95 (t, 6H, $CH_3$) ; 1.15-1.45 (m, 60H, $(CH_2)_{15}$) ; 1.45-2.00 (m, (2n+2n'+2)H, $\mathit{C}H_2CH_2N$ and $\mathit{C}H_2CHCO$) ; 2.00-2.90 (m, (n+n'+5)H, $CH_2S$, $CH_2CO$ and CHCO) ; 2.90-3.05 (m, 6n', $(CH_3)_2N$); 3.05-3.90 (m, (4n+4n'+4)H, $CH_2N$). )). $^{13}C$ NMR ($CDCl_3$) : δ 14.4 ($CH_3$) ; 23.6 ($\mathit{C}H_2CH_3$) ; 27.7, 27.9, 30.3, 30.4, 30.7 ($(CH_2)_{15}$) ; 33.0 ($\mathit{C}H_2CH_2CH_3$) ; 34.1 and 34.3 (bs, $CONH\mathit{C}H_2CH_2N(CH_3)_2$) ; 38.2 (bs, $CONH\mathit{C}H_2CH_2NH_3$) ; 40.4 (bs, $CH_2NH_3$) ; 43.8 (bs, $N(CH_3)_2$); 58.0 (bs, $CH_2N(CH_3)_2$) ; 118.0 (q, J = 292 Hz, $CF_3$) ; 162.8 (q, J = 35 Hz, CF3CO) ; 177.9 (bs, NCO). The resonances corresponding to the $CH\mathit{C}H_2CH$ carbons appear as large signal between 35 and 38 ppm. The $CH_2S$, $\mathit{C}HCO$, $CH_2N$ and $\mathit{C}H_2CO$ carbons are not detectable.

Co-telomer VII-L1b (aDP n = 10 and n' = 17). $^1H$ NMR ($CD_3OD$): The $^1H$ NMR spectrum is identical to that of telomer VII-L1a but with aDP n = 10 and n'=17. )). $^{13}C$ NMR ($CDCl_3$) : δ 14.3 ($CH_3$) ; 23.6 ($\mathit{C}H_2CH_3$) ; 30.3, 30.6 ($(CH_2)_{15}$) ; 32.9 ($\mathit{C}H_2CH_2CH_3$) ; 35.7 (bs, $CONH\mathit{C}H_2CH_2N(CH_3)_2$) ; 38.2 (bs, $CONH\mathit{C}H_2CH_2NH_3$) ; 40.5 (bs, $CH_2NH_3$) ; 43.9 (bs, $N(CH_3)_2$) ; 58.0 (bs, $\mathit{C}H_2N(CH_3)_2$) ; 118.2 (q, J = 292 Hz, $CF_3$) ; 162.5 (q, J = 35 Hz, $CF_3CO$) ; 177.8 (bs, NCO). The resonances corresponding to the carbons $CH\mathit{C}H_2CH$ appear as large signal between 35 and 38 ppm. The $CH_2S$, $\mathit{C}HCO$, $CH_2N$ and $\mathit{C}H_2CO$ carbons are not detectable.

### Example 2: Preparation of complexes composed of the polyamine telomer compounds of the invention and of the plasmid pTG11033 (French patent application 97/08267).

The quantities of compound used in the invention are calculated according to the desired DNA concentration of 0.1 mg/ml (for *in vitro* tests), the charge ratio, the molar weight and the number of potential positive charges in the compound selected according to the invention. The polyamine telomer/DNA complex is formulated by adding a desired volume of telomer preparation at a concentration of 10 mg/ml (in 20 mM HEPES buffer, pH 7.5, containing 10% DMSO and 10% EtOH) to the desired volume of DNA solution to reach desired DNA concentration (for example 0.1 mg/ml). Thus for the preparation of the polyamine telomer III-L 1h/DNA complex at charge ratio 5 and 0.1 mg/ml DNA, 40 μl of a polyamine telomer solution (10 mg/ml in CHCl3) are transferred to a borosilicate glass tube (16x100 mm). The solvent is evaporated in Rotavap evaporation system (45°C, 30 pm, 0.2 bar, 40 mm). 4 μl of DMSO, 4 μl of EtOH and 32 μl of HEPES 20 mM, pH 7.5 are added to the film obtained. The preparation is heated at 50°C, while stirring (30 rpm), for 5 min. After cooling to room temperature, 37 μl of this preparation are added to 963 μl DNA solution [(100 μl DNA (1 mg/ml) diluted with 863 μl HEPES buffer)]. This preparation can be used for further experiments.

When the composition further comprises DOPE, the desired volume of 1 mg/ml chloroform solution (to get mole/mole cationic telomer/DOPE) is added to the volume of polyamine telomer solution (10 mg/ml in CHCl3) and then the mixture is transferred to a borosilicate glass tube. The following steps are as described above.

### Example 3: Measurement of the size of the complexes formed

The analyses are performed on a Coulter N4Plus (Coultronics France S.A., Margency, France) at 25°C, after equilibrating the sample at 25°C for 20 minutes. One aliquot of the sample is aspirated and discharged several times

prior to pipetting. The sample is diluted in the measurement tube and homogenized. These analysis were thus carried out on complexes having a 10 µg/ml concentration of DNA. After waiting 180 seconds, the light diffraction at 90° is measured for 180 seconds. The range is 3 nm to 10.000 nm, using 31 bins. The size measurement is valid if the sample gives between 50.000 and 1.000.000 counts/second.

The capability of polyamine telomer compounds having a common hydrophobic part ($C_{18}$) and a variable number of amine groups to condense DNA was also analyzed. All the polyamine telomer compounds containing 6 to 60 primary amines are able to condense DNA to a size of less than 100 nm for a charge ratio of 10. Table 1 below shows that smaller (~50 nm) complexes can also be obtained, with or without addition of DOPE. The number of primary amines seems to affect the size of the complexes composed of polyamine telomer compounds such as III-L3c (n=6) or III-L2a (n=2) which are found to have a larger size than those obtained with III-L1g (n=20), III-L1h (n=40) or III-L1j (n=60).

Table 1

| Lipopolyamine | Charge Ratio +/- | DOPE | Diameter (nm) |
|---|---|---|---|
| III-L2a (n=2) | 10 | No | 96 |
| III-L2a (n=2) | 5 | No | 86/7137 |
| III-L2a (n=2) | 2.5 | No | 116 |
| III-L3c (n=6) | 10 | No | 89 |
| III-L3c (n=6) | 10 | Yes | 98 |
| III-L3d (n=11) | 10 | No | 52 |
| III-L3d (n=11) | 10 | Yes | 81 |
| III-L3e (n=16) | 10 | No | 47/103 |
| III-L3e (n=16) | 5 | No | 117 |
| III-L3e (n=16) | 10 | Yes | 12/80 |
| III-L3e (n=16) | 5 | Yes | 81 |
| III-L1g (n=20) | 10 | No | 61 |
| III-L1g (n=20) | 5 | No | 74 |
| III-L1g (n=20) | 2.5 | No | 84 |
| III-L1g (n=20) | 10 | Yes | 53 |
| III-L1g (n=20) | 5 | Yes | 53 |
| III-L1g (n=20) | 2.5 | Yes | 77 |
| III-L1h (n=40) | 10 | No | 63 |
| III-L1h (n=40) | 10 | Yes | 69 |
| III-L1h (n=40) | 2.5 | Yes | 115/327 |
| III-L1j (n=60) | 10 | No | 71 |
| III-L1j (n=60) | 5 | No | 60 |
| III-L1j (n=60) | 2.5 | No | 218 |
| III-L1j (n=60) | 10 | Yes | 56 |
| III-L1j (n=60) | 5 | Yes | 54 |
| III-L1j (n=60) | 2.5 | yes | 68 |

**Example 4: Efficiency of the transfection of A549 cells.**

Twenty-four hours before transfection, A549 cells (epithelial cells derived from human pulmonary carcinoma)

are cultivated in Dulbeco-modified Eagle culture medium (DMEM), containing 10% fetal calf serum (Gibco BRL), in 96 multi-wells plates wells (2 x $10^4$ cells per well), in a humid (37°C) and 5% $C0_2$/95% air atmosphere.Volume of DNA/polyamine telomer complex (40, 20, 5 and 1 µl, respectively) is diluted to 100 µl in DMEM or DMEM supplemented wit 10% fetal calf serum (for transfection performed in the presence of serum) in order to obtain various amounts of DNA (4, 2, 0.5 and 0.1 µg, respectively) in the preparation. The culture medium is removed and replaced by 100 µl of DMEM with or without 10% serum containing the desired amount of DNA. 50 µl of DMEM + 30% fetal calf serum (or 10% for the transfections made with serum) are added. After 20 hours, 100 µl of DMEM + 10% serum are added.

48 hours after transfection, the culture medium is discarded and the cells are washed with 100 µl of phosphate solution PBS and Lysol with 50 µl of lysis buffer (Promega). The lysates are frozen at -80°C awaiting analysis of luciferase activity. This measurement is done for one minute on 20 µl of the lysis mixture in a Berthold LB96P luminometer in dynamic mode, using the "Luciferase" determination system (Promega) in 96-well plates,

The results are illustrated in Figures 1, 2, 3 and 4. The values are expressed in fg luciferase per mg of protein. The total protein concentration per well is determined using conventional techniques (BCA test, Pierce). For example, a well contains round 20 to 30 µg of protein.

Cells treated with naked DNA under equivalent conditions showed expression level of about 50 fg luciferase/mg of protein.

These results indicate that the polyamines of the invention enable transfection of the plasmid into the cells and that the transfection efficiency depends on the length of polyamine chain, on the charge ratio as well as on the amount of DNA. The presence of an equimolar quantity of DOPE is more efficient than the use of complexes composed of strongly charged polyamines (III-L1g, III-L1h or III-L1j), especially in the presence of serum.

**Example 5: New complexes tested :**

2 µg of plasmid (pTG11033) and the desired amount of 10 mM cationic polymer pcTG146 (concentration corresponding to the monomeric amine) were each diluted to 10 µL with 20 mM HEPES buffer, pH 7.5 solution and vortexed. The two solutions were then mixed and gently vortexed. After 15 min the complexes formed were added to the cells.

HeLa cells were plated in 48 multi-wells plate to reach 70-80% confluence at time of transfection. They were transfected with 2 µg pTG11033 formulated with the desired amount of temoler pcTG146 to obtain N/P ratios of 1, 1.5, 2.5 and 5. The final concentration of DNA was 0.1 g/l. Transfection was carried out in the absence of serum and after 3 hours, medium was removed and replaced with fresh one supplemented with 10% fetal calf serum. Expression was stopped 48 hours later and luciferase quantitated. Each condition was performed in triplicate and results are expressed as mean value ± SD (standard deviation).

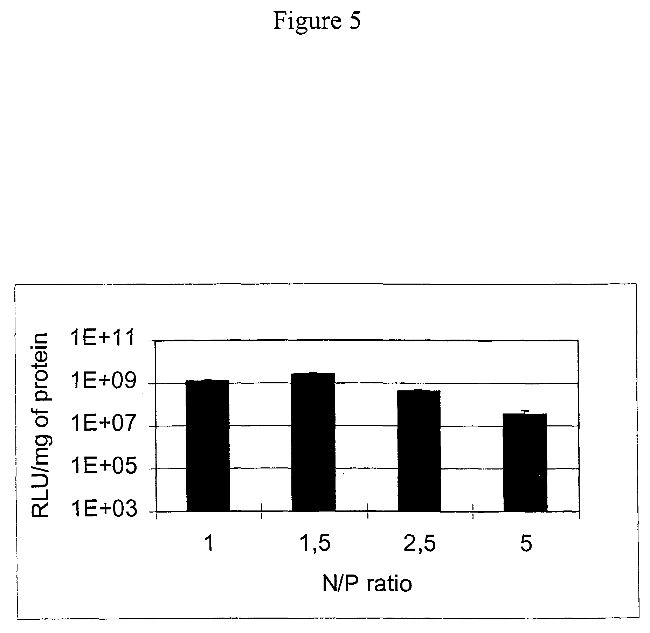

Figure 5 represents the transfection efficiency obtained at different N/P ratios. As shown in the figure, the pcTG146 (corresponding to telomer compound III-L0 with L0 = propyl) led to significant transgene expression at all N/P ratios tested.

Measurement of the size of the complexes formed : (see example 3 for details)

The complexes were prepared as described above. 60 µL were diluted in 540 µL of 20 mM HEPES buffer, pH 7.5 and size measured. Following table summarizes the results obtained.

| Charge Ratio +/- | Diameter (nm) |
|---|---|
| 5 | 91 ± 88 |
| 2.5 | 66 ± 34 |
| 1.5 | 115 ± 33 |
| 1 | 147 ± 42 |

The size of the complexes pcTG146/pTG11033 were relatively small (<150 nm) whatever the charge ratio used.

## Claims

1. Polyamine telomer compound comprising the group of the Formula I:

I 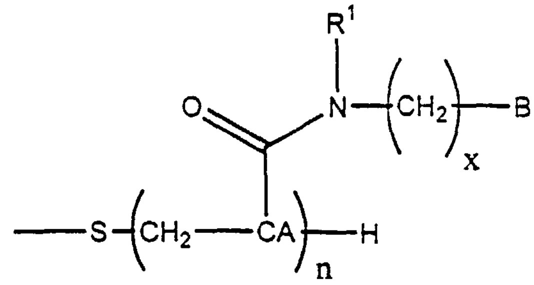

wherein:

A is H or an alkyl of 1 to 4 carbon atoms or an aryl of 5 to 7 carbon atoms,
the degree of polymerization n ranges from 1 to 100,
$R^1$ is selected, independently of one another in each $[CH_2\text{-}CA]_n$ repeat, from -H, $-CH_3$, $-C_2H_5$ and $-(CH_2)_u\text{-}B$,
x and u are integers from 2 to 4,
B being

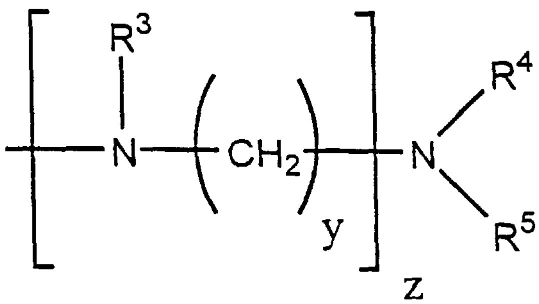

or

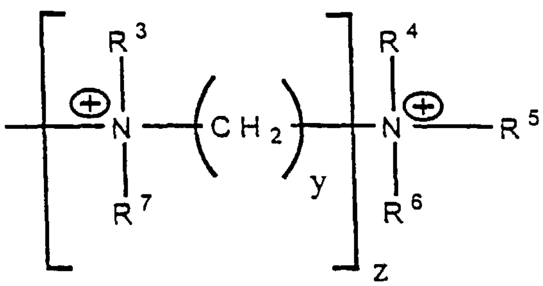

wherein:
y is an integer from 2 to 4,
z is an integer from 0 to 6,
$R^3$, $R^4$, $R^5$, $R^6$ and $R^7$ are selected, independently of one another, from H, alkyl or hydroxyalkyl of 1 to 4 carbon atoms.

**2.** The polyamine telomer compound of claim 1, wherein said compound is of formula II:

II

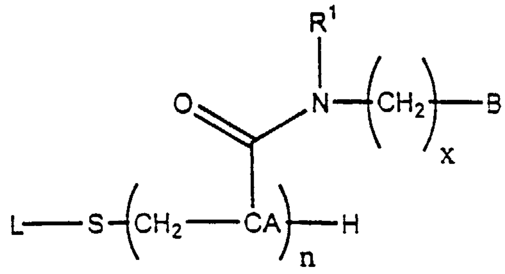

wherein:

A, $R^1$, B, n and x are as defined in claim 1,
L is selected from groups L0, L1, L2 and L3,
wherein L0 being selected from H, alkyl of 1 to 4 carbon atoms, optionally substituted with at least one substituent selected from OH, COOH and $NH_2$ and L1, L2 and L3 having the formulas:

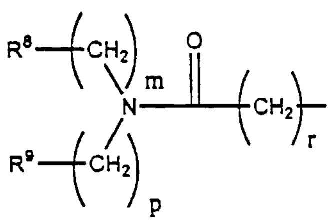

L1

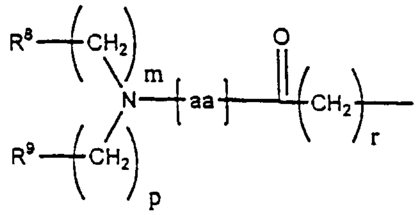

L2

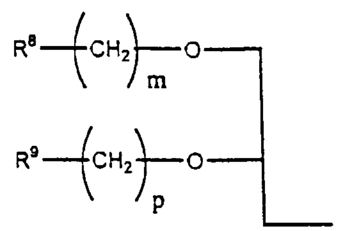

L3

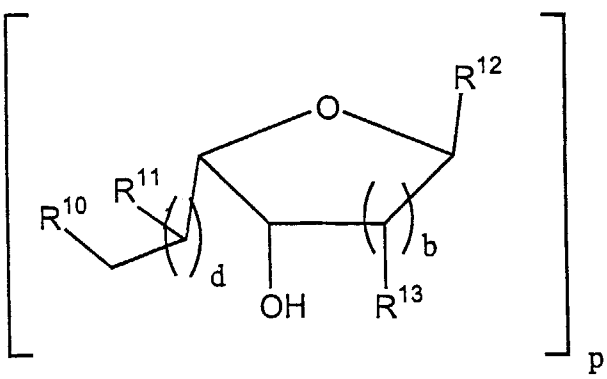

L4

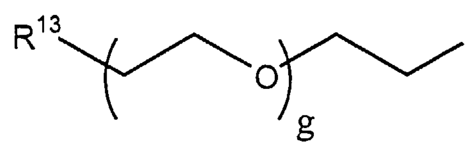

L5

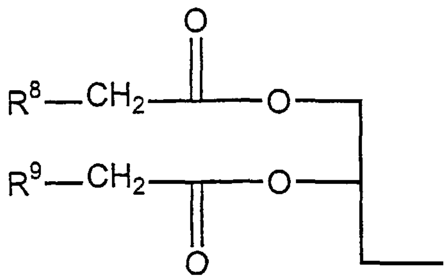

**L6**

wherein:

r = 1, 2 or 3,

b =1 or 2 and d= 0 or 1, b and d can be different of one another in each p repeat,

g ranges from 0 to 200,

m and p ranges, independently of one another from 1 to 20, preferably from 8 to 17,

$R^8$, $R^9$ are selected, independently of one another, from $CH_3$-, $CH_3$-$(CH_2)_w$-CH=CH-(cis or tans) with w = 5 or 7, $C_sF_{2s+1}$ or $C_sF_{2s+1}$-CH=CH- (cis or tans) with s = 2, 4, 6, 8 or 10,

at least one of $R^{10}$, $R^{11}$ or R $^{12}$ is the binding site where L4 is linked with S of the compound of formula II, the other one being selected, independently of one another, from the group -H, -OH, =O, $NH_2$, -NH-$COCH_3$ , alkyl radical of 1 to 4 carbon atoms optionally substituted with at least one substituent selected from OH, COOH and $NH_2$, especially -$CH_3$, -$C_2H_5$, -$C_3H_7$, -$CH_2CH_2$-OH, -$CH_2CH_2CH_2$-OH, -$CH_2CH_2$-COOH, -$CH_2$-CH(OH)-$CH_2$-OH, -$CH_2CH_2CH_2$-COOH, and $R^{10}$, $R^{11}$ or $R^{12}$ can be different of one another in each p repeat,

$R^{13}$ is selected from the group -H, -OH, -$NH_2$, -$NHCOCH_3$, alkyl radical of 1 to 4 carbon atoms optionally substituted with at least one substituent selected from OH, COOH and $NH_2$, especially -$CH_3$, -$C_2H_5$, -$C_3H_7$, -$CH_2CH_2$-OH, -$CH_2CH_2CH_2$-OH, -$CH_2CH_2$-COOH, -$CH_2$-CH(OH)-$CH_2$-OH, -$CH_2CH_2CH_2$-COOH, $R^{13}$ can be different of one another in each b repeat when b = 2,

[aa] is an $\alpha$-amino acid, bonded to the amine residue via its acid function and to the $CO(CH_2)_r$ moiety via its amine function, said $\alpha$-amino acid comprising an amine/ammonium function with a weak pKa.

**3.** The polyamine telomer compound of claim 2, wherein said amine/ammonium function with a weak pKa is an imidazole/imidazolium or a carboxylate/carboxylic function.

**4.** The polyamine telomer compound of claims 2 or 3, wherein said $\alpha$-amino acid is selected from the group consisting of histidine, 3-methylhistidine, aspartic and glutamic acids.

**5.** The polyamine telomer compound of claims 1 to 4, wherein n is an integer from 2 to 60.

**6.** The polyamine telomer compound of claims 1 to 5, wherein z = 0.

**7.** The polyamine telomer compound of claims 1 to 6, wherein said compound also contains one or more targeting elements bonded via at least one secondary or primary nitrogen atom of the polyamine part of said compound.

**8.** The polyamine telomer compound of claim 7, wherein said targeting element is selected from the group consisting in all or part of sugars, glycol derivatives, peptides, oligonucleotides, lipids, hormones, vitamins, antigens, antibodies, specific membrane receptor ligands, ligands capable of reaction with an anti-ligand, fusogen peptides, nuclear localization peptides, or a combination of said compounds

**9.** The polyamine telomer compound of claim 2 wherein said compound includes fluorinated or perfluoroalkyl termini on their lipidic part.

**10.** The polyamine telomer compound of claim 2, wherein said compound is selected from compounds of the Formula III-L1:

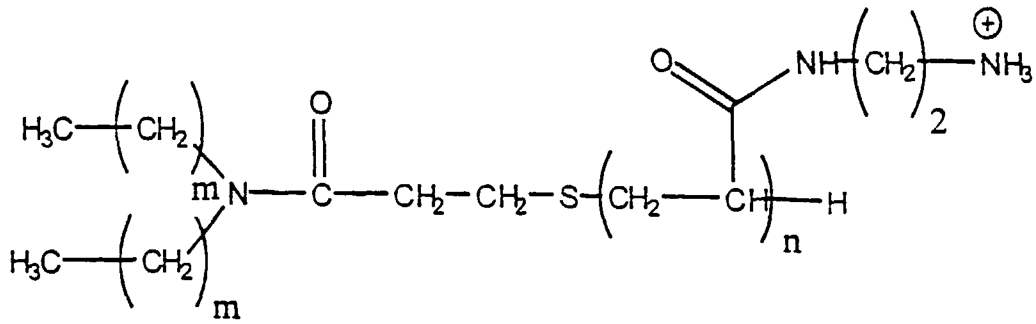

**III-L1**

wherein m=p , namely:
wherein m=p , namely:

compound III-L1a wherein m=17 and n=1
compound III-L1b wherein m=17 and n=2
compound III-L1c wherein m=17 and n=3
compound III-L1d wherein m=17 and n=4
compound III-L1e wherein m=17 and n=5
compound III-L1f wherein m=17 and n=10
compound III-L1g wherein m=17 and n=20
compound III-L1h wherein m=17 and n=40
compound III-L1i wherein m=17 and n=50
compound III-L1j wherein m=17 and n=60
compound III-L1k wherein m=3 and n=10
compound III-L1l wherein m=13 and n=20,
compound III-L1m wherein m = 13 and n= 70 and
compounds of the Formula III-L2

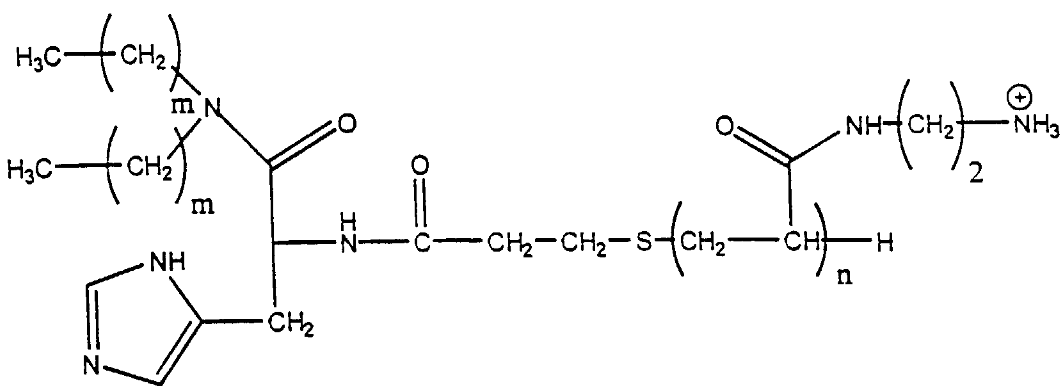

**III-L2**

wherein m=p , namely:
compound III-L2a wherein m=17 and n=2
compound III-L2b wherein m=17 and n=20
compound III-L2c wherein m=17 and n=30
compound III-L2d wherein m=17 and n=90
and compounds of Formula III-L3

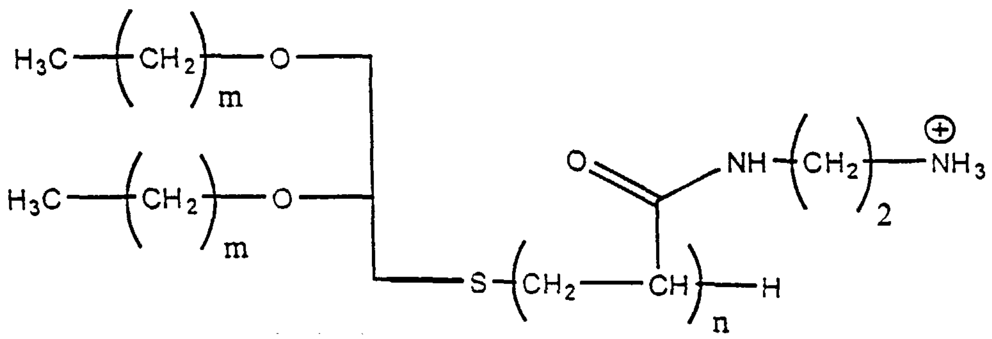

III-L3

wherein m=p namely:

compound III-L3a wherein m=17 and n=1
compound III-L3b wherein m=17 and n=3
compound III-L3c wherein m=17 and n=6
compound III-L3d wherein m=47 and n=11 and
compound III-L3e wherein m=17 and n=16.

11. The polyamine telomer compound of claims 1 to 10 wherein the thioether group has been oxidized into a sulfone (-$SO_2$-) or sulfoxide (-SO-).

12. Composition comprising at least one polyam ine telomer compound of claims 1 to 11 type and optionally at least one adjuvant.

13. Composition of claim 12, wherein said adjuvant is a negatively charged, neutral or zwitterionic lipid.

14. Composition of claim 13, wherein said adjuvant is selected from phosphatidylethanolamine (PE), phosphatidylcholine, phosphocholine, sphingomyelin, ceramide or cerebroside or one of their derivates.

15. Composition of claim 14, wherein said adjuvant is dioleylphosphatidylethanolamine (DOPE).

16. Composition of claim 12 or 13 wherein said adjuvant is selected from cationic lipid compounds.

17. Complex for transfering an active substance into a cell, said complex comprising at least one compound of claims 1 to 11 and/or at least one composition of claims 12 to 16 and at least one active substance comprising at least one negative charge.

18. Complex of claim 17, wherein said active substance is a nucleic acid or a protein.

19. Complex of claim 18, wherein said active substance is a nucleic acid selected from the group consisting of cDNA, genome DNA, plasmid DNA, messenger RNA, antisense RNA, ribozymes, transfer RNA, ribosomal RNA, or DNA encoding for such types of RNA.

20. Complex of claim 19, wherein said nucleic acid includes a therapeutically useful gene sequence and the elements enabling its expression.

21. Complex of claims 17 to 20, wherein said complex size is less than 500 nm.

22. Complex of claim 21, wherein said complex size ranges between 20 and 100 nm.

23. Complex of claims 17 to 22, wherein the ratio between the number of positive charges in said compound and/or said composition and the number of negative charges in the active substance is between 0.05 and 20.

24. Process for the preparation of a complex of claims 17 to 23 comprising the following steps:

- contacting at least one compound of claims 1 to 11 and/or at least one composition of claims 12 to 16 with at least one active substance, notably therapeutic substances, incorporating at least one negative charge
- and recovering said complex, eventually after a purification step.

**25.** Process of claim 24, wherein said compound and/or composition are first dissolved in a water-miscible solvent, preferably ethanol or dimethylsulfoxide or a mixture of the two.

**26.** Process of claim 24, wherein a suspension of said compound and/or composition is first prepared in a detergent solution.

**27.** Process of any of claims 24 to 26, wherein said process includes an additional step in which the complex is purified by dialysis.

**28.** Process of any of claims 24 to 27, wherein said compound and/or composition are subjected to a prior sonication/extrusion step.

**29.** Use of a compound of claims 1 to 11, or a composition of claims 12 to 16, or a complex of claims 17 to 23 for transfering *in vitro* of at least one active substance, preferably a nucleic acid, into a cell.

**30.** Use of claim 29, wherein said cell is a mammalian cell.

**31.** Process for in vitro transferring at least one active substance into a cell, wherein said cell is contacted wit a complex of claims 17 to 23.

**32.** Use of a compound of claims 1 to 11, a composition of claims 12 to 16, or a complex of claims 17 to 23 for preparing a pharmaceutical composition for curative, preventive or vaccinal treatment of man or animals, and more specifically for gene therapy use.

**33.** Use according to claim 32, wherein said pharmaceutical composition is administered by intramuscular injection, by inhalation, by intratracheal injection, by instillation, by aerosolization or by topical application.

**34.** Pharmaceutical composition comprising at least one complex of claims 17 to 23.

**35.** Pharmaceutical composition of claim 34, comprising at least one adjuvant.

**36.** Pharmaceutical composition of claim 35, wherein said adjuvant is selected from chloroquine, protic polar compounds such as propylene glycol, polyethylene glycol, glycerol, ethanol, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethyl urea, acetonitrile or their derivatives.

**37.** Pharmaceutical composition of any of claims 34 to 36, comprising a pharmaceutically acceptable medium enabling its administration in man or animals.

**38.** Cell transfected by a complex of claims 17 to 23.

Fig. 1

in vitro transfection with polyamine telomers

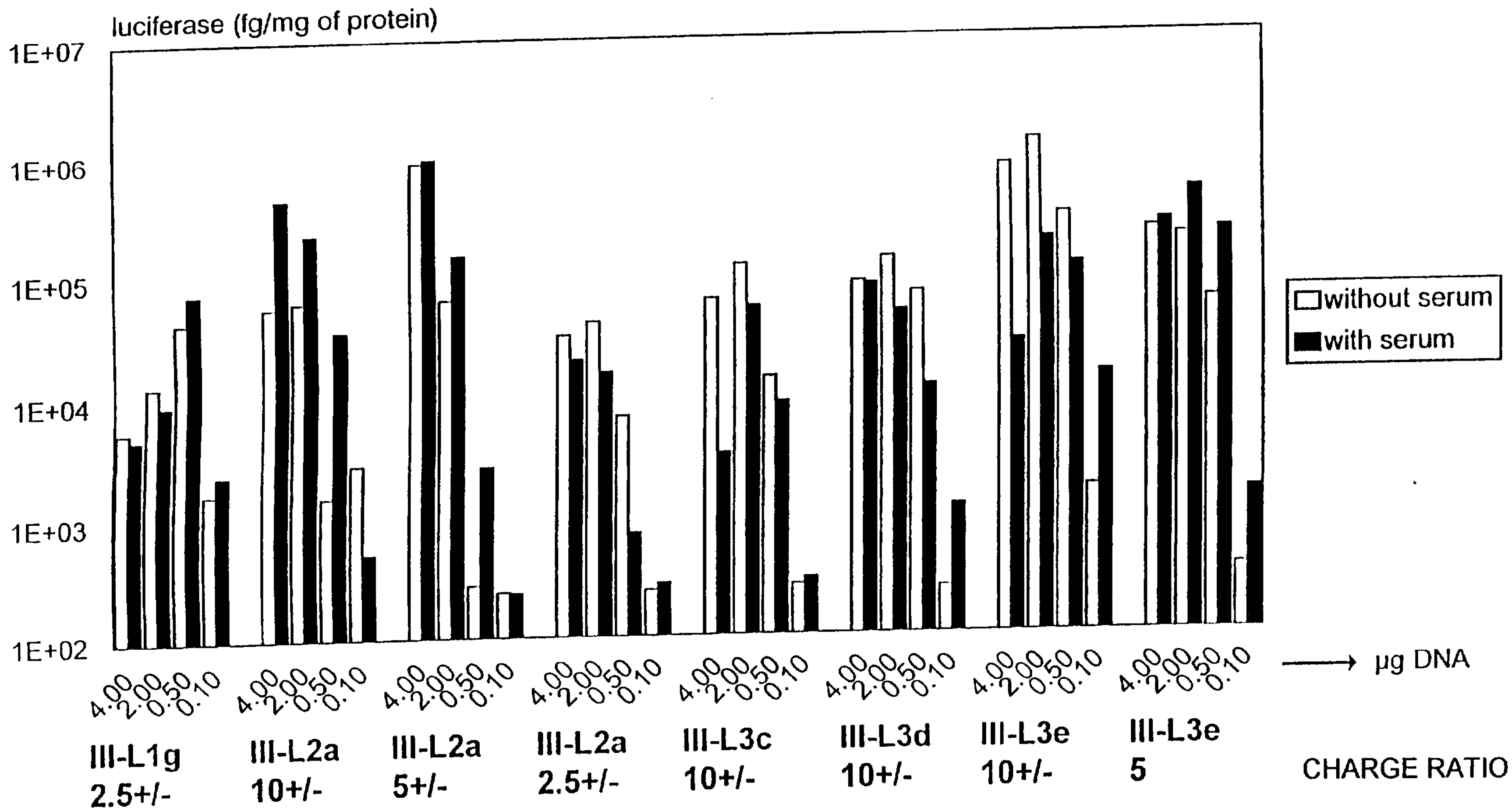

Fig. 2

in vitro transfection with polyamine telomers/DOPE

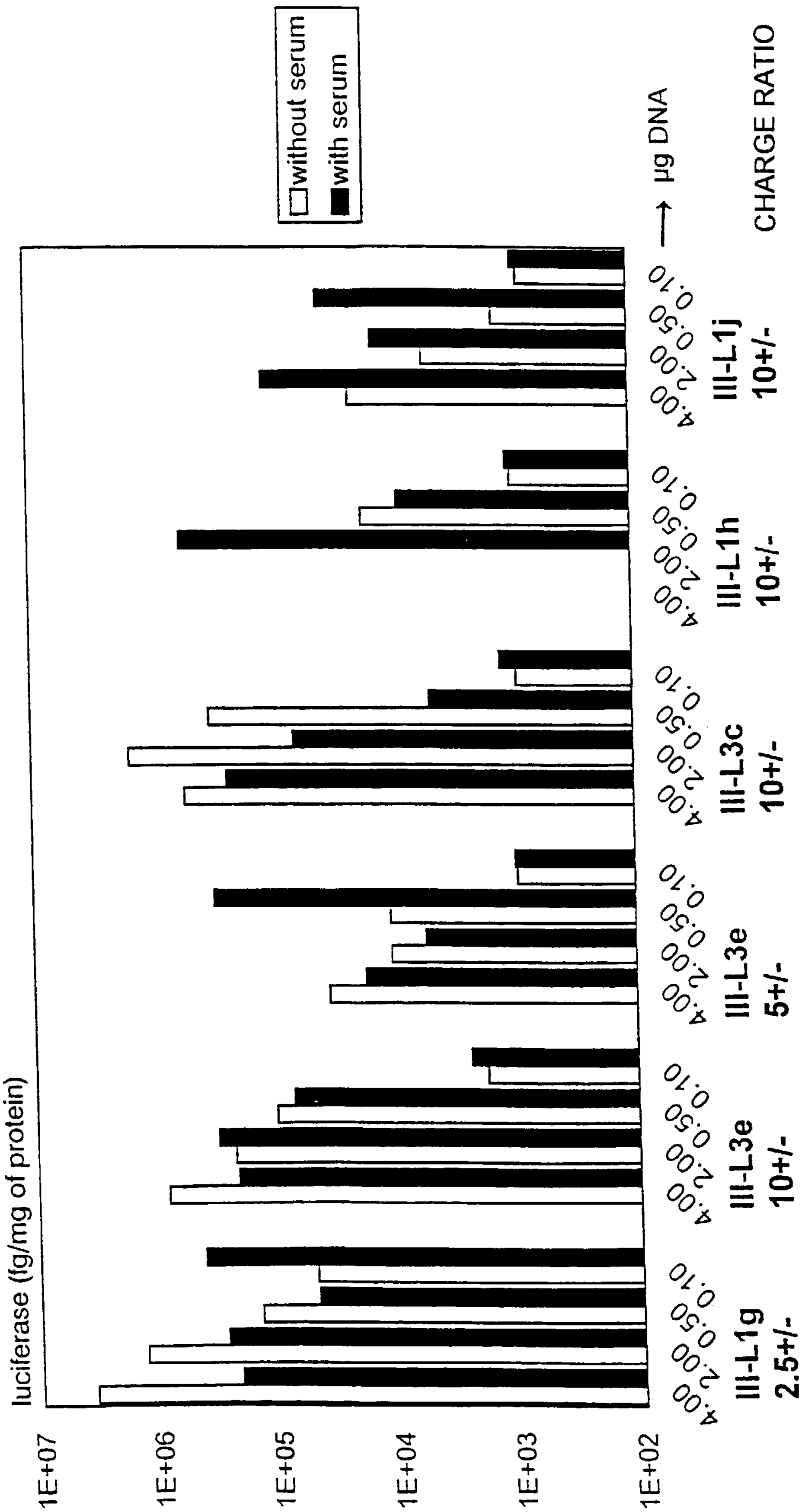

Fig. 3

in vitro transfection with polyamine telomers/DOPE

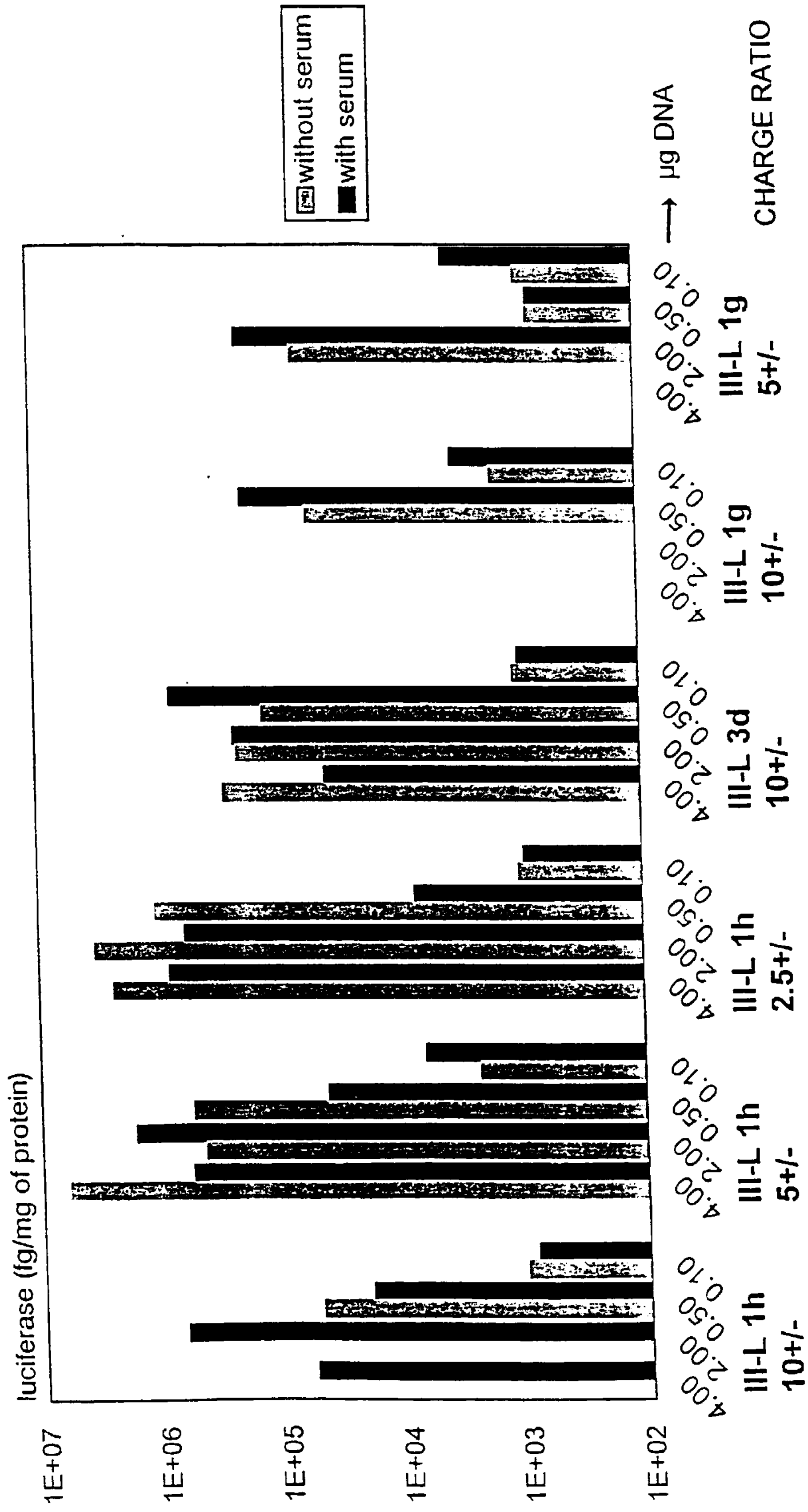

Fig. 4

in vitro transfection with polyamine telomers

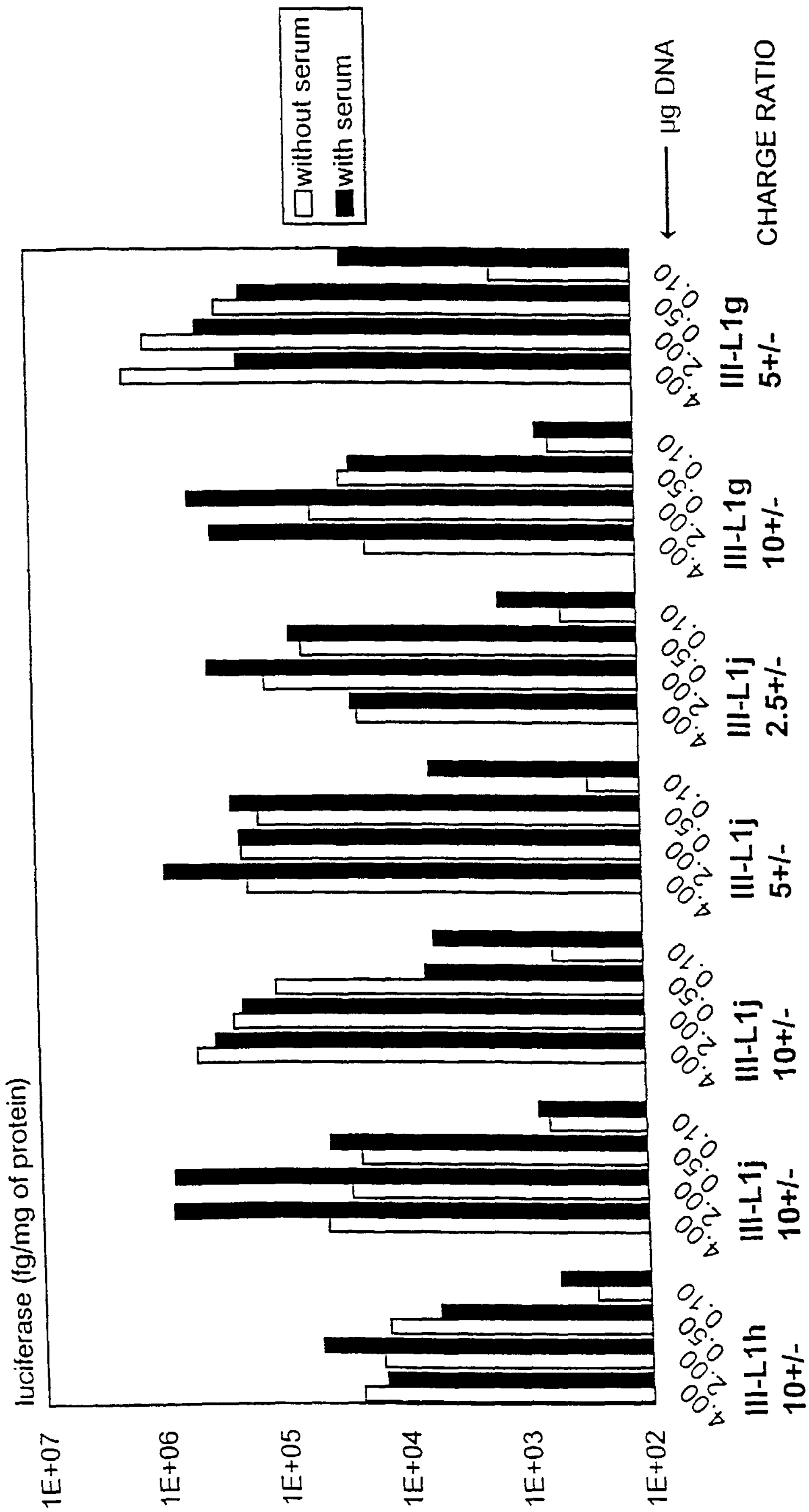

Figure 5